# FASCICULE DE BREVET EUROPEEN

(11) **EP 0 526 348 B1**
(45) Date de publication et mention de la délivrance du brevet: **18.02.1998**
(21) Numéro de dépôt: 92402213.0
(22) Date de dépôt: 03.08.1992
(51) Int. Cl.: C07D 209/42, C07D 401/06, C07D 401/12, C07D 403/06, C07C 311/29, C07D 295/18, C07D 295/20, C07C 311/21, A61K 31/40

(54) **Dérivés d'indoline portant une fonction amidique, leur préparation, les compositions pharmaceutiques en contenant**
Indolinderivate mit einer Amidgruppe, ihre Herstellung und sie enthaltende pharmazeutische Zusammensetzungen
Indoline derivatives carrying an amide function, their preparation and pharmaceutical compositions containing them

(30) Priorité: 02.08.1991 FR 9109908
(43) Date de publication de la demande: 03.02.1993
(73) Titulaire: SANOFI, 75008 Paris (FR)
(72) Inventeur: Wagnon, Jean, F-34000 Montpellier (FR); Serradeil-Legal, Claudine, F-31750 Escalquens (FR); Tonnerre, Bernard, F-34570 Vailhauques (FR); Plouzane, Claude, F-34680 Saint Georges D'Orques (FR); Nisato, Dino, F-34680 Saint Georges D'Orques (FR)
(74) Mandataire: Gillard, Marie-Louise

(56) Documents cités:
- EP-A- 0 093 084
- EP-A- 0 469 984
- DE-A- 3 705 934
- US-A- 3 838 167

## Description

La présente invention a pour objet des dérivés de la N-sulfonyl indoline, portant une fonction amidique, leur préparation et les compositions pharmaceutiques en contenant.

Le brevet US 3 838 167 décrit certains dérivés du N-sulfonyl indole répondant à la formule : 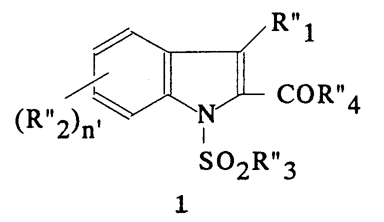 dans laquelle
- R''₁ représente l'hydrogène, un alkyle ou un phényle éventuellement substitué;
- R''₂ représente un halogène, un alkyle, un alcoxy, un nitro ou trifluorométhyle ;
- R''₃ représente un alkyle, un phényle ou un alkylphényle ;
- R''₄ représente un alkyle, un phényle, éventuellement substitué, un alcoxy ou un phénoxy ;
- n' = 0, 1 ou 2.

Ces composés 1 sont des intermédiaires de synthèse pour la préparation de dérivés indoliques, actifs sur le système nerveux central, de formule : 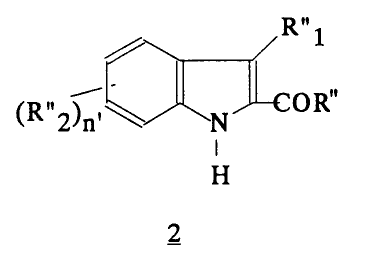 dans laquelle R'' représente un alkyle, un phényle éventuellement substitué ou un hydroxyle.

Des dérivés de la N-sulfonyl indoline de formule 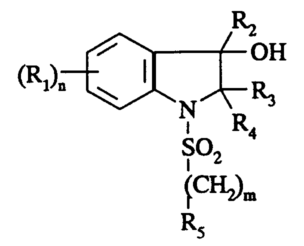 dans laquelle R₄ peut représenter un groupe carboxamide NR₆R₇ dans lequel R₇ est l'hydrogène ou une chaîne hydrocarbonée, sont décrits dans la demande EP 0 469 984.

Les dérivés de l'indoline selon la présente invention ont une affinité pour les récepteurs de la vasopressine et de l'ocytocine.

La vasopressine est une hormone connue pour son effet antidiurétique et son effet dans la régulation de la pression artérielle. Elle stimule plusieurs types de récepteurs : V₁, V₂, V₁ₐ, V_{1b} et exerce ainsi des effets cardiovasculaires, centraux, hépatiques, antidiurétiques, émétiques, agrégants, ainsi que des effets prolifératifs et mitotiques, notamment sur les tissus vasculaires et hépatiques. Les antagonistes des récepteurs de la vasopressine peuvent intervenir sur la régulation de la circulation centrale ou périphérique, notamment les circulations coronaire, rénale et gastrique ; ainsi que le métabolisme de l'eau et la libération de l'hormone adrénocorticotrophique (ACTH). Les récepteurs de la vasopressine comme ceux de l'ocytocine se trouvent aussi sur le muscle lisse de l'utérus. L'ocytocine a une structure peptidique proche de celle de la vasopressine. Ses récepteurs se trouvent également sur les cellules myoépithéliales de la glande mammaire et dans le système nerveux central. (Presse Médicale, 1987, 16 (10), 481-485, J. Lab. Clin. Med., 1989, 114 (6), 617-632 et Pharmacol. Rev., 1991, 43 (1), 73-108).

Ainsi les composés selon l'invention sont utiles notamment dans le traitement des affections du système nerveux central, du système cardiovasculaire et de la sphère gastrique chez l'homme et chez l'animal.

La présente invention a pour objet des composés de formule : 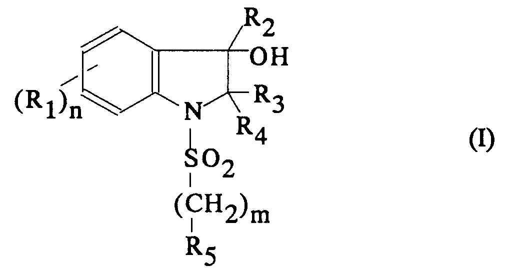 dans laquelle
- R₁ représente un atome d'halogène, un alkyle en C₁-C₄, un hydroxyle, un alcoxy en C₁-C₄, un groupe benzyloxy, un groupe cyano, un groupe trifluorométhyle, un groupe nitro ou un groupe amino ;
- R₂ représente un alkyle en C₁-C₆, un cycloalkyle en C₃-C₇, un cycloalcène en C₅-C₇, un phényle non substitué ou substitué une ou plusieurs fois par un alkyle en C₁-C₄, un alcoxy en C₁-C₄, un halogène, un groupe trifluorométhyle, un groupe amino, ou R₂ représente un nitrophényle non substitué ou substitué une fois par un groupe trifluorométhyle ou une ou plusieurs fois par un alkyle en C₁-C₄ ou un halogène ;
- R₃ représente un atome d'hydrogène ;
- R₄ représente un groupe carbamoyle de formule CONR₆R₇ ;
- R₅ représente un alkyle en C₁-C₄ ; un naphtyl-1 ; un naphtyl-2 ; un diméthylamino-5 naphtyl-1 ; un phényle non substitué ou substitué par un ou plusieurs substituants choisis parmi un halogène, un alkyle en C₁-C₄, un groupe trifluorométhyle, un groupe amino libre ou substitué par un ou 2 alkyles en C₁-C₄, un hydroxyle, un alcoxy en C₁-C₄, un alcénoxy en C₂-C₄, un alkylthio en C₁-C₄, un groupe trifluorométhoxy, un groupe benzyloxy, un groupe cyano, un groupe carboxy, un groupe alcoxycarbonyle en C₁-C₄, un groupe carbamoyle libre ou substitué par un ou deux alkyles en C₁-C₄ ou un groupe alkylamido en C₁-C₄, ou R₅ représente un nitrophényle non substitué ou substitué une fois par un groupe trifluorométhyle ou un alcénoxy en C₂-C₄ ou une ou plusieurs fois par un halogène, un alkyle en C₁-C₄, un alcoxy en C₁-C₄, un alkylthio en C₁-C₄, un groupe trifluorométhoxy ou un groupe benzyloxy ;
- R₆ représente un alkyle en C₁-C₆, ou R₆ est semblable à R₇ ;
- R₇ représente un groupe pipéridin-4-yl ; un groupe azétidin-3-yl, lesdits groupes étant substitués ou non sur l'azote par un alkyle en C₁-C₄, par un benzyloxycarbonyle ou par un alcoxycarbonyle en C₁-C₄ ; un groupe (CH₂)ᵣ lui-même substitué par un groupe pyridyl -2, -3 ou -4, par un groupe hydroxyle, par un groupe amino libre ou substitué par un ou deux alkyles en C₁-C₄, un groupe carboxyle, un groupe alcoxycarbonyle en C₁-C₄, un groupe benzyloxycarbonyle, un groupe carbamoyle libre ou substitué par un ou 2 alkyles en C₁-C₄ ;
- ou R₆ et R₇ ensemble constituent avec l'atome d'azote auquel ils sont liés un hétérocycle choisi parmi :
   . la morpholine,
   . la thiomorpholine,
   . la thiazolidine ou la 2,2-diméthylthiazolidine non substituées ou substituées par R₈,
   . la pipérazine non substituée ou substituée en 4 par un groupe R''₈,
   . un cycle insaturé monoazoté à 5 chaînons substitué par R₈ ou un cycle saturé monoazoté à 3, 4, 5, 6 ou 7 chaînons substitué par R₈ et R₉ ;
- R₈ représente R'₈ ou un groupe (CH₂)ᵣ lui-même substitué par un hydroxyle ou par un amino libre ou substitué par un ou deux alkyles en C₁-C₄ ;
- R'₈ représente un groupe (CH₂)_{q} lui-même substitué par un groupe carboxyle, un groupe alcoxycarbonyle en C₁-C₄, un groupe benzyloxycarbonyle, un groupe carbamoyle libre ou substitué par un hydroxyle ou par un ou 2 alkyles en C₁-C₄, ou un groupe aminocarbothioyle libre ou substitué par un ou 2 alkyles en C₁-C₄ ;
- R''₈ représente R'₈ ou un groupe (CH₂)₂NH₂ libre ou substitué par un ou deux alkyles en C₁-C₄;
- R₉ représente l'hydrogène, un halogène, un groupe (CH₂)ᵣOR₁₀, un groupe (CH₂)ᵣ NR₁₁R₁₂, un groupe (CH₂)ₛCONR₁₁ R'₁₁, un groupe azido;
- R₁₀ représente l'hydrogène, un alkyle en C₁-C₄, un mésyle ou un tosyle ;
- R₁₁, R'₁₁ et R₁₂ représentent chacun un hydrogène ou un alkyle en C₁-C₄ ou R₁₁ représente l'hydrogène et R₁₂ représente un benzyloxycarbonyle ou un alcoxycarbonyle en C₁-C₄ ;
- n représente 0, 1 ou 2 ;
- m représente 0, 1 ou 2 ;
- q représente 0, 1, 2 ou 3 ;
- r représente 0, 1, 2 ou 3, avec la limitation que r n'est pas nul lorsque R₈ ou R₉ est en position alpha de l'azote amidique intracyclique ;
- s représente 0 ou 1 ;
ainsi que leurs sels éventuels.

Les sels des composés de formule (I) selon la présente invention comprennent ceux avec des acides minéraux ou organiques qui permettent une séparation ou une cristallisation convenable des composés de formule (I), tels que l'acide picrique, l'acide oxalique ou un acide optiquement actif, par exemple un acide mandélique ou un acide camphosulfonique, et ceux qui forment des sels pharmaceutiquement acceptables tels que le chlorhydrate, l'hydrogénosulfate, le dihydrogénophosphate, le méthanesulfonate, le maléate, le fumarate, le 2-naphtalènesulfonate.

Les sels des composés de formule (I) comprennent également les sels avec des bases organiques ou minérales, par exemple les sels des métaux alcalins ou alcalino-terreux comme les sels de sodium, de potassium, de calcium, les sels de sodium et de potassium étant préférés, ou avec une amine, telle que le trométamol, ou bien les sels d'arginine, de lysine, ou de toute amine pharmaceutiquement acceptable.

Les composés (I) présentent une isomérie cis-trans autour de la liaison 2,3 de l'indoline. Les différents isomères font partie intégrante de l'invention.

Par convention, on appelle isomère cis, les composés (I) dans lesquels R₂ et R₄ sont du même côté du cycle.

Par convention, on appelle isomère trans les composés (I) dans lesquels R₂ et R₄ sont chacun d'un côté du cycle.

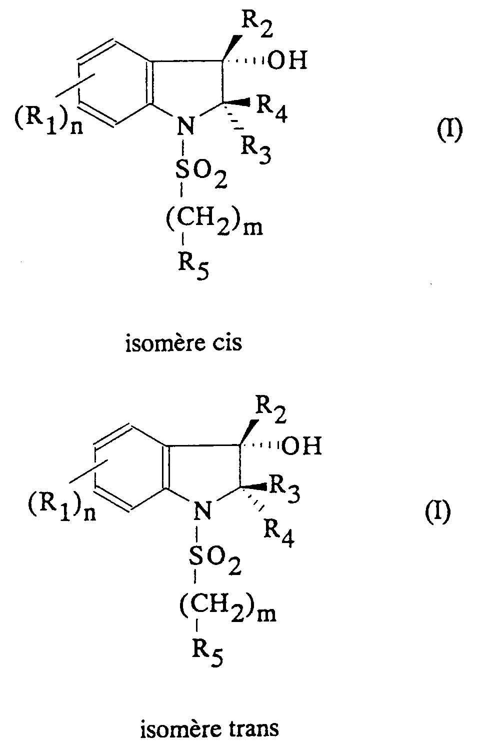

En outre, les composés selon l'invention présentent 2 atomes de carbone asymétriques ou davantage lorsque R₄ comprend 1 ou 2 carbones asymétriques. Les isomères optiques des composés (I) font partie de l'invention.

Dans la présente description et dans les revendications qui vont suivre, on entend par halogène un atome de fluor, de chlore, de brome ou d'iode ; par groupe alkyle on entend des groupes hydrocarbonés linéaires ou ramifiés.

Des composés (I) préférés selon l'invention sont ceux dans lesquels au moins l'une des conditions suivantes est respectée :
- R₁ représente un atome de chlore, de brome ou un groupe méthoxy et n = 1;
- R₂ représente un chlorophényle, un méthoxyphényle ou un cyclohexyle ;
- R₄ représente un groupe CONR₆R₇ dans lequel R₆ et R₇ ou NR₆R₇ ont l'une des définitions suivantes :
   . NR₆R₇ représente un groupe pyrrolidino substitué en position 2 par un groupe (CH₂)_{q} lui-même substitué par un groupe carboxyle ou carbamoyle avec q = 0,1,2 ou 3,
   . NR₆R₇ représente un groupe pipéridino substitué en position 4 par un groupe amino, un alkylamino en C₁-C₄ ou un dialkylamino en C₁-C₄,
   . NR₆R₇ représente un groupe thiazolidino substitué par un groupe (CH₂)_{q} lui-même substitué par un groupe carboxyle ou carbamoyle avec q = 0, 1, 2 ou 3.
   . NR₆R₇ représente un groupe pyrrolidino substitué en 2 par un groupe (CH₂)_{q} lui-même substitué par un groupe carboxyle ou carbamoyle avec q = 0, 1, 2 ou 3 et substitué en 4 par un groupe amino, un alkylamino en C₁-C₄ ou un dialkylamino en C₁-C₄.
- R₆ représente un alkyle en C₁-C₄ et R₇ représente un groupe (CH₂)ᵣ lui-même substitué par un groupe carboxyle ou un groupe carbamoyle avec r = 1, 2 ou 3;
- R₅ représente un phényle substitué en position 3 et 4 ou en position 2 et 4 par un groupe méthoxy, ou bien R₅ représente un phényle substitué en position 4 par un méthyle ;
- m = 0.

Particulièrement préférés sont les composés (I) sous forme d'isomère cis.

Dans la description et dans les exemples, les abréviations suivantes sont utilisées.
DCM : dichlorométhane
Ether iso : éther isopropylique
AcOEt : acétate d'éthyle
MeOH: méthanol
EtOH: éthanol
Ether : éther éthylique
DMF: diméthylformamide
THF: tétrahydrofuranne
TEA : triéthylamine
DMSO : diméthylsulfoxyde
DIPEA: diisopropyléthylamine
DCC: N,N'-dicyclohexylcarbodiimide
DBU : 1-8 diazabicyclo [5.4.0] undec-7-ène
TBD: triaza-1,5,7 bicyclo [4.4.0] dec-5-ène
DBN : diaza-1,5 bicyclo [4.3.0] non-5-ène
DMAP : diméthylamino-4 pyridine
DMPU : diméthyl-1,3 oxo-2 hexahydropyrimidinone
TMEDA: tétraméthyléthylènediamine
LDA : lithium diisopropylamide
HMPA: hexaméthylphosphoramide
HOBT: hydroxy-1 benzotriazole hydrate
BOP : hexafluorophosphate de benzotriazolyloxy trisdiméthylamino phosphonium
TFA : acide trifluoroacétique
Réactif de Lawesson : bis (méthoxy-4 phényl)-2,4 dithia-1,3 disphosphétane-2,4 disulfure-2,4
Fc: point de fusion
Solution saline : eau saturée en chlorure de sodium
Carboglace : dioxyde de carbone solide
CCM: chromatographie en couche mince
CLHP ou HPLC: chromatographie liquide à haute performance
RMN: résonance magnétique nucléaire
s : singulet
m : multiplet
s.e. : singulet élargi
d : doublet
Eau chlorhydrique : acide chlorhydrique dilué, environ 1N
NaH à 80 % : dispersion d'hydrure de sodium dans l'huile minérale (Janssen Chemica)
Me : méthyle
Et : éthyle
iPr : isopropyle, Pr : propyle
iPentyl : isopentyle
iBu : isobutyle
tBu : tert-butyle, Bu : butyle
Bz : benzyle
Ph : phényle
TA : température ambiante

La présente invention a également pour objet le procédé de préparation des composés (I).

Ce procédé est caractérisé en ce que
a) on fait réagir sur un dérivé d'amino-2 phénone de formule : 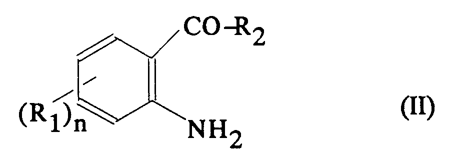 dans laquelle R₁, R₂ et n ont les significations indiquées ci-dessus pour I, un dérivé sulfonyle de formule :

   Hal - SO₂ - (CH₂)ₘ - R₅ (III)

   dans laquelle
   - Hal représente un halogène, de préférence le chlore ou le brome,
   - m et R₅ ont les significations indiquées ci-dessus pour (I) ;
b) le composé ainsi obtenu de formule : 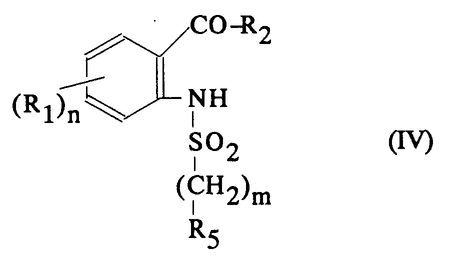 est traité par un dérivé halogéné de formule :

   Hal' - CH₂ COA (V)

   dans laquelle
   Hal' représente un halogène préférentiellement le brome, et A représente soit le groupe NR₆R₇, soit le groupe OR dans lequel R est un tertiobutyle ou un benzyle;
c) le cas échéant, lorsque A représente un groupe OR, l'ester ainsi obtenu de formule : 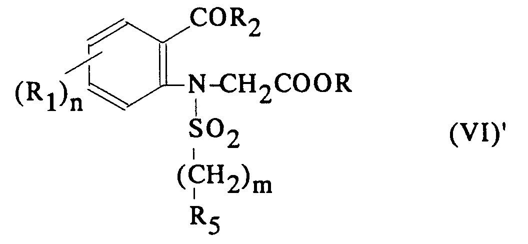 est déprotégé dans les conditions convenables ;
d) le cas échéant, l'acide ainsi obtenu à l'étape c) de formule : 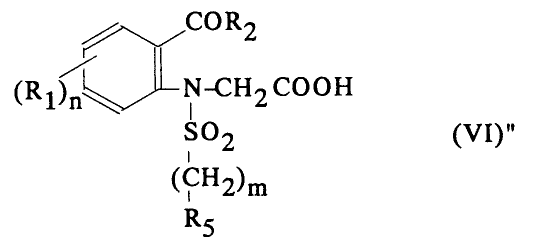 ou son chlorure d'acide de formule : 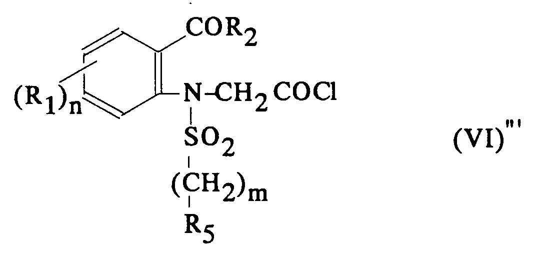 est traité par un composé HNR₆R₇ selon les techniques convenables du couplage amidiques ;
e) le composé ainsi obtenu à l'étape b) ou à l'étape d), de formule : 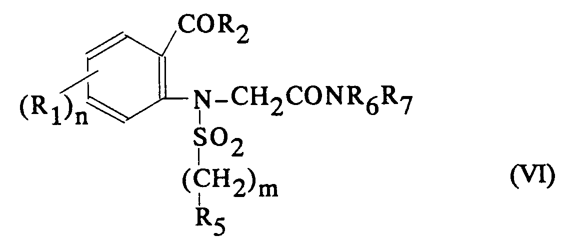 est cyclisé en milieu basique pour préparer le composé (I) selon l'invention ;
f) on sépare éventuellement les isomères cis et trans du composé (I) et, éventuellement, on sépare les énantiomères.

Les dérivés d'amino-2 phénone (II) sont connus ou préparés par des méthodes connues, telles que celles décrites par A.K. Singh et al., Synth. Commun. 1986, 16 (4), 485 et G.N. Walker J. Org. Chem., 1962, 27, 1929. Les amino-2 trifluorométhyl-2' benzophénones et les autres dérivés trifluorométhylés sont préparés selon le brevet US 3 341 592.

Le chlorure de diméthoxy-2,4 benzènesulfonyle est préparé selon J. Am. Chem. Soc., 1952, 74, 2008.

Les dérivés sulfonyle de formule (III) sont connus ou préparés par des méthodes connues. Ainsi par exemple, le chlorure de diméthylamino-4 benzènesulfonyle est préparé selon C.N. Sukenik et al., J. Am. Chem. Soc., 1977, 99, 851-858 ; le chlorure de p-benzyloxybenzènesulfonyle est préparé selon la demande de brevet européen EP 229 566.

Le chlorure d'alcoxybenzènesulfonyle est préparé à partir de l'alcoxybenzène sulfonate de sodium, lui-même préparé par action d'un halogénure d'alkyle sur l'hydroxybenzènesulfonate de sodium.

Les dérivés halogénés de formule (V) sont connus ou préparés par des méthodes connues, telles que celles décrites par A.I. Vogel : a Text Book of Practical Organic Chemistry: Longman, 3rd ed. 1956, p. 383, ou G. Kirchner et al., J. Am. Chem. Soc., 1985, 107, 24, 7072.

L'étape a) du procédé est réalisée dans la pyridine par chauffage à une température comprise entre la température ambiante et le point d'ébullition du solvant pendant une période de temps comprise entre quelques heures et quelques jours. On peut éventuellement opérer en présence de diméthylaminopyridine que l'on utilise en quantité catalytique ou stoechiométrique.

L'étape b) du procédé est réalisée entre le sulfonamide de formule (IV) et le dérivé halogéné de formule (V), en excès, dans un solvant tel que le diméthylformamide ou le diméthylsulfoxyde, sous une atmosphère inerte, à une température comprise entre 0°C et la température ambiante, pendant un temps compris entre quelques heures et 24 heures, en présence d'hydrure de sodium.

Lorsque le groupe -NR₆R₇ contient une deuxième fonction amine, c'est à dire lorsque R₆ et/ou R₇ sont substitués par un groupe amino, on peut choisir d'utiliser un dérivé halogéné (V) de formule Hal'- CH₂ - CO₂R dans lequel R est un tertiobutyle ou un benzyle, pour préparer les intermédiaires de formule (VI)' puis (VI)''. Dans ce cas, l'étape c) pour la formation de l'acide de formule (VI)'' est effectuée par soit action de l'hydrogène en présence d'un catalyseur tel que le Palladium sur charbon lorsque R est le benzyle, soit en milieu acide, lorsque R est le tertiobutyle, par exemple en présence de TFA, ou en présence d'acide bromhydrique dans l'acide acétique, ou bien en présence de ZnBr₂ dans le DCM.

L'étape d) est alors effectuée dans les conditions classiques du couplage amidique, par exemple en présence de BOP ou de HOBT et de DCC.

Les composés HNR₆R₇ sont connus ou préparés par des méthodes connues. A titre d'exemple, la synthèse stéréospécifique des acides (R) et (S) pyrrolidin-2-yl acétique est effectuée selon H. Rueger et al. dans Heterocycles, 1982, 19 (9), 1677 à partir d'un dérivé de la proline de configuration appropriée La préparation du N-Boc dehydro-3,4 (α)prolinate de méthyle est effectuée selon J.R. Dormoy, Synthesis, 1982, 753. La préparation de dérivés optiquement purs de l'acide pipécolique est décrite par exemple dans Tetrahedron, 1992, 48 (3) 431-442 et Tetrahedron, 1991, 47 (24) 4039-4062.

La préparation des dérivés de l'acide aziridine carboxylique est effectuée selon K. Nakajima et al. dans Bull. Chem. Soc. Jap., 1978, 51 (5), 1577.

L'étape e) du procédé s'apparente à une réaction d'aldolisation : le groupement méthylène en α de l'amide est déprotoné, puis la fonction carbonyle de la phénone agit comme un électrophile interne, ce qui conduit à la cyclisation avec apparition de deux carbones asymétriques (C*).

On peut schématiser la réaction comme suit :

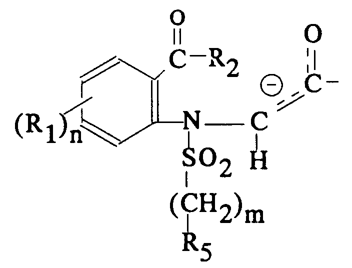

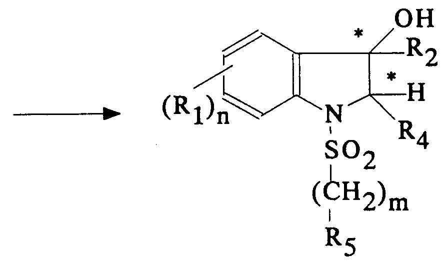

Les principes de la réaction d'addition aldolique ont été revus par C.H. Heathcock dans Asymetric Synthesis, vol. 3 : Stereodifferentiating additions reactions, part B, 111-112 ; Academic Press, 1984, J.D. Morrison ed.

Il est connu que la réaction aldolique d'anions d'amides achirales conduit à la formation de 2 diastéréoisomères racémiques de β-hydroxy amides dans un rapport qui dépend largement des conditions expérimentales utilisées. Parmi ces conditions, on peut citer : la nature de la base minérale ou organique utilisée, la nature des cations ou contre-ions, la présence éventuelle d'additifs dans le milieu réactionnel, le solvant, la température de la réaction, ainsi que la structure du composé qui subit cette réaction.

Lorsque les groupes R₆ et R₇ ne comportent par de fonction hydrolysable en milieu alcalin, on peut utiliser la soude dans l'eau, en présence d'un cosolvant, avec ou sans l'ajout d'un catalyseur de transfert de phase ; on peut également utiliser un hydroxyde d'ammonium quaternaire, par exemple l'hydroxyde de benzyltriméthylammonium dans le méthanol.

Pour effectuer cette réaction d'aldolisation, on peut également utiliser des bases organiques, par exemple :
- des guanidines comme le triaza-1,5,7 bicyclo [4.4.0] dec-5-ène,
- des amidines comme le diaza-1,8 bicyclo [5.4.0] undec-5-ène ou le diaza-1,5 bicyclo [4.3.0] non-5-ène,
dans un solvant ou un mélange de solvants choisis parmi, par exemple, le benzène, le THF, le dichlorométhane, le méthanol, le diméthylformamide ; la réaction est effectuée sous atmosphère inerte entre -10°C et 110°C ; la quantité de base utilisée est au moins stoechiométrique ; on peut également effectuer la réaction sans solvant, à la température du bain.

Préférentiellement, l'étape e) du procédé selon l'invention est effectuée en présence de diaza-1,8 bicyclo[5.4.0]undec-5-ène (DBU) dans un solvant tel que le dichlorométhane ou le méthanol, à une température comprise entre -10°C et la température de reflux du solvant.

On peut également utiliser un alcoolate d'alcool primaire, secondaire ou tertiaire avec le lithium, le sodium, le potassium, le calcium ou le magnésium.

L'alcoolate est utilisé en quantité catalytique ou stoechiométrique dans un solvant anhydre, par exemple un alcool (éventuellement en présence d'un cosolvant tel que le THF), ou bien en quantité stoechiométrique dans le THF, le DMF ou le DMSO, éventuellement en présence d'éthers-couronne, par exemple le dicyclohexyl-18 crown-6 ; la réaction est effectuée entre -15°C et 80°C.

L'emploi d'amidure de type RR'NLi ou RR'NMgBr, dans lesquels R et R' sont des radicaux monovalents, comme agent de déprotonation est une méthode de formation d'énolates d'amides, intermédiaires de la réaction d'aldolisation ; cette méthode a été revue récemment par R.E. Ireland et al., J. Org. Chem., 1991, 56, 650. Le solvant de la réaction peut être le benzène, l'hexane ou le THF, utilisé anhydre, sous atmosphère inerte. On peut ajouter des adjuvants comme LiF, LiCl, LiBr, LiI, LiBu, TMEDA, DMPU, HMPA ou éther couronne. (M. Murakate et al., J. Chem. Soc. Commun., 1990, 1657). A titre d'exemple, on peut citer l'utilisation du diisopropylamidure de lithium entre -78°C et -30°C dans la THF anhydre, sous atmosphère inerte ou dans le THF en présence d'additifs tels que la tétraméthylènediamine, DMPU ou HMPA par exemple. D'autres amidures connus et utilisables sont, par exemple, le cyclohexylamidure de lithium, le tétraméthyl-2 ,2,6,6 cyclohexylamidure de lithium. On peut également préparer d'autres amidures, par action de la quantité nécessaire de butyllithium dans l'hexane sur des amines secondaires linéaires ou cycliques, la réaction se faisant dans l'un des solvants cités ci-dessus.

Enfin diverses publications décrivent des amidures d'amines secondaires optiquement actives : L. Duhamel et al., Bull. Soc. Chim. France, 1984, II, 421 ; J.K. Whitesell et al., J. Org. Chem., 1980, 45, 755 ; M. Murakata et al., J. Chem. Soc. Chem. Commun., 1990, 1657 ; M. Yamaguchi, Tetrahedron Lett., 1986, 27 (8), 959 ; P.J. Cox et N.S. Simpkins, Tetrahedron: Asymmetry, 1991, 2 (1), 1.

Les silylamidures de lithium, de sodium ou de potassium constituent un autre groupe de bases utilisables parmi lesquelles on peut citer : (Me₃Si)₂NLi, (Me₂PhSi)₂NLi, (Et₃Si)₂NLi, (Me₃Si)₂NK, (Me₃Si)₂NNa.

On peut également utiliser des amidures mixtes tels que décrits par Y. Yamamoto, Tetrahedron, 1990, 46, 4563, par exemple, le sel de lithium de N-(triméthylsilyl) benzylamine, ou un analogue dans lequel la benzylamine est remplacée par une amine, primaire chirale comme la (R) ou la (S) α-méthylbenzylamine.

Lorsque le composé de formule (I) à préparer possède 2 atomes de carbone asymétriques, l'utilisation d'amidures ou d'alcoolates chiraux à l'étape e) permet un enrichissement énantiomérique de chacun des stéréoisomères cis ou trans. On détermine alors la proportion de chacun des énantiomères par dosage sur une colonne chirale de chromatographie liquide à haute performance.

Lorsque le composé de formule (I) à préparer possède 3 ou 4 atomes de carbone asymétriques, l'étape de cyclisation c) peut s'accompagner d'un enrichissement diastéréoisomérique et l'utilisation d'une base chirale appropriée permet de modifier cet enrichissement diastéréoisomérique.

A l'étape f) les isomères géométriques cis et trans du composé (I) formés sont extraits par les méthodes classiques et séparés par chromatographie ou cristallisation fractionnée.

Eventuellement, on sépare les isomères optiques de chacun des isomères cis et trans, par exemple, par chromatographie préparative sur une colonne chirale suivie éventuellement d'une cristallisation fractionnée ou par formation d'un sel optiquement actif en présence d'un acide ou d'une base chirales convenablement choisis.

Ainsi, lorsque le composé selon l'invention possède 2 atomes de carbone asymétriques, les énantiomères peuvent être séparés par CLHP chirale.

Lorsque le composé selon l'invention possède 3 ou 4 atomes de carbone asymétriques, on peut séparer les diastéréoisomères en utilisant les méthodes de chromatographie et les méthodes de cristallisation fractionnée.

Pour différencier et caractériser l'isomère cis et l'isomère trans d'un composé (I), plusieurs méthodes sont possibles. Lorsque R₃ est l'hydrogène, on effectue une analyse comparative par RMN à haut champ (250 Mhz), assortie par exemple de l'étude de l'effet Overhauser (N.O.E.) entre, par exemple, le proton de l'indoline (R₃ = H) et le proton de l'hydroxyle.

Les spectres IR de l'isomère cis et de l'isomère trans en solution dans le DCM sont différents. L'isomère cis présente le plus souvent une forte bande d'absorption fine et symétrique vers 3550-3520 cm⁻¹, due à la vibration de l'hydroxyle, alors que l'isomère trans ne présente aucune bande de vibration résolue dans cette région.

Grâce aux données recueillies, on a constaté que l'isomère cis est en général le plus mobile en CCM sur une plaque d'oxyde d'alumine (60F254 neutral, Type E, Merck), en éluant par du DCM contenant des proportions variables d'AcOEt. De même, par chromatographie sur colonne d'alumine (oxyde d'alumine 90, granulométrie 0,063-0,200 mm), l'isomère cis est élué le plus souvent le premier, l'éluant étant du DCM contenant des proportions variables d'AcOEt ou de MeOH.

Ainsi l'isomérie cis ou trans d'un composé (I) selon l'invention peut le plus souvent être déterminée par une méthode analytique. On procède également par analogie entre des composés voisins ou entre des composés préparés l'un à partir de l'autre.

La configuration absolue de certains composés selon l'invention a été déterminée par une analyse aux rayons X. Par déduction en en prenant en compte la valeur du pouvoir rotatoire, on peut connaitre également la configuration absolue d'autres composés obtenus de manière analogue.

On peut préparer un composé (I) dans lequel R₁ est un groupe amino et/ou un composé dans lequel R₅ représente un groupe phényle substitué par un amino, par transformation d'un composé (VI) obtenu à l'étape b) dans lequel R₁ est un groupe nitro et/ou R₅ est un groupe phényle substitué par un nitro, les autres substituants ayant les valeurs souhaitées pour (I), par hydrogénation catalytique, par exemple en présence de Palladium sur charbon, de Rhodium sur alumine ou de Nickel de Raney.

Les composés (I) dans lesquels les substituants R₆ et/ou R₇ ou le groupement NR₆R₇ comprennent un groupe alcoxycarbonyle en C₁-C₄, permettent d'obtenir, par hydrolyse de l'ester, les composés (I) dans lesquels R₆ et/ou R₇ ou le groupement NR₆R₇ comprennent un groupe carboxyle, les autres substituants de (I) étant inchangés. De plus, les composés dans lesquels R₆ et/ou R₇ ou NR₆R₇ comprennent un groupe carboxyle, permettent d'obtenir par une réaction classique de couplage amidique, les composés (I) dans lesquels R₆ et/ou R₇ ou le groupement NR₆R₇ comprennent un groupe carbamoyle libre ou substitué par un ou deux alkyles en C₁-C₄, les autres substituants étant identiques.

Enfin, les composés (I) dans lesquels R₆ et/ou R₇ ou le groupement NR₆R₇ comprennent un groupe carbamoyle permettent d'obtenir par un réarrangement d'Hofmann, les composés (I) dans lesquels R₆ et/ou R₇ ou le groupe NR₆R₇ comprennent un groupe amino, les autres substituants étant identiques (J. Org. Chem., 1979, 44 (10), 1746).

Ainsi selon la présente invention le procédé de préparation des composés (I) dans lesquels R₆ et/ou R₇ ou le groupement NR₆R₇ comprennent un groupe amino libre ou substitué par un ou deux alkyles en C₁-C₄, peut présenter deux variantes :
i) on effectue l'étape b) du procédé en traitant le composé (IV) obtenu à l'étape a) avec un dérivé halogéné (V) de formule Hal'- CH₂CONR₆R₇ dans laquelle R₆ et/ou R₇ ou le groupement NR₆R₇ comprennent un groupe précurseur de l'amine, par exemple un carboxyester, un carboxy ou un carbamoyle ; on effectue ensuite l'étape de cyclisation e) puis on transforme le groupe précurseur de l'amine en amine, par exemple on hydrolyse le groupe carboxyester du composé (I) ainsi obtenu en un groupe carboxy, celui-ci est ensuite transformé en un groupe carbamoyle puis en un groupe amino par le réarrangement d'Hofmann.
ii) on effectue l'étape b) en traitant le composé (IV) obtenu à l'étape a) par un dérivé halogéné (V) de formule Hal'- CH₂COOR dans laquelle R représente un benzyle ou un tertiobutyle ; on déprotège l'ester du composé (VI)′ ainsi obtenu par un traitement approprié, selon l'étape c) ; puis on effectue un couplage avec le composé HNR₆R₇ dans lequel le groupe amino de R₆ et/ou R₇ est éventuellement protégé ; le composé (VI) ainsi obtenu est ensuite cyclisé selon l'étape e) ; et, éventuellement on prépare le composé (I) dans lequel le groupe amino est libre par déprotection de l'amine.

Les composés (I) dans lesquels les groupes R₆ et/ou R₇ ou le groupe NR₆R₇ comprennent un groupe benzyloxycarbonyle ou alcoxycarbonyle comme substituant d'une fonction amine permettent d'obtenir les composés (I) dans lesquels la fonction amine est libre, les autres substituants étant identiques.

Les composés de formule (VI) utiles comme intermédiaires pour la préparation des composés (I) selon l'invention sont nouveaux et font partie de l'invention. De même, les composés (VI)' et (VI)'' sont nouveaux et font partie de l'invention.

Ainsi la présente invention a également pour objet les composés de formule : 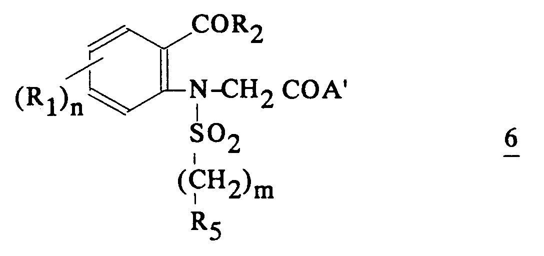 dans laquelle
A' représente un groupe choisi parmi : NR₆R₇, OH, OtBu, OBz ;
- R₁ représente un atome d'halogène, un alkyle en C₁-C₄, un hydroxyle, un alcoxy en C₁-C₄, un groupe benzyloxy, un groupe cyano, un groupe trifluorométhyle, un groupe nitro ou un groupe amino ;
- R₂ représente un alkyle en C₁-C₆; un cycloalkyle en C₃-C₇, un cycloalcène en C₅-C₇, un phényle non substitué ou substitué une ou plusieurs fois par un alkyle en C₁-C₄, un alcoxy en C₁-C₄, un halogène, un groupe trifluorométhyle, un groupe amino, ou R₂ représente un nitrophényle non substitué ou substitué une fois par un groupe trifluorométhyle ou une ou plusieurs fois par un alkyle en C₁-C₄, un alcoxy en C₁-C₄ ou un halogène ;
- R₅ représente un alkyle en C₁-C₄ ; un naphtyl-1 ; un naphtyl-2 ; un diméthylamino-5 naphtyl-1 ; un phényle non substitué ou substitué par un ou plusieurs substituants choisis parmi un halogène, un alkyle en C₁-C₄, un groupe trifluorométhyle, un groupe amino libre ou substitué par un ou 2 alkyles en C₁-C₄, un hydroxyle, un alcoxy en C₁-C₄, un alcénoxy en C₂-C₄, un alkylthio en C₁-C₄, un groupe trifluorométhoxy, un groupe benzyloxy, un groupe cyano, un groupe carboxy, un groupe alcoxycarbonyle en C₁-C₄, un groupe carbamoyle libre ou substitué par un ou deux alkyles en C₁-C₄ ou un groupe alkylamido en C₁-C₄, ou R₅ représente un nitrophényle non substitué ou substitué une fois par un groupe trifluorométhyle ou un alcénoxy en C₂-C₄, ou une ou plusieurs fois par un halogène, un alkyle en C₁-C₄, un alcoxy en C₁-C₄, un alkylthio en C₁-C₄, un groupe trifluorométhoxy ou un groupe benzyloxy ;
- R₆ représente un alkyle en C₁-C₆, ou R₆ est semblable à R₇ ;
- R₇ représente un groupe pipéridin-4-yl ; un groupe azétidin-3-yl, lesdits groupes étant substitués ou non sur l'azote par un alkyle en C₁-C₄ par un benzyloxycarbonyle ou par un alcoxycarbonyle en C₁-C₄ ; un groupe (CH₂)ᵣ lui-même substitué par un groupe pyridyl -2, -3 ou -4, par un groupe hydroxyle, par un groupe amino libre ou substitué par un ou deux alkyles en C₁-C₄, un groupe carboxyle, un groupe alcoxycarbonyle en C₁-C₄, un groupe benzyloxycarbonyle, un groupe carbamoyle libre ou substitué par un ou 2 alkyles en C₁-C₄ ;
- ou R₆ et R₇ ensemble constituent avec l'atome d'azote auquel ils sont liés un hétérocycle choisi parmi :
   . la morpholine,
   . la thiomorpholine,
   . la thiazolidine ou la 2,2-diméthylthiazolidine substitué ou non par R₈,
   . la pipérazine non substituée ou substituée en 4 par un groupe R''₈ ;
   . un cycle insaturé monoazoté à 5 chaînons substitué par R₈ ou
   . un cycle saturé monoazoté à 3, 4, 5, 6 ou 7 chaînons substitué par R₈ et R₉ ;
- R₈ représente R'₈ ou un groupe (CH₂)ᵣ lui-même substitué par un hydroxyle ou par un amino libre ou substitué par un ou deux alkyles en C₁-C₄ ;
- R'₈ représente un groupe (CH₂)_{q} lui-même substitué par un groupe carboxyle, un groupe alcoxycarbonyle en C₁-C₄, un groupe benzyloxycarbonyle, un groupe carbamoyle libre ou substitué par un hydroxyle ou par un ou 2 alkyles en C₁-C₄, ou un groupe aminocarbothioyle libre ou substitué par un ou 2 alkyles en C₁-C₄ ;
- R''₈ représente R'₈ ou un groupe (CH₂)₂NH₂ libre ou substitué par un ou deux alkyles en C₁-C₄ ;
- R₉ représente l'hydrogène, un halogène, un groupe (CH₂)ᵣ OR₁₀, un groupe (CH₂)ᵣ NR₁₁ R₁₂, un groupe (CH₂)ₛCONR₁₁ R'₁₁, un groupe azido;
- R₁₀ représente l'hydrogène, un alkyle en C₁-C₄, un mésyle ou un tosyle ;
- R₁₁, R'₁₁ et R₁₂ représentent chacun un hydrogène ou un alkyle en C₁-C₄ ou R₁₁ représente l'hydrogène et R₁₂ représente un benzyloxycarbonyle ou un alcoxycarbonyle en C₁-C₄ ;
- n représente 0, 1 ou 2 ;
- m représente 0, 1 ou 2 ;
- q représente 0, 1, 2 ou 3 ;
- r représente 0, 1, 2 ou 3, avec la limitation que r n'est pas nul lorsque R₈ ou R₉ est en position alpha de l'azote amidique intracyclique ;
- s représente 0 ou 1.

Selon un autre aspect de la présente invention, les composés (I) selon l'invention, dans lesquels soit R₇, soit le groupement NR₆R₇ comprend un groupement carboxyle sont utiles pour la préparation de composés analogues décarboxylés.

Ainsi, l'invention concerne selon cet aspect l'utilisation de composés de formule (I') : 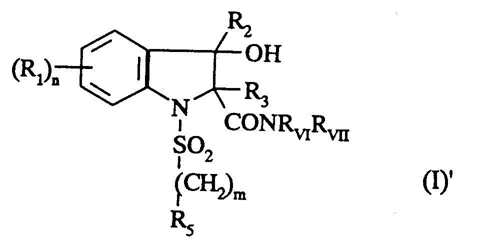 dans laquelle R₁, R₂, R₃, R₅, m et n ont les significations données plus haut pour les composés de formule (I)
- R_{VI} représente un alkyle en C₁-C₆,
- R_{VII} représente un groupe (CH₂)ᵣCOOH avec r = 1, 2 ou 3.
- ou R_{VI} et R_{VII} ensemble avec l'atome d'azote auquel ils sont liés constituent un hétérocycle choisi parmi :
   . la thiazolidine ou la 2,2-diméthylthiazolidine substituées par un groupe (CH₂)_{q}COOH,
   . la pipérazine substituée en 4 par un groupe (CH₂)_{q}-COOH,
   . un cycle insaturé monoazoté à 5 chaînons substitué par un groupe (CH₂)_{q}COOH,
   . un cycle saturé monoazoté à 3, 4, 5, 6 ou 7 chaînons substitué par un groupe (CH₂)_{q}COOH,
   avec q = 0, 1, 2 ou 3,
   pour la préparation d'un composé de formule (I)'' ayant la même configuration autour de la liaison 2, 3 de l'indoline que le produit de départ 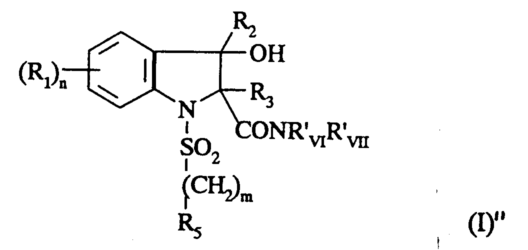 dans laquelle :
- R₁, R₂, R₃, R₅, m et n sont tels que définis ci-dessus,
- R'_{VI} représente un alkyle en C₁-C₆,
- R'_{VII} représente le groupe (CH₂)ᵣH,
   ou R'_{VI} et R'_{VII} ensemble avec l'atome d'azote auquel ils sont liés constituent un hétérocycle choisi parmi :
   . la thiazolidine ou la 2,2-diméthylthiazolidine substituée par un groupe (CH₂)_{q}H,
   . la pipérazine substituée en 4 par un groupe (CH₂)_{q}H,
   . un cycle insaturé monoazoté à 5 chaînons substitué par un groupe (CH₂)_{q}H,
   . un cycle saturé monoazoté à 3, 4, 5, 6 ou 7 chaînons substitué par un groupe (CH₂)_{q}H.

La réaction de décarboxylation radicalaire est effectuée selon D.H.R. Barton et al. dans J. Chem. Soc; Chem. Commun.; 1984, 1298.

L'affinité des composés selon l'invention pour les récepteurs de la vasopressine a été déterminée in vitro en utilisant la méthode décrite dans J. Biol. Chem., 1985, 260 (5), 2844-2851. Cette méthode consiste à étudier le déplacement de la vasopressine tritiée fixée aux sites V₁ de membranes de foie de rats. Les concentrations inhibitrices de 50 % (CI₅₀) de la fixation de la vasopressine tritiée des composés selon l'invention sont faibles, allant jusqu'à 10⁻⁹M.

Par ailleurs, on a mesuré l'inhibition de l'agrégation plaquettaire induite par la vasopressine sur un plasma humain riche en plaquettes (PRP humain) en utilisant la méthode décrite dans Thrombosis Res., 1987, 45, 7-16. Les composés selon l'invention inhibent l'agrégation induite par des concentrations 50 à 100 nM de vasopressine avec des DI₅₀ (doses inhibitrices) faibles, allant jusqu'à 10⁻⁹M. Ces résultats montrent l'activité antagoniste des récepteurs V₁ des composés selon l'invention.

L'affinité des composés (I) selon l'invention pour les récepteurs V₂ a été mesurée selon une méthode adaptée de P. Crause et al., Molecular and Cellular Endocrinology, 1982, 28, 529-541.

Les composés selon l'invention de configuration cis autour de la liaison 2,3 de l'indoline ont une sélectivité marquée pour les récepteurs V₁.

L'affinité des composés (I) selon l'invention pour les récepteurs de l'ocytocine a été déterminée in vitro par déplacement de l'ocytocine tritiée fixée aux récepteurs d'une préparation membranaire de glandes de rates gestantes. Les CI₅₀ des composés selon l'invention sont faibles, comprise entre 10⁻⁵M et 10⁻⁸M.

Les composés selon l'invention sont actifs après administration par différentes voies, notamment par voie orale.

Aucun signe de toxicité n'est observé avec ces composés aux doses pharmacologiquement actives.

Ainsi les composés selon l'invention peuvent être utilisés dans le traitement ou la prévention de différentes affections vasopressine-dépendantes, notamment les affections cardiovasculaires, comme l'hypertension, l'insuffisance cardiaque, la trombose, ou le vasospasme coronaire, en particulier chez le fumeur ; les affections du système nerveux central, les oedèmes cérébraux, les états psychotiques, les troubles de l'appétit, les troubles de la mémoire, par exemple ; les affections du système rénal comme le vasospasme rénal, la nécrose du cortex rénal ; les affections du système gastrique, par exemple les ulcères ou encore le syndrome de la sécrétion inappropriée de l'hormone antidiurétique (SIADH).

Les composés selon l'invention peuvent aussi être utilisés comme antiémétiques, notamment dans le mal des transports ainsi que comme agents antiprolifératifs, par exemple dans le cancer ou l'athérosclérose.

Chez la femme, les composés selon l'invention peuvent également être utilisés pour traiter la dysménorrhée ou le travail prématuré.

La présente invention a également pour objet des compositions pharmaceutiques contenant une dose efficace d'un composé selon l'invention ou d'un sel pharmaceutiquement acceptable et des excipients convenables. Lesdits excipients sont choisis selon la forme pharmaceutique et le mode d'administration souhaité.

Dans les compositions pharmaceutiques de la présente invention pour l'administration orale, sublinguale, sous-cutanée, intramusculaire, intraveineuse, topique, intratrachéale, intranasale, transdermique ou rectale, les principes actifs de formule I ci-dessus, ou leurs sels éventuels, peuvent être administrés sous formes unitaires d'administration, en mélange avec des supports pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules et les solutions ou suspensions orales, les formes d'administration sublinguale, buccale, intratrachéale, intranasale, les formes d'administration sous-cutanée, intramusculaire ou intraveineuse et les formes d'administration rectale. Pour l'application topique, on peut utiliser les composés selon l'invention dans des crèmes, pommades ou lotions.

Afin d'obtenir l'effet prophylactique ou thérapeutique désiré, la dose de principe actif peut varier entre 0,01 et 50 mg par kg de poids du corps et par jour.

Chaque dose unitaire peut contenir de 0,5 à 1000 mg, de préférence de 1 à 500 mg, d'ingrédients actifs en combinaison avec un support pharmaceutique. Cette dose unitaire peut être administrée 1 à 5 fois par jour de façon à administrer un dosage journalier de 0,5 à 5000 mg, de préférence de 1 à 2500 mg.

Lorsqu'on prépare une composition solide sous forme de comprimés, on mélange l'ingrédient actif principal avec un véhicule pharmaceutique tel que la gélatine, l'amidon, le lactose, la stéarate de magnésium, le talc, la gomme arabique ou analogues. On peut enrober les comprimés de saccharose, d'une dérivé cellulosique, ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir ou pour l'administration sous forme de gouttes peut contenir l'ingrédient actif conjointement avec un édulcorant acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les compositions de la présente invention peuvent contenir, à côté des produits de formule I ci-dessus ou d'un des sels pharmaceutiquement acceptables, d'autres principes actifs qui peuvent être utiles dans le traitement des troubles ou maladies indiquées ci-dessus.

Ainsi, la présente invention a pour objet des compositions pharmaceutiques contenant plusieurs principes actifs en association dont l'un est un composé selon l'invention.

Les exemples suivants illustrent l'invention.

Les composés sont caractérisés par leur point de fusion (F°C) (ou leur point d'ébullition Eb) et/ou leur spectre RMN enregistré à 200 mHz dans le DMSO, et/ou leur pouvoir rotaire (αD) mesuré à 25°C (sauf indication contraire).

La valeur du pouvoir rotatoire mesurée est dépendante de la quantité de solvant résiduel présente dans le produit préparé.

Sauf indication contraire, l'appellation "isomère cis" ou "isomère trans" signifie que le composé isolé est un mélange d'énantiomères, soit de configuration cis, soit de configuration trans.

La pureté optique des composés est étudiée par chromatographie liquide à haute performance (CLHP).

### EXEMPLE 1

### N-méthyl N-méthoxycarbonylméthyl bromo-5 (fluoro-2 phényl)-3 (diméthoxy-3,4 phénylsulfonyl)-1 hydroxy-3 indoline-2 carboxamide, isomère cis.

### A) N-bromoacétyl sarcosinate de méthyle.

Ce composé est préparé selon T.D. Harris et al. dans J. Heterocyclic Chem., 1981, 18, 423.

### B) bromo-5 (diméthoxy-3,4 phénylsulfonamido)-2 fluoro-2' benzophénone.

On chauffe à 85°C pendant 48 heures dans 120 ml de pyridine sèche 20 g d'amino-2 bromo-5 fluoro-2' benzophénone en présence de 20 g de chlorure de diméthoxy-3,4 phénylsulfonyle. On refroidit, verse dans de l'eau glacée, filtre le solide, extrait le solide par AcOEt, lave la phase organique par de l'eau, une solution d'acide chlorhydrique (1N), de l'eau, puis de l'eau saline. Après séchage sur sulfate de magnésium et évaporation du solvant sous vide, on obtient un solide qui est recristallisé dans DCM/éther iso.
m = 28 g
Fc = 125-128 °C.

### C) bromo-5 [N-(diméthoxy-3,4 phénylsulfonyl) N-(N'-méthyl N'-méthoxycarbonylcarbamoylméthyl)] amino-2 fluoro-2' benzophénone.

On dissout dans le DMF anhydre à 0°C sous argon, 3,5 g du composé préparé à l'étape B et l'on ajoute 250 mg d'hydrure de sodium à 80 % ; après 15 minutes, on ajoute 4,85 g du composé préparé à l'étape A et on laisse sous agitation à TA pendant 12 heures. On verse le milieu réactionnel sur de l'eau, filtre le solide, puis on dissout le solide dans AcOEt, lave la phase organique par de l'eau puis par de l'eau saline et évapore le solvant sous vide. On filtre l'huile obtenue sur silice en éluant par un mélange DCM/AcEOt (85/15 ; v/v). On recristallise dans un mélange DCM/éther iso/MeOH.
m = 3,2 g
Fc = 136-137°C.

### D) N-méthyl N-méthoxycarbonylméthyl bromo-5 (fluoro-2 phényl)-3 (diméthoxy-3,4 phénylsulfonyl)-1 hydroxy-3 indoline-2 carboxamide, isomère cis.

3,2 g de produit obtenu à l'étape précédente sont dissous dans DCM (3ml), on ajoute 750 mg de DBU et laisse sous agitation à TA pendant 24 heures. Le milieu réactionnel est versé sur une colonne de silice ; en éluant par DCM/AcOEt (90/10; v/v) on obtient un produit qui est le mélange des 2 isomères (cis et trans) du composé attendu. Ce produit est trituré dans un mélange hexane-éther iso et on filtre le solide obtenu. Les jus de filtration sont chromatographiés sur une colonne d'alumine prééquilibrée dans un mélange DCM/AcOEt (70/30 ; v/v). On élue le composé le moins polaire par un mélange DCM/AcOEt (60/40 ; v/v) puis on le recristallise dans le mélange DCM/hexane/éther iso.
Fc = 95°C avec dégagement gazeux.

### EXEMPLES 2 et 3

### [(benzyloxycarbonyl-4) pipérazin-1-yl]-carbonyl-2 chloro-5 (chloro-2 phényl)-3 (diméthoxy-3,4 phénylsulfonyl)-1 hydroxy-3 indoline, isomère cis et isomère trans.

### A) bromacétyl-1 (benzyloxycarbonyl)-4 pipérazine.

On refroidit à 0°C un mélange de 22 g de benzyloxycarbonyl-4 pipérazine et de 10,1 g de triéthylamine dans 200 ml d'éther. On ajoute en 30 minutes 20,2 g de bromure de bromacétyle dans 100 ml d'éther et on laisse revenir à TA. Après 4 heures, le milieu réactionnel est lavé à l'eau, séché, concentré puis on chromatographie sur silice. Le mélange DCM/AcOEt (95/5 ; v/v) élue le composé attendu qui est recristallisé dans DCM/éther iso.
m = 9 g
Fc = 100-101°C.

### B) dichloro-2',5 (diméthoxy-3,4 phénylsulfomanido)-2 benzophénone.

On chauffe dans la pyridine pendant une nuit à 100°C, 5,6 g d'amino-2 dichloro-2',5 benzophénone et 5 g de chlorure de diméthoxy-3,4 phénylsulfonyle. On évapore la pyridine à siccité, ajoute de l'eau, et extrait par de l'acétate d'éthyle contenant un peu de DCM. Après lavages à l'eau et séchage sur sulfate de sodium, on évapore sous vide et recristallise 7,7 g du produit attendu dans le mélange DCM/AcOEt.
Fc = 164°C.

### C) dichloro-2,,5 [N-(diméthoxy-3,4 phénylsulfonyl)-N-(benzyloxycarbonyl-4 pipérazin-1-yl carbonylméthyl)] amino-2 benzophénone.

2,3 g de la benzophénone préparé à l'étape B sont placés dans 10 ml de DMF et traités par 200 mg d'hydrure de sodium à 80 % dans l'huile. Après 30 minutes, on ajoute 5,3 g du composé préparé à l'étape A et l'on agite 60 heures à TA. On verse le milieu sur de l'eau, filtre le précipité, on le reprend par DCM, on sèche puis concentre et chromatographie sur silice. Le mélange DCM/AcOEt (90/10 ; v/v) élue le produit attendu qui cristallise dans le mélange DCM/éther iso.
m = 2 g
Fc = 173- 175°C.

### D) [(benzyloxycarbonyl-4) pipérazin-1-yl]-carbonyl-2 chloro-5 (chloro-2 phényl)-3 (diméthoxy-3,4 phénylsulfonyl)-1 hydroxy-3 indoline, isomère cis et isomère trans.

1 g du composé obtenu à l'étape précédente est mis en suspension dans 20 ml de méthanol et 20 ml de THF et traité par 75 mg de méthylate de sodium. Après 2 heures, on neutralise par addition d'un peu de carboglace, concentre à sec puis reprend par de l'eau ; on extrait ensuite par du DCM, sèche et concentre. Le produit brut est chromatographié sur alumine, le mélange DCM/AcOEt (80/20 ; v/v) élue succéssivement les 2 isomères.

L'isomère le moins polaire est recristallisé du mélange DCM/hexane. Ce composé est l'isomère cis.
m = 262 mg
Fc = 169-179°C.

L'isomère le plus polaire est recristallisé du mélange DCM/éther iso.
m = 200 mg
Fc = 209-211°C.

### EXEMPLE 4

### Chloro-5 (chloro-2 phényl)-3 (diméthoxy-3,4 phénylsulfonyl)-1 hydroxy-3 (pipérazin-1-ylcarbonyl)-2 indoline, isomère cis.

200 mg de l'isomère cis préparé à l'exemple précédent sont mis en solution dans 10 ml d'éthanol et 5 ml de THF et hydrogénolysé à TA en présence de Pd/C à 10 %.Après 30 minutes, on filtre le milieu sur Célite ®, concentre les jus de filtration puis on chromatographie sur silice. Le mélange MeOH/DCM (10/90 ; v/v) élue le produit attendu que l'on recristallise du mélange DCM/éther iso.
m = 110 mg
Fc = 230-233°C.

### EXEMPLES 5 et 6

### Chloro-5 (chloro-2 phényl)-3 (diméthoxy-3,4 phénylsulfonyl)-1 hydroxy-3 morpholinocarbonyl-2 indoline, isomère cis et isomère trans.

### A) dichloro-2',5 [N-diméthoxy-3,4 phénylsulfonyl-N-(morpholinocarbonylméthyl)] amino-2 benzophénone.

5 g de dichloro-2',5 (diméthoxy-3,4 phénylsulfonamido)-2 benzophénone sont traités par 350 mg d'hydrure de sodium à 80 % dans 30 ml de DMF à TA, pendant 20 minutes. On ajoute 4,5 g de bromacétamide de morpholine puis on agite à TA pendant 48 heures. Le milieu est versé sur de l'eau, le précipité filtré, on le dissout dans DCM, sèche et concentre. On recristallise le produit formé dans le mélange DCM/éther iso. On obtient 5,4 g.
Fc = 173-176°C.

### B) Chloro-5 (chloro-2 phényl)-3 (diméthoxy-3,4 phénylsulfonyl)-1 hydroxy-3 morpholinocarbonyl-2 indoline, isomère cis.

1 g du produit obtenu à l'étape précédente est solubilisé dans le mélange méthanol (10 ml) THF (20 ml) et traité par 92 mg de méthylate de sodium à TA pendant 1 heure. On neutralise le milieu par de la carboglace, évapore en partie les solvants, reprend par de l'eau, extrait au DCM, sèche, concentre et chromatographie sur alumine. Le mélange DCM/AcOEt (70/30 ; v/v) élue l'isomère le moins polaire qui est recristallisé du mélange DCM/éther iso.
m = 215 mg : isomère cis
Fc = 260-264°C.

### C) Chloro-5 (chloro-2 phényl)-3 (diméthoxy-3,4 phénylsulfonyl)-1 hydroxy-3 morpholinocarbonyl-2 indoline, isomère trans.

Par la chromatographie de l'étape précédente, on recueille un produit plus polaire en éluant par le mélange AcOEt/MeOH (90/10 ; v/v). Après recristallisation dans la mélange DCM/éther iso, on obtient :
m = 513 mg: isomère trans
Fc = 240-241°C.

### EXEMPLE 7

### N-méthyl N-carboxyméthyl bromo-5 (fluoro-2 phényl)-3 (diméthoxy-3,4 phénylsulfonyl)-1 hydroxy-3 indoline-2 carboxamide, isomère cis.

On dissout 200 mg du composé préparé à l'exemple 1 dans 3 ml de MeOH et 1 ml d'eau contenant 13 mg de soude. Après 24 heures sous agitation à TA, on ajoute une goutte de soude concentrée pour terminer la réaction, puis après 15 minutes on acidifie à pH 3 par addition d'une solution de sulfate acide de potassium. On ajoute de l'eau, extrait par AcOEt, lave à l'eau, sèche sur sulfate de magnésium et évapore le solvant sous vide. Le produit obtenu est recristallisé dans DCM/éther iso.
Fc = 206-208°C.

### EXEMPLES 8 et 9

### chloro-5 (chloro-2 phényl)-3 (diméthoxy-3,4 phénylsulfonyl)-1 hydroxy-3 (éthylcarboxylate-4 pipéridinocarbonyl)-2 indoline, isomère cis, isomère trans.

### A) N-bromoacétyl pipéridine-4 carboxylate d'éthyle.

Ce produit est préparé à partir de la pipéridine-4 carboxylate d'éthyle, commerciale.

### B) dichloro-2′,5 [N-(diméthoxy-3,4 phénylsulfonyl) N-(éthylcarboxylate-4 pipéridinocarbonylméthyl)] amino-2 benzophénone.

On dissout 8 g de dichloro-2′,5 (diméthoxy-3,4 phénylsulfonamido)-2 benzophénone dans 100 ml de DMF, puis on ajoute 541 mg d'hydrure de sodium. Après 30 minutes d'agitation, on ajoute 9,5 g du composé de l'étape A et on laisse sous agitation 18 heures à TA. On concentre sous vide, reprend par de l'eau, extrait par de l'acétate d'éthyle, sèche et concentre. L'huile obtenue est chromatographiée sur silice en éluant par le mélange AcOEt/DCM/hexane (40/10/50 ; v/v/v). Le produit attendu cristallise dans l'éther.
m = 3,5 g
Fc = 128°C.

### C) chloro-5 (chloro-2 phényl)-3 (diméthoxy-3,4 phénylsulfonyl)-1 hydroxy-3 (éthylcarboxylate-4 pipéridinocarbonyl)-2 indoline, isomère cis, isomère trans.

On porte à 60°C pendant 18 heures un mélange contenant 3,4 g du composé préparé à l'étape précédente et 869 mg de DBU dans 10 ml de chloroforme. Le milieu réactionnel est ensuite filtré sur colonne d'alumine en éluant par le mélange DCM/AcOEt (90/10 ; v/v) pour obtenir l'isomère cis.
m = 700 mg
Fc = 110°C.

L'acétate d'éthyle pur élue l'isomère trans.
m = 610 mg
Fc = 187°C.

### EXEMPLES 10 et 11

### N-méthyl N-(pyrid-2-yl éthyl) chloro-5 (chloro-2 phényl)-3 (diméthoxy-3,4 phénylsulfonyl)-1 hydroxy-3 indoline-2 carboxamide, isomère cis, isomère trans.

### A) Acide N-[(chloro-2 phénylcarbonyl)-2 chloro-5 phényl] N-(diméthoxy-3,4 phénylsulfonyl) glycine.

a) dichloro-2′,5 (diméthoxy-3,4 phénylsulfonamido)-2 benzophénone.
   Ce composé est préparé à l'exemple 2-3, étape B.
b) dichloro-2′,5 [N-(diméthoxy-3,4 phénylsulfonyl) N-benzyloxyacarbonylméthyl] amino-2 benzophénone.
   172 g du produit préparé précédemment est dissous dans 800 ml de DCM et refroidi à 0°C. On ajoute progressivement sous azote 11,7 g d'hydrure de sodium à 80 % puis, après 30 minutes, on ajoute 256 g de bromoacétate de benzyle et on laisse 24 heures sous agitation à TA. On évapore le DMF, reprend par de l'eau, extrait au DCM, sèche et concentre. Le produit attendu cristallise dans l'éther iso puis on le recristallise dans le mélange DCM/éther iso. m = 136,5 g Fc = 102-104°C.
c) Acide N-[(chloro-2 phénylcarbonyl)-2 chloro-5 phényl] N-(diméthoxy-3,4 phénylsulfonyl) glycine.
   On dissout 50 g de l'ester benzylique obtenu précédemment dans 500 ml d'AcOEt et on ajoute, sous azote, 2,5 g de Pd/C à 5 %. La solution est agitée fortement et l'on fait passer un courant d'hydrogène pendant 5 heures. A la fin de l'hydrogénation, le produit cristallise. On filtre le milieu sur Célite ®, lave abondamment le gateau par du DCM chaud puis on concentre la phase organique. Le produit attendu cristallise puis on le recristallise dans le mélange DCM/éther iso.
   m = 33,7 g
   Fc = 177-178°C.

### B) dichloro-2′,5 [N-(diméthoxy-3,4 phénylsulfonyl) N-(N′-pyrid-2-yl-2 éthyl N′-méthyl) carbamoyl méthyl] amino-2 benzophénone.

2 g de l'acide préparé à l'étape A sont placés dans 30 ml de DCM et l'on ajoute 1,13 g de (méthylamino-2 éthyl)-2 pyridine puis 844 mg de triéthylamine et enfin 1,92 g de BOP puis on laisse sous agitation pendant 18 heures à TA. On reprend par de l'eau, décante la phase organique, lave par une solution de carbonate de sodium, sèche et concentre. Après chromatographie sur silice, on recueille le produit attendu en éluant par le mélange DCM/MeOH (95/5 ; v/v).
m = 2 g
Fc = 150°C

### C) N-méthyl N-(pyrid-2-yl éthyl) chloro-5 (chloro-2 phényl)-3 (diméthoxy-3,4 phénylsulfonyl)-1 hydroxy-3 indoline-2 carboxamide.

On chauffe à 55°C pendant 18 heures un mélange contenant 1,7 g du produit obtenu à l'étape précédente et 442 mg de DBU dans DCM. On chromatographie le milieu réactionnel sur alumine. Le mélange AcOEt/DCM (40/60 ; v/v) élue l'isomère cis :
m = 410 mg
Fc = 191°C.

AcOEt pur élue l'isomère trans :
m = 790 mg
Fc = 154°C.

### EXEMPLE 12

### (carboxy-4 pipéridinocarbonyl)-2 chloro-5 (chloro-2 phényl)-3 (diméthoxy-3,4 phénylsulfonyl)-1 hydroxy-3 indoline, isomère cis.

500 mg de l'isomérie cis préparé à l'exemple 9 sont placés dans 5 ml de méthanol en présence de 48 mg de soude dans 1 ml d'eau. Après 18 heures d'agitation, on verse sur de l'eau, acidifie par de l'acide chlorhydrique dilué puis on extrait au DCM, sèche et concentre. Le solide obtenu est purifié par chromatographie sur silice en éluant par le mélange DCM/MeOH (95/5 ; v/v), puis on cristallise le produit obtenu du mélange DCM/éther iso.
m = 250 mg
Fc = 150°C.

### EXEMPLES 13 et 14

### N-méthyl N-(méthyl-1 pipéridin-4-yl) chloro-5 (chloro-2 phényl)-3 (diméthoxy-3,4 phénylsulfonyl)-1 hydroxy-3 indoline-2 carboxamide, isomère cis et isomère trans.

### A) dichloro-2′,5 [N-(diméthoxy-3,4 phénylsulfonyl) N-(N′-méthyl N′-méthylpipéridin-4-yl) carbamoylméthyl] amino-2 benzophénone.

2 g de l'acide préparé à l'exemple 10-11, étape A dans 50 ml de DCM sont mélangés à 650 mg de méthylamino-4 méthyl-1 pipéridine en présence de 1,90 g de BOP. Après 2 heures sous agitation à TA, la phase organique est lavée à l'eau carbonatée, séchée et concentrée. Puis on chromatographie sur silice en éluant par le mélange DCM/MeOH (90/10 ; v/v). On obtient 1,2 g du produit attendu.
Fc = 165-166°C.

### B) N-méthyl N-(méthylpipéridin-4-yl) chloro-5 (chloro-2 phényl)-3 (diméthoxy-3,4 phénylsulfonyl)-1 hydroxy-3 indoline-2 carboxamide, isomère cis et isomère trans.

650 mg du produit obtenu à l'étape précédente sont traités pendant une nuit par 100 mg de méthylate de sodium dans 5 ml de méthanol. On ajoute de la carboglace, évapore le solvant, reprend par de l'eau carbonatée, extrait au DCM, sèche et concentre puis on chromatographie sur silice. Le mélange méthanol/DCM (5/95 ; v/v) élue successivement les 2 isomères. Chacun est ensuite recristallisé du mélange DCM/éther iso.
isomère trans le moins polaire, dans ces conditions,
   m = 205 mg
   Fc = 181°C.
isomère cis :
   m = 150 mg
   Fc = 97°C : contient 0,25 M d'éther iso.

### EXEMPLES 15 et 16

### chloro-5 (chloro-2 phényl)-3 (diméthoxy-3,4 phénylsulfonyl)-1 hydroxy-3 [(méthyl-4) pipérazin-1-yl carbonyl]-2 indoline, isomère cis et isomère trans.

### A) dichloro-2′,5 [N-(diméthoxy-3,4 phénylsulfonyl) N-((méthyl-4 pipérazin-1-yl) carbamoylméthyl)] amino-2 benzophénone.

Ce composé est obtenu par action de la N-méthyl pipérazine sur l'acide préparé à l'exemple 10-11 étape A.
Fc = 165-167°C.

### B) chloro-5 (chloro-2 phényl)-3 (diméthoxy-3,4 phénylsulfonyl)-1 hydroxy-3 [(méthyl-4) pipérazin-1-yl carbonyl]-2 indoline, isomère cis et isomère trans.

Le composé de l'étape précédente est cyclisé en procédant comme à l'exemple 12-13. Les 2 isomères formés sont séparés par chromatographie sur alumine. Le mélange DCM/AcOEt (75/25 ; v/v) élue le produit le moins polaire : l'isomère cis, qui est recristallisé du mélange DCM/éther iso.
Fc = 120°C: contient 0,25 M d'éther iso.

Le mélange DCM/MeOH élue le composé le plus polaire, l'isomère trans qui est ensuite recristallisé du méthanol.
Fc = 189°C.

### EXEMPLES 17 et 18

### N-isopropyl N-méthoxycarbonyléthyl chloro-5 (chloro-2 phényl)-3 (diméthoxy-3,4 phénylsulfonyl)-1 hydroxy-3 indoline-2 carboxamide, isomère cis et isomère trans.

### A) N-isopropyl N-méthoxycarbonyléthyl bromoacétamide.

On ajoute goutte à goutte 90 g d'isopropylamine sur 130 g de solution refroidie à -10°C d'acrylate de méthyle dans 300 ml de méthanol. Après 72 heures à TA, on évapore le milieu puis on distille le résidu. L'huile obtenue (168,3 g) est le (N-isopropyl) amino-3 propionate de méthyle.
Eb = 73-78°C sous 15 mm Hg.

A 0°C, on mélange 29 g du composé obtenu dans 100 ml de DCM à 20,2 g de bromure de bromacétyle dans 100 ml de DCM. Après 12 heures à TA, on évapore le solvant, reprend par de l'eau, extrait à l'acétate d'éthyle, sèche et concentre. L'huile obtenue est utilisée telle quelle à l'étape suivante

### B) Dichloro-2′,5 [ N-(diméthoxy-3,4 phénylsulfonyl) N-(N′-isopropyl N′-méthoxycarbonyléthyl) carbamoylméthyl] amino-2 benzophénone.

Ce composé est obtenu en suivant le mode opératoire habituel, par action du produit préparé à l'étape A sur le dichloro-2′,5 (diméthoxy-3,4 phénylsulfonamido)-2 benzophénone en présence d'hydrure de sodium.
Fc = 135-137°C (recristallisation : DCM/éther iso).

### C) N-isopropyl N-méthoxycarbonyléthyl chloro-5 (chloro-2 phényl)-3 (diméthoxy-3,4 phénylsulfonyl)-1 hydroxy-3 indoline-2 carboxamide, isomère cis et isomère trans.

Ce produit est obtenu par cyclisation du composé préparé à l'étape B en présence de DBU. L'isomère cis est séparé par chromatographie sur alumine en éluant par le mélange DCM/AcOEt (90/10 ; v/v). On cristallise ensuite le produit dans le mélange AcOEt/hexane.
FC = 153-155°C.

L'isomère trans est obtenu en éluant la colonne d'alumine par l'acétate d'éthyle. On recristallise ensuite le produit dans le mélange méthanol/éther iso.
Fc = 182-185°C.

### EXEMPLES 19 et 20

### N-méthyl N-méthoxycarbonylméthyl chloro-5 (chloro-2 phényl)-3 (diméthoxy-3,4 phénylsulfonyl)-1 hydroxy-3 indoline-2 carboxamide, isomère cis et isomère trans.

Les 2 isomères de ce composé sont préparés selon le mode opératoire décrit à l'exemple 1. Ils sont séparés par chromatographie sur alumine. Le mélange DCM/AcOEt (80/20 ; v/v) élue l'isomérie cis. Celui-ci cristallise du mélange DCM/éther iso, sous forme d'une poudre blanche contenant 0,25 mole d'éther iso. Il se transforme en mousse par chauffage sous vide.

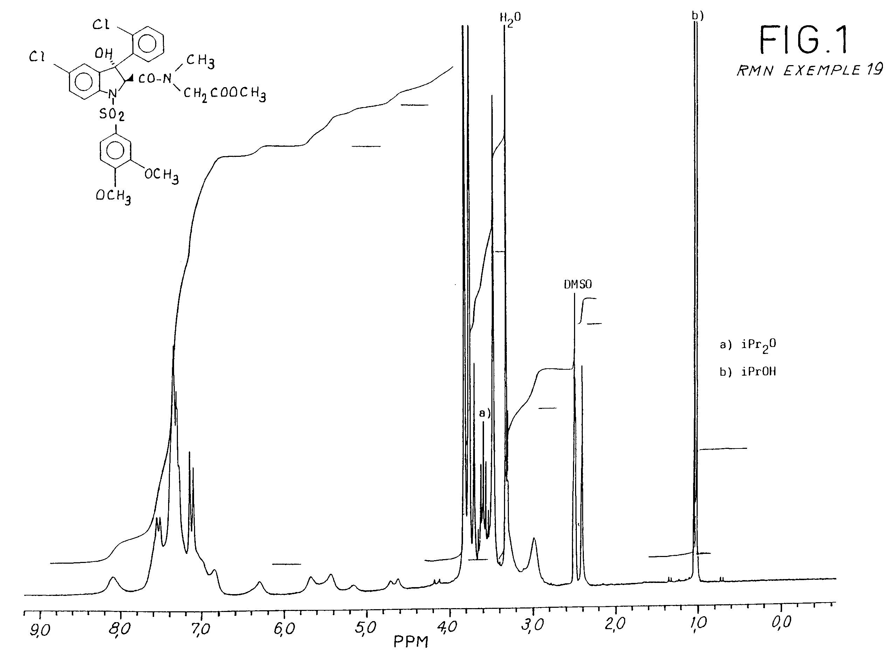

Le spectre RMN de l'isomère cis (exemple 19) est donné en Figure 1.

L'isomère trans est élué par AcOEt pur. On le recristallise dans DCM/éther iso
Fc = 176-178°C.

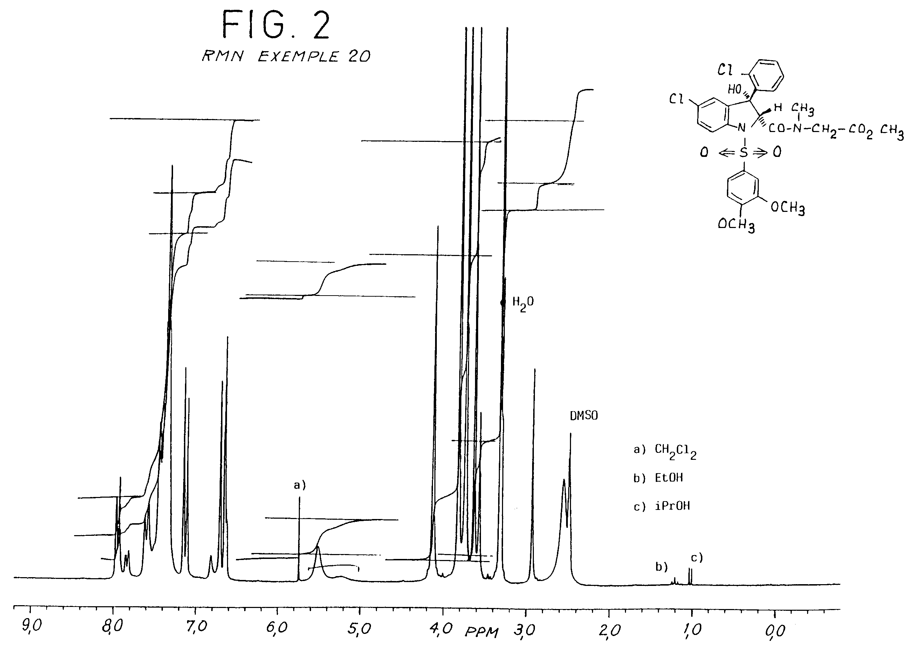

Le spectre RMN de l'isomère trans (exemple 20) est donné en Figure 2.

### EXEMPLES 21 et 22

### N-méthyl N-carboxyméthyl chloro-5 (chloro-2 phényl)-3 (diméthoxy-3,4 phényl sulfonyl)-1 hydroxy-3 indoline-2 carboxamide, isomère cis et isomère trans.

Ces composés sont préparés chacun à partir des composés décrits aux exemples 19 et 20, selon le mode opératoire décrit à l'exemple 8.
isomère cis : Fc = 220-222°C après recristallisation dans le mélange DCM/éther iso/MeOH.
isomère trans : Fc = 222-225°C après recristallisation dans le mélange DCM/éther iso.

### EXEMPLES 23 et 24

### N-méthyl N-carbamoylméthyl chloro-5 (chloro-2 phényl)-3 (diméthoxy-3,4 phénylsulfonyl)-1 hydroxy-3 indoline-2 carboxamide, isomère cis et isomère trans.

Chaque isomère est obtenu à partir de l'isomère correspondant de l'acide préparé à l'exemple 21-22.

605 mg de l'isomère trans de l'acide obtenu à l'exemple précédent, sont dissous dans 10 ml de DCM, on ajoute 435 mg de BOP et 260 mg de DIPEA. Après 5 minutes à TA, on ajoute sous forte agitation 6 ml de solution d'ammoniaque à 20 % et on laisse 4 heures sous agitation. On ajoute une solution de carbonate de sodium, puis on extrait par DCM. On lave la phase organique successivement par de l'eau, une solution de sulfate acide de sodium, de l'eau, puis on sèche sur sulfate de magnésium. Après évaporation, le résidu est chromatographié sur gel de silice et on élue par un mélange AcOet/MeOH (95/5 ; v/v). Le produit obtenu est cristallisé 2 fois dans le mélange DCM/EtOH à 0°C.
Fc = 236°C.

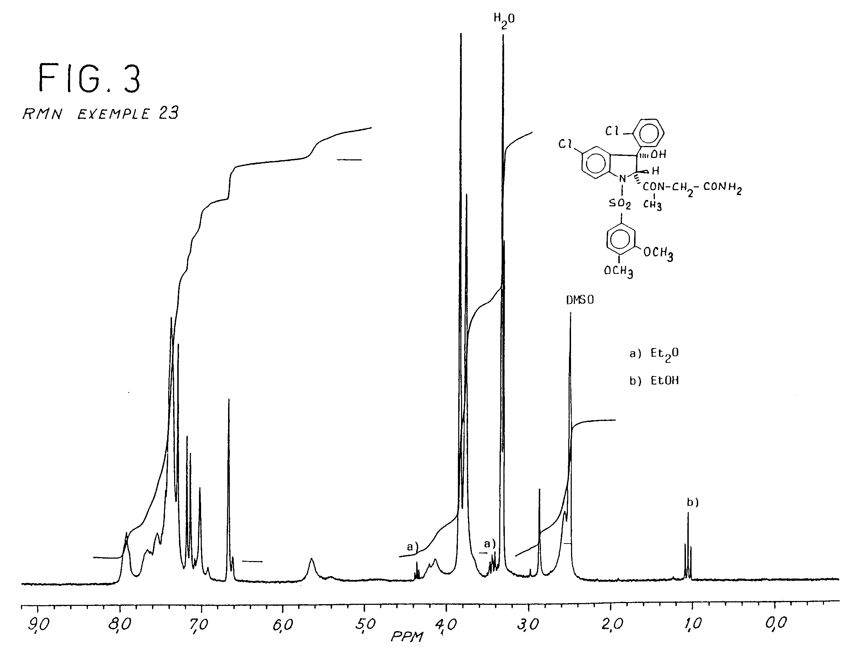

Le spectre RMN de l'isomère trans (exemple 23) est donné en Figure 3.

En utilisant le même mode opératoire, on prépare l'isomère cis.

Le produit attendu cristallise dans DCM/éther iso. Le composé micronisé, séché sous vide à 70°C pendant 8 heures, contient 0,25 mole d'éther iso.

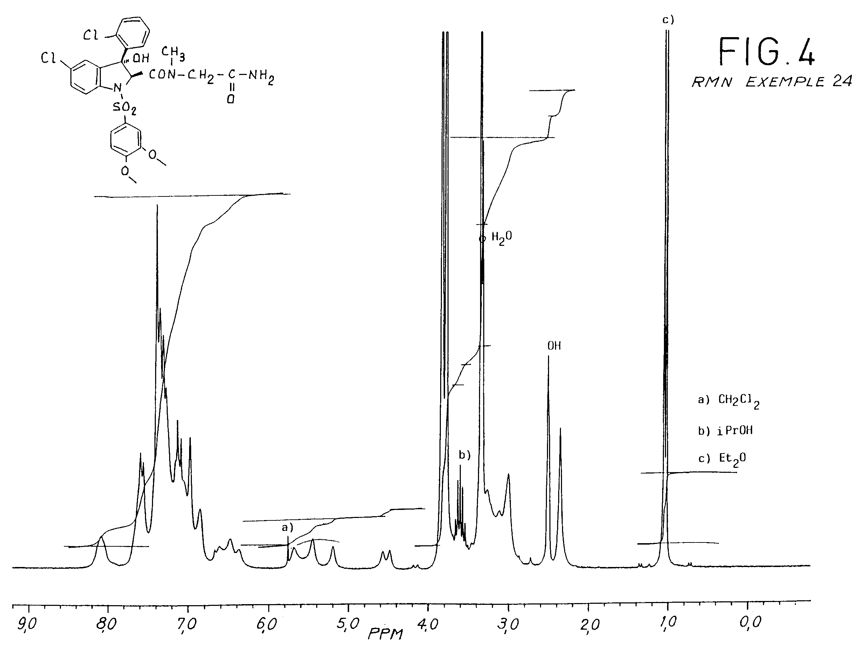

Le spectre RMN de l'isomère cis (exemple 24) est donné en Figure 4.

### EXEMPLES 25 et 26

### chloro-5 (chloro-2 phényl)-3 (diméthoxy-3,4 phénylsulfonyl)-1 hydroxy-3 (hydroxy-4 pipéridin-1-yl) carbonyl-1 indoline, isomère trans.

Ce composé est préparé à partir de l'acide N-[(chloro-2 phénylcarbonyl)-2 chloro-5 phényl] N-(diméthoxy-3,4 phénylsulfonyl) glycine décrit à l'exemple 11-12, étape A.

On procède ensuite comme dans l'exemple 11-12 pour l'addition de l'hydroxy-4 pipéridine, en présence de BOP et de triéthylamine. Le produit obtenu est ensuite cyclisé selon la méthode habituelle en présence de DBU. Les 2 isomères sont séparés par chromatographie sur alumine. Le mélange DCM/MeOH (99/1 ; v/v) élue l'isomère cis.

Le produit cristallise dans le mélange DCM/hexane/MeOH puis le solide obtenu est trituré dans DCM/hexane pour fournir une poudre amorphe.

L'isomère cis est caractérisé par son spectre RMN à 388°K.
1-1,8 ppm : m : 4H: C**H**₂ en 3 et 5 de la pipéridine
2,8-3,65 ppm : m : 5H: CH₂ en 2 et 6 de la pipéridine et C**H** en 4
3,75 ppm: 2s: 6H: 2OC**H**₃
4,15 ppm: d: 1H: OH sur pipéridine
5,45 ppm : s : 1H : CH (indoline)
6,1 ppm: s: 1H: OH indoline
6,8-7,6 ppm : m : 10H : H aromatiques
DMSO: 2,4 ppm
DOH: 2,75 ppm

Le mélange DCM/MeOH (97/3 ; v/v) élue l'isomère trans qui est recristallisé dans DCM/éther iso.
Fc = 232-234°C.

### SYNTHESES dans la série (L) Proline: Exemples 27, 28, 29, 30.

### EXEMPLES 27 et 27 bis

### chloro-5 (chloro-2 phényl)-3 (diméthoxy-3,4 phénylsulfonyl)-1 hydroxy-3 [(2S)(méthoxycarbonyl-2) pyrrolidincarbonyl]-2 indoline, (isoméres cis : 2 composés).

### A)(L) N-(bromoacétyl) prolinate de méthyle.

A une solution de 16,7 g de chlorhydrate de (L) prolinate de méthyle dans 20 ml de DCM, on ajoute simultanément 20 g de triéthylamine et 20 g de bromure de bromacétyle dans 30 ml de DCM en maintenant la température à -5°C, puis on agite à TA pendant 24 heures. On ajoute de l'eau, lave par une solution de KHSO₄, par de l'eau, par une solution de bicarbonate de sodium, par de l'eau, puis on sèche sur sulfate de magnésium. Après évaporation on obtient une huile que l'on sèche sous vide. Cette huile, pure en CCM, est utilisé telle quelle à l'étape suivante.

### B) dichloro-2',5 [N-(diméthoxy-3,4 phénylsulfonyl) N-((2S)(méthoxycarbonyl-2) pyrrolidinocarbonylméthyl)] amino-2 benzophénone.

On dissout 4,66 g de dichloro-2',5 (diméthoxy-3,4 phénylsulfonamido)-2 benzophénone dans 40 ml de DMF anhydre sous argon, à 0°C, on ajoute 340 mg d'hydrure de sodium à 80 %, puis après 30 minutes, 6,5 g du composé obtenu à l'étape A. Après 4 jours à TA, on verse dans de l'eau, extrait par AcOEt, lave par de l'eau, de l'eau saline puis sèche sur sulfate de magnésium et évapore sous vide. On chromatographie sur gel de silice et élue par le mélange DCM/AcOEt (85/15 ; v/v) un solide contenant un peu de dérivé bromé de départ. Un échantillon est recristallisé dans DCM/éther iso.
m = 1,2 g
Fc = 141-142°C
α_{D}²⁵ = -43,7°(c = 1; MeOH/THF : 8/2 ; v/v)

| | | | | |
|---|---|---|---|---|
| analyse | calculé | C : 54,81 | H : 4,44 | N : 4,41 |
| | trouvé | 54,40 | 4,54 | 4,55 |

### C) chloro-5 (chloro-2 phényl)-3 (diméthoxy-3,4 phénylsulfonyl)-1 hydroxy-3 [(2S)(méthoxycarbonyl-2) pyrrolidinocarbonyl]-2 indoline, (isomérie cis).

On chauffe dans 4 ml de chlorure de méthylène pendant 24 heures, 1,1 g du composé obtenu à l'étape précédente avec un équivalent de DBU. L'analyse CLHP d'un aliquote montre l'existence des 4 isomères attendus. Après 24 heures, on verse le milieu réactionnel sur une colonne de l'alumine, prééquilibrée dans le mélange DCM/AcOEt (90/10 ; v/v) et on élue par le mélange DCM/AcOEt (90/10 ; v/v jusqu'à 70/30 ; v/v). On obtient 510 mg d'un mélange des 2 composés les moins polaires dans le rapport 4/1 (mesuré par HPLC).
1°) Deux cristallisations successives dans DCM/éther iso à froid fournissent un composé majoritaire.
   m = 180 mg
   α_{D}²⁵ = -247°(c = 0,4 ; chloroforme)
   Fc = 187-190°C.
2°) Les eaux-mères de cristallistion du composé précédent sont chromatographiées sur alumine en éluant par DCM/AcOEt (85/15 ; v/v). On sépare ainsi le composé précédent du second, ce dernier est dissous dans un minimum de DCM puis il précipite par addition d'un minimum d'hexane.
   α_{D}²⁶ = +136 (c = 0,24 ; chloroforme)

### EXEMPLE 28

### ((2S)carboxy-2 pyrrolidinocarbonyl)-2 chloro-5 (chloro-2 phényl)-3 (diméthoxy-3,4 phénylsulfonyl)-1 hydroxy-3 indoline, isomère cis.

On dissout 430 mg du composé préparé à l'exemple 27 dans 6 ml de méthanol, on ajoute 41 mg de soude dans 1 ml d'eau et agite pendant 24 heures à TA. On acidifie à pH 3 par quelques gouttes d'une solution de sulfate acide de potassium, et extrait par de l'acétate d'éthyle. On lave par de l'eau puis sèche sur sulfate de magnésium. On chromatographie sur une colonne de silice préparée dans le mélange DCM/pentane (80/20 ; v/v). L'ester (composé de l'exemple 27) qui n'a pas réagi est élué par le mélange DCM/AcOEt (70/30 ; v/v). Le mélange AcOEt/MeOH (80/20 ; v/v) élue l'acide attendu qui est ensuite recristallisé dans DCM/éther iso.
Fc = 232-234°C
α_{D}²⁶ = -254°(c = 0,3 ; chloroforme).

### EXEMPLES 29 et 29 bis

### ((2S)carbamoyl-2 pyrrolidinocarbonyl)-2 chloro-5 (chloro-2 phényl)-3 (diméthoxy-3,4 phénylsulfonyl)-1 hydroxy-3 indoline, (isomères cis : 2 composés).

On dissout 230 mg du composé préparé à l'exemple 28 dans 5 ml de DCM, on ajoute 50 mg de DIPEA puis 165 mg de BOP et laisse 5 minutes à TA. On refroidit le milieu dans un bain de glace puis l'on fait barbotter un courant d'ammoniac gazeux pendant 1 minute, et après 15 minutes, à nouveau pendant 1 minute. On ajoute de l'eau puis un grand volume d'acétate d'éthyle pour obtenir 2 phases. On lave la solution organique par une solution de carbonate de sodium, de l'eau, une solution de sulfate acide de potassium, de l'eau puis de l'eau saline. Après séchage, on chromatographie sur silice en éluant par le mélange DCM/MeOH (93/7 ; v/v). Le produit obtenu est trituré dans le mélange DCM/éther iso/hexane. Il contient 1/3 mole d'éther iso.
α_{D}²⁶ = -189 °(c = 0,23 ; chloroforme).

Le composé de l'exemple 29 peut être préparé selon un autre mode opératoire.

### A) Dichloro-2′,5 [N-(diméthoxy-3,4 phénylsulphonyl) N-((2S)carbamoyl-2 pyrrolidinocarbonylméthyl)] amino-2 benzophénone.

33,9 g de l'acide préparé à l'exemple 10-11, étape A sont dissous dans 300 ml de chloroforme. On ajoute 15 g de chlorure de thionyle et on porte à reflux pendant 1 heure et demie. On évapore à sec, puis on reprend le milieu par DCM et évapore à nouveau. On dissout le milieu dans 300 ml de DCM, porte à 0°C et ajoute 10,5 g de chlorhydrate de (L) prolinamide, puis on ajoute lentement 18 g de DIPEA dans 20 ml de DCM, sans laisser la température du milieu réactionnel dépasser 3°C. Après une nuit à TA, on lave le milieu réactionnel par du bicarbonate de sodium (2 fois) puis du sulfate acide de potassium (2 fois) ; on sèche et concentre. Le brut obtenu est dissous dans un minimum de DCM et ajouté goutte à goutte dans de l'éther iso (1,21) en agitant. Après 2 heures d'agitation, le précipité obtenu est filtré puis séché sous vide pendant 6 heures à 60°C. On recueille 42 g.
α_{D}²⁵ = -40,8°(c = 1,007 ; chloroforme).

### B) ((2S)carbamoyl-2 pyrrolidinocarbonyl)-2 chloro-5 (chloro-2 phényl)-3 (diméthoxy-3,4 phénylsulfonyl)-1 hydroxy-3 indoline, (isoméres cis : 2 composés).

On dissout 5 g du produit préparé à l'étape précédente dans 50 ml de méthanol. On refroidit à -10°C ; on ajoute 1,35 g de DBU et maintient 60 heures à - 10°C. Un composé cristallise ; on le filtre (composé cis 1). Les eaux de cristallisation sont neutralisées par KHSO₄ et le milieu est évaporé à sec. On reprend par de l'eau, extrait 2 fois au DCM, sèche et concentre. Le brut obtenu est chromatographié sur silice en éluant par un mélange AcOEt/DCM (28/72 ; v/v). On recueille un mélange que l'on dissout dans le minimum de méthanol à chaud ; on filtre l'insoluble puis les jus sont placés une nuit à -4°C et le composé cis 2 cristallise.
m = 1,25 g
α_{D}²⁵ = - 196°(c = 0,351 ; chloroforme).

L'analyse du spectre RMN montre la présence d'une mole de MeOH par mole de produit. Une recristallisation du produit dans l'éthanol permet d'élimination du solvant dans les cristaux.
Fc = 154-162°C
α_{D}²⁵ = -204(c = 0,3; chloroforme)
α_{D}²⁵ = -131°(c = 0,27 ; chloroforme/méthanol : 8/2 : v/v)

Ce composé est identique, au solvant près, à celui préparé par le premier mode opératoire du présent exemple.

Le composé qui a cristallisé à l'étape B) ci-dessus, appelé composé cis 1, est recristallisé dans le méthanol.
Fc = 190°C
α_{D}=+ 115°(c=0,3 ; chloroforme).

### EXEMPLE 30

### chloro-5 (chloro-2 phényl)-3(diméthoxy-3,4 phénylsulfonyl)-1 hydroxy-3 [(2S)(hydroxyméthyl-2) pyrrolidinocarbonyl]-2 indoline, isomérés cis.

### dichloro-2′,5 [(N-(diméthoxy-3,4 phénylsulfonyl) N-((hydroxyméthyl-2) pyrrolidinocarbonylméthyl)] amino-2 benzophénone.

Ce composé est obtenu par action du (L) prolinol sur l'acide préparé à l'exemple 10-11, étape A, en suivant le mode opératoire habituel.

### chloro-5 (chloro-2 phényl)-3 (diméthoxy-3,4 phénylsulfonyl)-1 hydroxy-3 [(2S)(hydroxyméthyl-2) pyrrolodinocarbonyl]-2 indoline, isomère cis.

1,5 g du composé à l'étape précédente est cyclisé en présence de 380 mg de DBU dans 2 ml de DCM. Après 3 jours à TA, on ajoute 1 ml de DCM, puis on chauffe à 40°C pendant une nuit. On observe la déformation de 3 composés majoritaires en CCM sur silice (éluant AcOEt).

Par chromatographie sur silice, on élue la fraction la moins polaire par DCM/AcOEt (60/40 à 80/20 ; v/v). On effectue ensuite une chromatographie sur alumine en éluant par DCM/MeOH(99/1 ; v/v). La fraction obtenue est homogène en CCM. Le produit est recristallisé trois fois dans DCM/éther iso. On obtient le produit attendu avec une pureté CLHP>99%.
m = 155 mg
Fc = 194-197°C
α_{D}²⁵=-195° (c=0,2 ; chloroforme).

### SYNTHESES dans la série (D) Proline: Exemple 31.

### EXEMPLE 31

### chloro-5 (chloro-2 phényl)-3 (diméthoxy-3,4 phénylsulfonyl)-1 hydroxy-3 [(2R)(méthoxycarbonyl-2) pyrrolidinocarbonyl]-2 indoline, isomère cis.

### A) dichloro-2′,5 [N-(diméthoxy-3,4 phénylsulfonyl) N-((2R)(méthoxycarbonyl-2) pyrrolidinocarbonylméthyl)] amino-2 benzophénone.

Ce composé est obtenu à partir de l'acide préparé à l'exemple 10-11, étape A (3 g) auquel on ajoute 1,2 g de (D) prolinate de méthyle et 2,8 g de BOP dans 10 ml de DCM en présence de 1,15 g de triéthylamine. On laisse 1 heure à TA puis on dilue par du DCM, lave la phase organique au carbonate de sodium, à l'hydrogénosulfate de potassium, sèche et concentre. Le brut est chromatographié sur silice en éluant par un mélange DCM/AcOEt (95/5 ; v/v). Le produit obtenu est ensuite recristallisé dans la mélange DCM/éther iso.
Fc = 140-141°C.
α_{D}²⁵ = +28,5°(c = 0,27 ; chloroforme).

### B) chloro-5 (chloro-2 phényl)-3 (diméthoxy-3,4 phénylsulfonyl)-1 hydroxy-3 [(2R)(méthoxycarbonyl-2) pyrrolidinocarbonyl]-2 indoline, isomère cis.

1,5 g du composé précédent est porté à reflux dans 5 ml de DCM en présence de 360 mg de DBU pendant une nuit. Le milieu est chromatographié sur alumine. Le mélange DCM/AcOEt (95/5 ; v/v) élue la fraction la moins polaire, (m = 300 mg) qui est recristallisée 2 fois dans le mélange DCM/éther iso.
Fc = 186-188°C
α_{D}²⁵ = +245°(c = 0,4, chloroforme)

Ce composé est l'énantiomère obtenu à partir de la (D) Proline de celui décrit à l'exemple 27.

### EXEMPLE 32 et 32 bis

### N-méthyl N-méthoxycarbonylméthyl chloro-5 (chloro-2 phényl)-3 (éthoxy-4 phénylsulfonyl)-1 hydroxy-3 indoline-2 carboxamide, isomère trans et isomère cis.

### A) dichloro-2′,5 [N-(éthoxy-4 phénylsulfonyl) N-(N′-méthyl N′-méthoxycarbonylméthyl)carbamoylméthyl] amino-2 benzophénone.

Dans 40 ml de DMF, on dissout sous argon, 5,7 g de dichloro-2′,5 éthoxy-4 phénylsulfonamido-2 benzophénone et on ajoute à 0°C, 400 mg d'hydrure de sodium à 80 % ; après 15 minutes, on ajoute 4,3 g de N-bromacétyl sarcosinate de méthyle. Après 48 heures, on extrait le produit attendu de la manière habituelle puis on le purifie par chromatographie sur silice en éluant par DCM/AcOEt (90/10 ; v/v) et recristallisation dans le mélange DCM/éther iso.
Fc = 158-160°C.

### B) N-méthyl N-méthoxycarbonylméthyl chloro-5 (chloro-2 phényl)-3 (éthoxy-4 phénylsulfonyl)-1 hydroxy-3 indoline-2 carboxamide, isomère trans.

1 g du composé obtenu à l'étape précédente est dissous dans 4 ml de DCM et traité pendant 90 minutes à TA par 312 mg de TBD. On ajoute une solution de sulfate acide de potassium, évapore le DCM sous vide, extrait par AcOEt, lave et sèche sur sulfate de magnésium. Une chromatographie sur gel de silice en éluant par DCM/AcOEt (90/10 ; v/v) fournit le produit attendu.
m = 590 mg
Fc = 168-171°C après recristallisation dans DCM/hexane.

### C) N-méthyl N-méthoxycarbonylméthyl chloro-5 (chloro-2 phényl)-3 (éthoxy-4 phénylsulfonyl)-1 hydroxy-3 indoline-2 carboxamide, isomère cis.

On place en suspension 2,96 g du composé obtenu à l'étape A dans 20 ml de méthanol et 10 ml de THF ; on ajoute 100 mg de méthylate de sodium puis on laisse 7 heures au réfrigérateur. On ajoute de l'eau, neutralise par une solution de sulfate acide de potassium et évapore une partie du méthanol sous vide. Après extraction par AcOEt, on chromatographie sur alumine puis on élue par un mélange DCM/AcOEt (80/20 ; v/v). On obtient 850 mg du produit attendu que l'on recristallise dans un mélange DCM/éther iso.

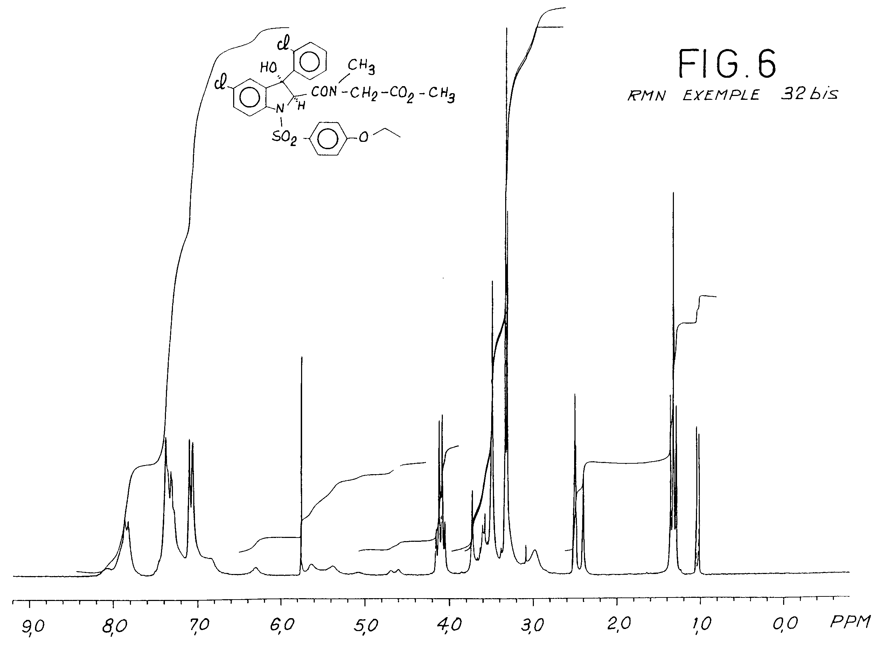

Le spectre RMN est donné en figure 6.

En utilisant des procédés semblables à ceux décrits ci-dessus, on a préparé des composés (VI) intermédiaires pour la synthèse de composés (I) selon l'invention.

Les composés (VI) préparés sont décrits dans le tableau 1 ci-après.

Les composés (I) préparés sont décrits dans le tableau 2 ci-après.

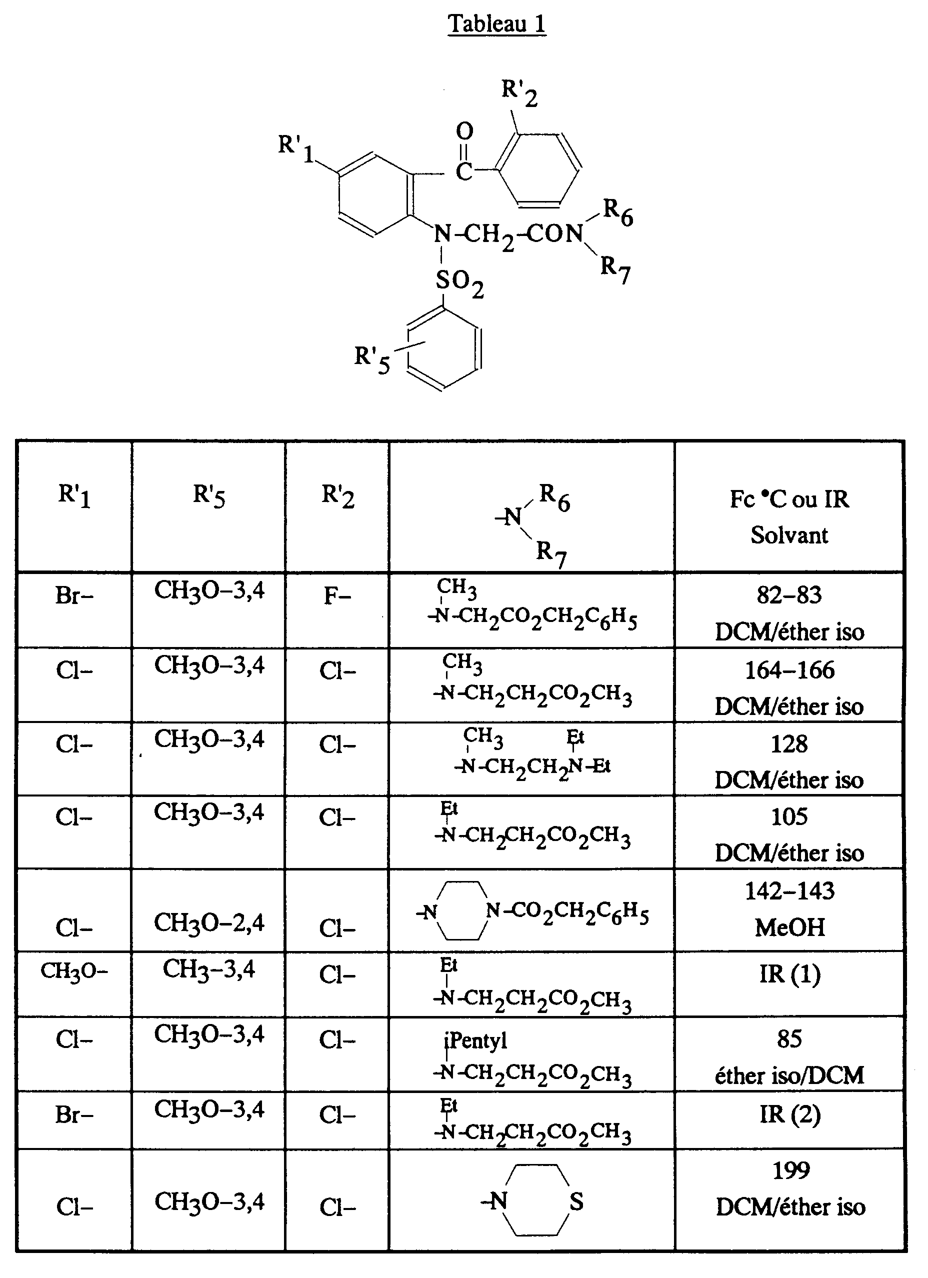

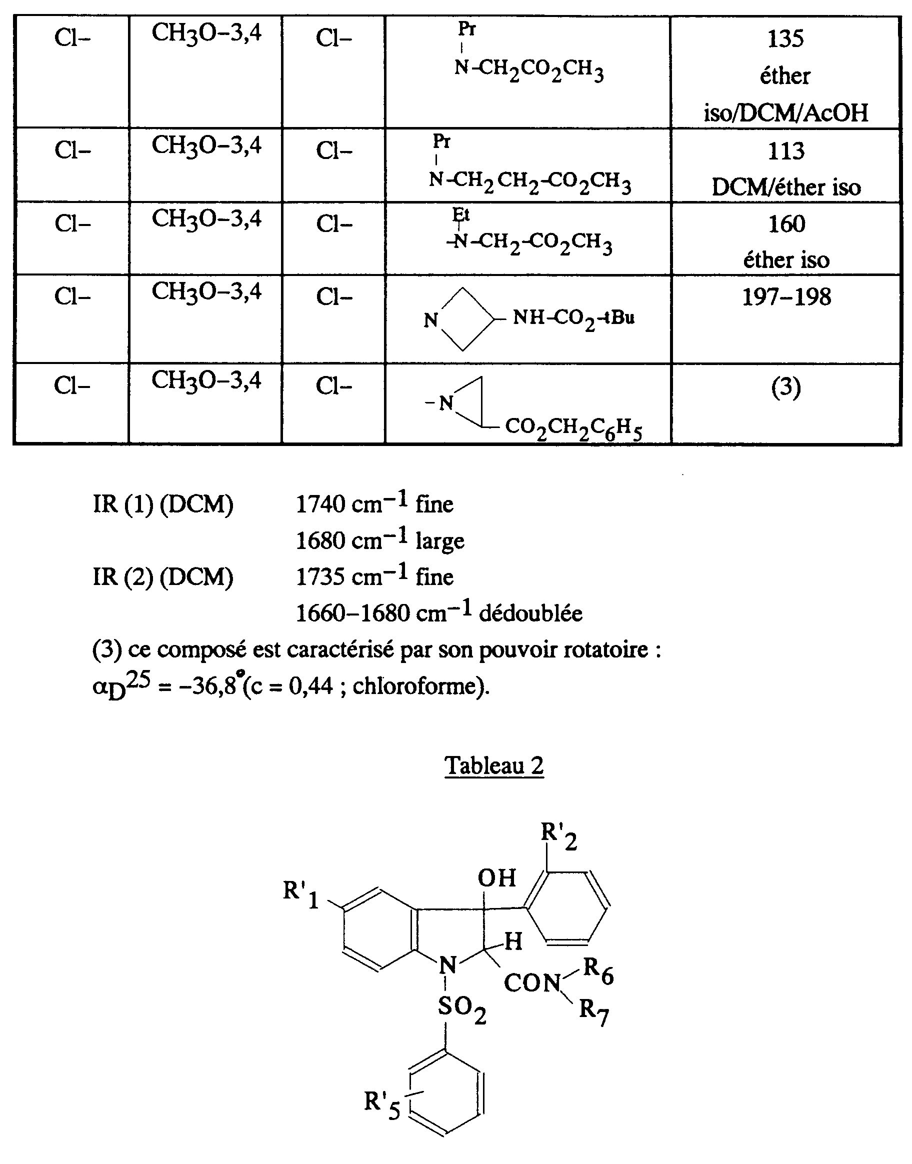

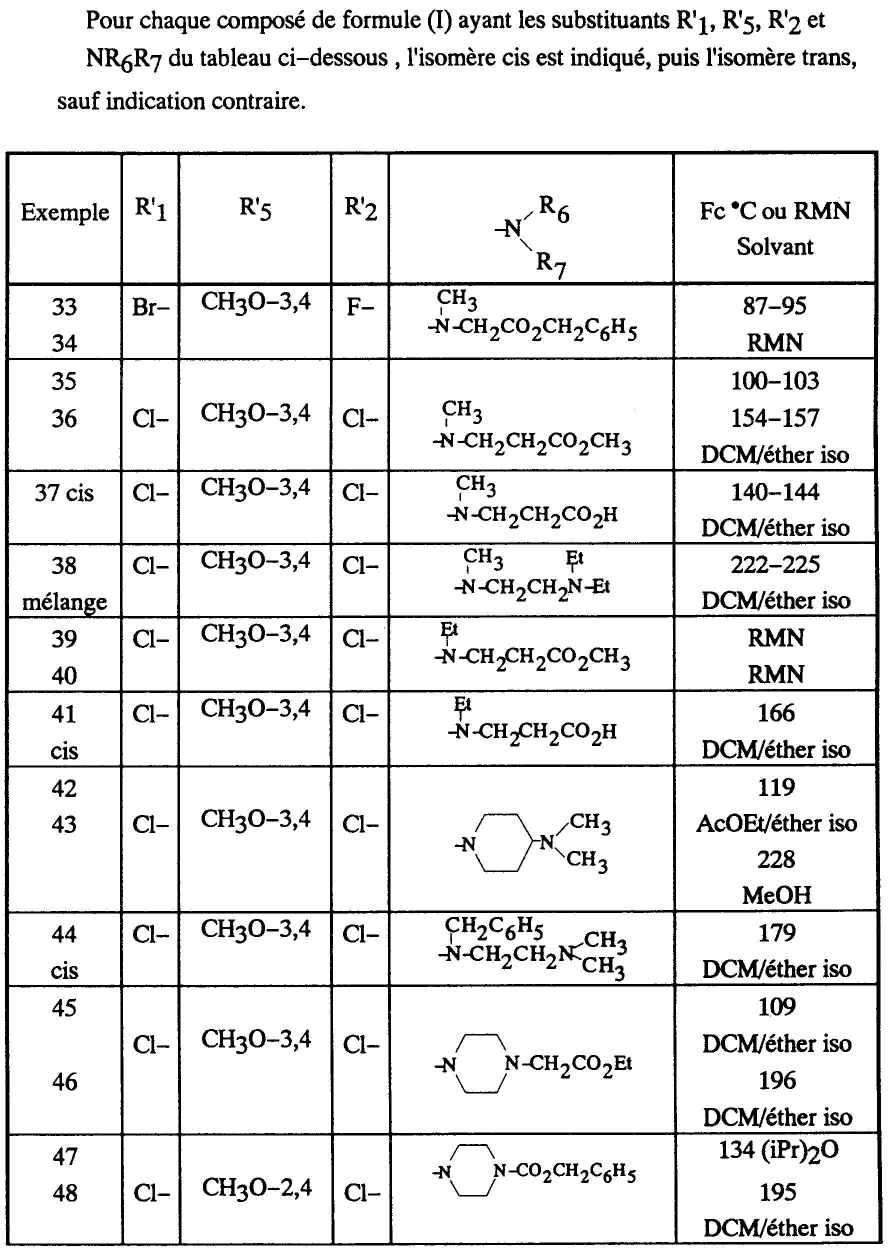

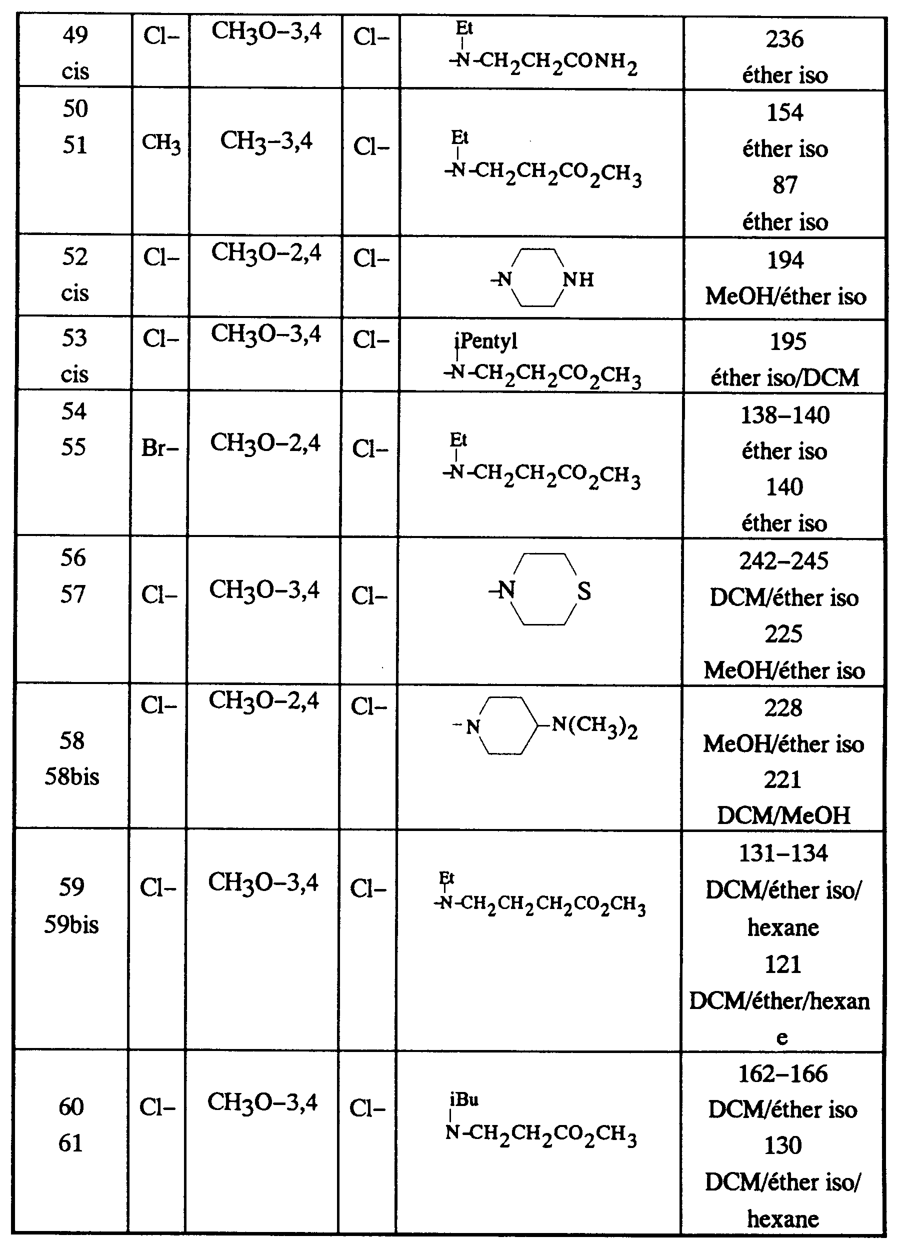

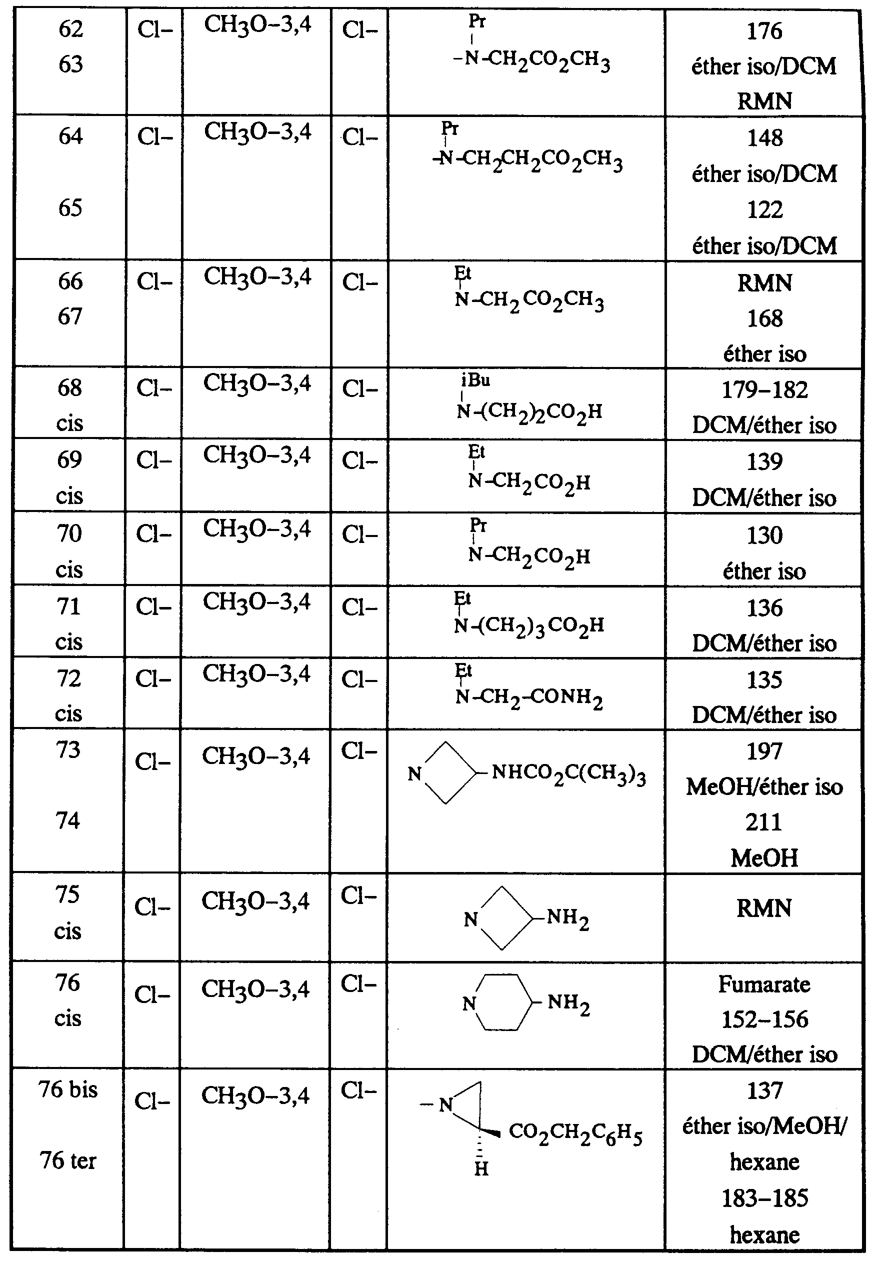

- exemple 34

| | | | | |
|---|---|---|---|---|
| Analyse : | calculé | C : 55,54 | H : 4,24 | N : 3,93 |
| | trouvé | 55,72 | 4,57 | 3,83 |

### Spectres de RMN à 200 MHz (DMSO : 2,5 ppm)

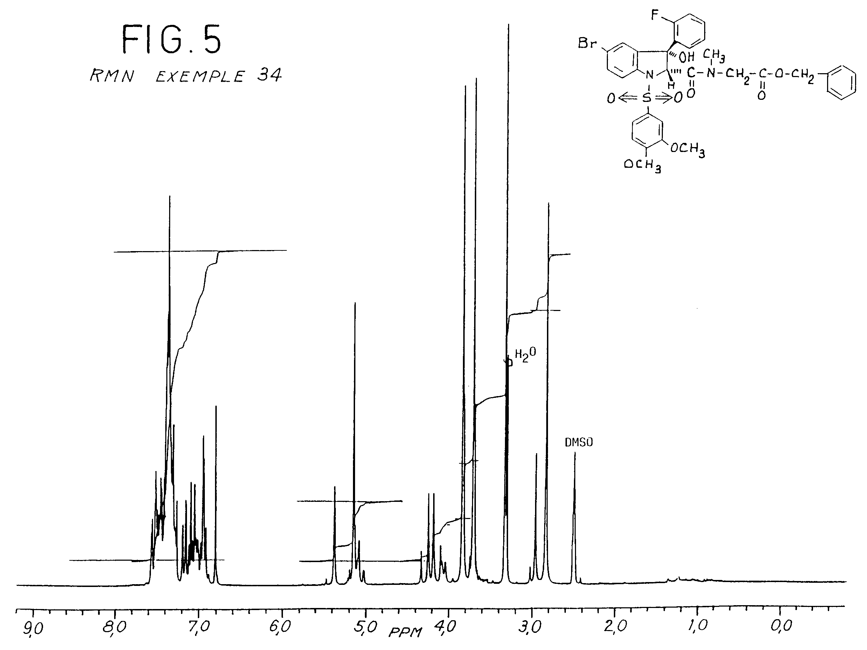

- exemple 34 : Figure 5
- exemple 38
   0,7-1,1 ppm : m : 6H : 2CH₃ (Et)
   2-4 ppm : m : 17H : 2CH₂ (Et), 2CH₂-N, N-CH₃, 2OCH₃
   5,2-5,7 ppm : 3S : 1H : H (indoline)
   6,2-8,2 ppm : m : 11H : OH + aromatiques
- exemple 39
   0,3-1,2 ppm : m : 3H : CH₃ (Et)
   1,5-4,3 ppm : m : 15 H : CH₂-CO, CH₂ (Et), CH₂-N, 2OCH₃, CO₂CH₃
   5,2-5,6 ppm :3S : 1H : H (indoline)
   6,2-8,2 ppm : m : 11H : OH + aromatiques
- exemple 40
   0,8-1,1 ppm : m : 3H : CH₃ (Et)
   2,2-3,9 ppm : m : 15H : CH₂CO, CH₂ (Et), CH₂N, CO₂CH₃, 2OCH₃
   5,3-5,7 ppm : 2S : 1H : H (indoline)
   6,6-8,2 ppm : m : 11H : OH + aromatiques
- exemple 63
   0,4-1 ppm : t dédoublé : 3 H : CH₂-CH₂-CH₃
   5 ppm : m : 2H : CH₂-CH₂-CH₃
   2,5-4,4 ppm : m : 13H : CH₂-CH₂-CH₃, NCH₂COOCH₃, 2OCH₃
   5,2-5,8 ppm : s.e. : 1H : H (indoline)
   6,5-8,3 ppm : m : 11H : OH + aromatiques
- exemple 66
   0 à 1,5 ppm : m : 3H : CH₂-CH₃
   2,3 à 5,8 ppm : m : 14H : CH₂-CH₃, NCH₂COOCH₃, 2OCH₃, H (indoline)
   6,1-8,3 ppm : m : 11H : OH + aromatiques
- exemple 75
   1,95 ppm : s.e.: 2 H:NH₂
   2,7 à 5,3 ppm : m : 12H : 2OCH₃, 2NCH₂, H (indoline), CH NH₂
   6 à 8,3 ppm : m : 11H : OH + aromatiques
- exemple 76 bis
   α_{D}²⁵ = + 102 (c = 0,35 ; chloroforme)
- exemple 76 ter
   α_{D}²⁵ = - 158°(c = 0,2 ; chloroforme).

Certains composés selon l'invention décrits dans le tableau 2 sont utiles à la préparation d'autres composés selon l'invention. Par exemple, le composé 41 a été obtenu à partir du composé 39 par traitement en milieu basique dans le méthanol MeOH/H₂O. A partir du composé 41, on a préparé le composé 49 par traitement par l'ammoniaque en présence de DIPEA et de BOP.

### EXEMPLE 77

### N-éthyl N-(amino-2 éthyl) chloro-5 (chloro-2 phényl)-3 (diméthoxy-3,4 phénylsulfonyl)-1 hydroxy-3 indoline-2 carboxamide, (isomère cis).

On dissout 500 mg du composé 49 dans 10 ml d'acétonitrile et 10 ml d'eau, on ajoute 252 mg de pyridine et 380 mg de bis (trifluoroacétoxy) iodobenzène. Après 2 heures d'agitation, on reprend par une solution d'acide chlorhydrique, extrait à l'éther, alcalinise par de la soude diluée, extrait au DCM, sèche et concentre. On obtient une huile, puis le produit attendu cristallise dans l'éther.
m = 150 mg
Fc = 164°C

### EXEMPLE 78

### N-éthyl N-[(1S)(éthoxycarbonyl-1) éthyl] chloro-5 (chloro-2 phényl)-3 (diméthoxy-3,4 phénylsulfonyl)-1 hydroxy-3 indoline-2 carboxamide, (isomère cis).

### A) Chlorure de l'acide N-[(chloro-2 phénylcarbonyl)-2 chloro-5 phényl] N-(diméthoxy-3,4 phénylsulfonyl) glycine.

On chauffe pendant 1 heure à 60°C un mélange contenant 11 g de l'acide préparé à l'exemple 10-11, étape A) et 5 g de chlorure de thionyle dans 10 ml de chloroforme. On laisse revenir à TA, concentre sous vide, reprend par DCM (2 fois). On obtient une huile jaune qui est utilisé telle quelle à l'étape suivante.
IR: 1800 cm⁻¹ (C=0)

### B) Dichloro-2′,5 N-(diméthoxy-3,4 phénylsulfonyl) N-[N′-éthyl N′-((1S)éthoxycarbonyléthyl-1) éthoxycarbamoylméthyl] amino-2 benzophénone.

La préparation de ce composé est effectuée selon J. Org. Chem., 1985, 50, 945-950.

On traite par 10 ml de TFA à 0°C, 5,15 g de (L) Boc (N-Et) AlaOEt pour enlever le groupement Boc. On concentre sous vide, reprend par 20 ml de DCM, refroidit à -78°C et ajoute 2 équivalents de TEA et le chlorure d'acide préparé à l'étape précédente, dissous dans du DCM. Après 18 heures à TA, on extrait par DCM, lave à l'eau, puis on chromatographie sur silice en éluant par un mélange DCM/AcOEt (90/10 ; v/v). Le produit attendu cristallise dans l'éther iso.
Fc = 112°C
m = 8 g

### C) N-éthyl N-[(1S)(éthoxycarbonyl-1) éthyl] chloro-5 (chloro-2 phényl)-3 (diméthoxy-3,4 phénylsulfonyl)-1 hydroxy-3 indoline-2 carboxamide, (isomère cis).

On agite à TA pendant 18 heures le composé obtenu à l'étape précédente dans 10 ml de THF et 20 ml d'éthanol, en présence de 1,46 g de DBU. On concentre sous vide, reprend le résidu par du DCM, lave à l'eau, concentre et chromatographie le produit sur alumine en éluant par AcOEt/DCM (10/90 ; v/v).
RMN
   0-0,9 ppm : d dédoublé : 3 H: CH-CH₃
   0,9-1,7 ppm: m: 6 H: 2CH₃ (éthyl)
   2,6 à 5,8 ppm: m: 12 H: 2OCH₃, NCH₂, OCH₂, NCH, COCH
   6,1 à 8,3 ppm: m: 11 H: OH -10H aromatiques

### EXEMPLES 79 et 80

### N,N-di(méthoxycarbonyl-2 éthyl) chloro-5 (chloro-2 phényl)-3 (diméthoxy-3,4 phénylsulfonyl)-1 hydroxy-3 indoline-2 carboxamide, isomère cis et isomère trans.

### A) N,N-di(méthoxycarbonyl-2 éthyl) benzylamine

Préparation selon J. Am. Chem. Soc., 1950, 72, 3298.

On refroidit dans un bain de glace, 107 g de benzylamine dans 200 ml d'éthanol et on ajoute lentement 172,2 g d'acrylate de méthyle dans 250 ml d'éthanol. Après 13 jours à TA, on évapore sous vide le solvant puis on distille une partie du résidu huileux.
Eb = 135-140°C sous 0,6 mm Hg
m = 30 g
IR : 1730 cm⁻¹

### B) N,N-(diméthoxycarbonyl-2 éthyl)amine.

27,9 g de l'amine obtenue à l'étape précédente, placés dans 500 ml de méthanol, sont mélangés avec 3 g de palladium sur charbon à 5% et traités sous pression d'hydrogène pendant 1 heure. On filtre le milieu sur Célite ®, rince par du méthanol et évapore sous vide le solvant ; l'huile résiduelle est utilisée telle quelle à l'étape suivante.

### C) (N,N-diméthoxycarbonyl-2 éthyl) bromoacétamide.

On refroidit dans un bain de glace un mélange contenant 14,3 g de l'amine préparée à l'étape précédente, 100 ml de DCM et 10,6 ml de TEA ; on ajoute goutte à goutte 15,3 g de bromure de bromoacétyle puis on laisse 48 heures sous agitation à TA. On extrait au DCM, lave à l'eau puis on effectue une chromatographie sur silice en éluant par un mélange DCM/MeOH (97/3 ; v/v). Le produit attendu est obtenu sous forme d'une huile.
m = 15,9 g
IR: 1650 cm⁻¹ et 1730 cm⁻¹

### D) Dichloro-2′,5[N-(diméthoxy-3,4 phénylsulfonyl) N-[(N′,N-di(méthoxycarbonyl-2 éthyl)) carbamoylméthyl]] amino-2 benzophénone.

14,3 g de dichloro-2′,5 (diméthoxy-3,4 phénylsulfonamido)-2 benzophénone sont placés dans 180 ml de DMF et l'on ajoute par portions 1,1 g d'hydrure de sodium. Après 1 heure d'agitation à TA, on refroidit dans un bain de glace et l'on ajoute 14,3 g du produit préparé à l'étape précédente et on laisse 72 heures sous agitation à TA. On extrait au DCM, lave à l'eau puis on chromatographie sur silice en éluant par un mélange DCM/AcOEt (93/7 ; v/v).
m = 28,4 g
Fc = 130°C

### E) N,N-di[(méthoxycarbonyl-2)éthyl] chloro-5 (chloro-2 phényl)-3 (diméthoxy-3,4 phénylsulfonyl)-1 hydroxy-3 indoline-2 carboxamide, isomère cis.

A 0°C, on mélange 12 g du composé préparé à l'étape précédente et 0,930 g de méthylate de sodium dans 150 ml de méthanol puis on laisse sous agitation une nuit à TA. Le milieu réactionnel est neutralisé par addition de KHSO₄ à5% puis on évapore le solvant sous vide. Le résidu est chromatographié sous alumine en éluant par un mélange DCM/AcOEt (8/2 ; v/v). On récupère 2,4 g du produit attendu que l'on cristallise dans le méthanol.
Fc = 175°C

### F) N,N-di[(méthoxycarbonyl-2)éthyl] chloro-5 (chloro-2 phényl)-3 (diméthoxy-3,4 phénylsulfonyl)-1 hydroxy-3 indoline-2 carboxamide isomère trans.

On poursuit la chromatographie de l'étape précédente et on élue par un mélange DCM/MeOH (9,5/0,5 ; v/v). On obtient 1,82 g d' l'isomérie trans qui cristallise dans l'éther iso.
Fc = 85°C

### EXEMPLE 81,82 et 83

### ((2R)carbamoyl-2 thiazolidinocarbonyl)-2 chloro-5 (chloro-2 phényl)-3 (diméthoxy-3,4 phénylsulfonyl)-1 hydroxy-3 indoline, (isomère cis : 2 composés et isomère trans).

### A) (L) thiazolidine carboxamide-4.

Ce composé est préparé selon J. Med. Chem., 1981, 24, 692.

### B) Dichloro-2′,5 [N-(diméthoxy-3,4 phénylsulfonyl) N-(((2R)carbamoyl-2) thiazolidinocarbonylméthyl)] amino-2 benzophénone.

Ce composé est obtenu par les méthodes habituelles à partir de l'acide préparé à l'exemple 10-11, étape A).
Fc = 125°C après cristallisation dans l'éther.

### C) ((2R)carbamoyl-2 thiazolidinocarbonyl)-2 chloro-5 (chloro-2 phényl)-3 (diméthoxy-3,4 phénylsulfonyl)-1 hydroxy-3 indoline.

4,3 g du produit obtenu à l'étape B) dans 90 ml de MeOH sont cyclisés à TA en présence de 1 g de DBU. On concentre, reprend par de l'eau et du DCM, décante, lave par KHSO₄ puis sèche et concentre. On chromatographie sur alumine en éluant par DCM/MeOH (97/3 ; v/v). On obtient le composé sous forme cis (mélange de 2 diastéréoisomères) : 1,5 g, puis sous forme trans (mélange de 2 diastéréoisomères) : m = 1 g.
a) La fraction cis est cristallisée dans MeOH/DCM pour obtenir le composé cis 1 .
   Fc = 176°C après cristallisation dans l'éther iso.
   α_{D} = + 57°(c = 0,1 chloroforme).
b) Les eaux de cristallisation du produit précédent sont chromatographiées sur silice en éluant par AcOEt/DCM (30/70 ; v/v). Le composé cis 2 obtenu est recristallisé dans l'éther.
   Fc = 205°C
   α_{D} = - 185°(c = 0,3 chloroforme).
c) La fraction trans (mélange de 2 diastéréoisomères ) est recristallisée dansl'éther iso.
   Fc = 170°C

### EXEMPLES 84, 85, 86 et 86bis

### Chloro-5 (chloro-2 phényl)-3 (diméthoxy-3,4 phénylsulfonyl)-1 hydroxy-3 (N,N-(2S)diméthylthiocarbamoyl-2 pyrrolidinocarbonyl)-2 indoline, isomérie cis (2 composés), (isomérie trans: 2 composés).

### A) (L) N,N-diméthyl (N′-Boc) prolinethioamide.

Ce composé est préparé selon J. Med. Chem., 1989, 2178.

Dans du toluène anhydre, on chauffe sous argon à 80°C pendant 4 heures, 2,36 g de N,N-diméthyl (N′-Boc) prolinamide en présence de 2,3 g de réactif de Lawesson. Après 24 heures, on évapore le solvant, ajoute de l'isopropanol. On décante le précipité formé, évapore l'isopropanol et chromatographie sur silice en éluant par hexane/AcOEt (30/70,; v/v). Le produit obtenu est recristallisé à froid dans DCM/éther iso (30/70 ; v/v).
Fc = 62°C

### B) Dichloro-2′,5 [N-(diméthoxy-3,4 phénylsulfonyl) N-((2S)(N′,N′-diméthylthiocarbamoyl-2) pyrrolidinocarbonylméthyl)] amino-2 benzophénone.

On dissout 3 g du produit préparé à l'étape précédente dans 10 ml de DCM et traite à 0°C pendant 2 heures par 10 ml de TFA. On évapore à siccité puis on ajoute à 0°C, 20 ml de DCM, 6,1 g de l'acide préparé à l'exemple 10-11, étape A) et on neutralise par 3 g de DIPEA. On dissout 5,15 g de BOP dans 30 ml de DCM et l'on ajoute cette solution à la solution précédente, à 0°C en trente minutes ; on maintient à pH neutre par addition de DIPEA et on laisse sous agitation pendant 3 heures à 0°C. Après une nuit à TA, on extrait de la manière habituelle puis on chromatographie sur silice en éluant par DCM/AcOEt (85/15 ; v/v). Le produit obtenu est recristallisé dans l'éther iso.
Fc = 182-185°C
α_{D} = -72 ° (c=0,32, chloroforme)

### C) Chloro-5 (chloro-2 phényl)-3 (diméthoxy-3,4 phénylsulfonyl)-1 hydroxy-3 (N,N-(2S)diméthylthiocarbamoyl-2 pyrrolidinocarbonyl)-2 indoline, (isomérie cis : 2 composés et isomère trans : 2 composés).

On dissout 3,8 g du composé obtenu à l'étape précédente dans 15 ml de DCM et l'on chauffe à reflux pendant 36 heures en présence de 850 mg de DBU. Les différents isomères formés sont séparés par des chromatographies successives sur silice.
a) Par DCM/AcOET (85/15 ; v/v), on élue d'abord le composé attendu sous forme d'un mélange de 2 diastéréoisomères cis. Le diastéréoisomère le moins soluble est cristallisé 2 fois du mélange DCM/éther iso-méthanol, puis on recristallise dans un minimum de DMF à 60°C, puis addition de 2 volumes d'éthanol.
   Fc = 270°C
   α_{D} = - 278 (c= 1, chloroforme)
b) Les eaux de cristallisation du mélange précédent sont reprises et le second diastéréoisomérie cis cristallise du mélange DCM/éther iso.
   Fc = 249-251°C
   α_{D} = + 42°(c=0,22, chloroforme)
c) On réunit les fractions de chromatographie éluées en dernier ainsi que les eaux mères de cristallisation des fractions a) et b) et l'on effectue une nouvelle chromatographie sur silice en éluant par hexane/AcOEt (20/80 ; v/v). On isole d'abord une fraction qui est recristallisée 3 fois du mélange DCM/éther iso et l'on élimine un insoluble sur papier entre chaque recristallisation. On obtient ainsi l'isomère trans 1.
   Fc = 191-193
   α_{D} = + 74,5°(c=0,2 ; chloroforme)
d) la seconde fraction contient l'isomère trans 2 qui est recristallisé du mélange DCM/éther iso et cristallise avec 1/3 mole d'éther iso.
   Fc = 170°C
   α_{D} = - 266°(c=0, 14, chloroforme)

### EXEMPLES 87, 88, 89 -

### ((2S)Carbamoyl-2 pyrrolidinocarbonyl)-2 chloro-5 cyclohexyl-3 (diméthoxy-3,4 phénylsulfonyl)-1 hydroxy-3 indoline, (isomères cis : 2 composés, isomére trans).

### A) A) Chloro-5 [N-(diméthoxy-3,4 phénylsulfonyl) amino]-2 cyclohexylphénone.

On laisse 24 heures sous agitation à TA une solution de 35,6 g d'amino-2 chloro-5 cyclohexylphénone et 39,5 g de chlorure de diméthoxy-3,4 phénylsulphonyle dans 340 ml de pyridine. On évapore le solvant sous vide puis on lave à l'eau et par une solution acide (HCl 0,5 N). Le produit attendu cristallise dans l'éthanol.
Fc = 135°C
m = 56,1 g

### B) [(N-benzyloxycarbonylméthyl N-diméthoxy-3,4 phénylsulfonyl) amino]-2 chloro-5 cyclohexylphénone.

On ajoute par portions 3,2 g d'hydrure de sodium à 52,6 g du composé préparé à l'étape précédente dans 520 ml de DMF et on laisse 1 heure à TA sous agitation. Après refroidissement au bain de glace, on ajoute goutte à goutte 21 ml de bromure de benzyloxycarbonylméthyle et on laisse 24 heures sous agitation à TA.

On évapore sous vide le solvant et reprend par de l'eau. On extrait au DCM et lave à l'eau ; le produit obtenu est utilisé tel quel à l'étape suivante.

### C) N-(chloro-4 cyclohexylcarbonyl-2) phényl, N-(diméthoxy-3,4 phénylsulfonyl)glycine.

On place sous hydrogène (1 atmosphère) le composé obtenu à l'étape précédente avec 3,9 g de Palladium sur charbon à 5% dans 700 ml d'acide acétique. A la fin de la réaction on filtre le Palladium sur Célite ® et rince avec de l'acide acétique chaud ; on évapore le solvant sous vide et reprend par de l'eau. On extrait au DCM, lave à l'eau puis par une solution concentrée de NaHCO₃. Le résidu obtenu est chromatographié sur silice en éluant par un mélange DCM/MeOH (97/3 ; v/v). Le produit attendu cristallise dans l'éthanol.
Fc = 160°C
m = 22,4 g

### D) Chloro-5 [N-(diméthoxy-3,4 phénylsulfonyl) N-((2S)carbamoyl-2 pyrrolidinocarbonylméthyl)] amino-2 cyclohexylphénone.

On refroidit à 0°C un mélange contenant 9,92 g de l'acide préparé à l'étape précédente, 3 g de chlorhydrate de (L) prolinamide et 3,5 ml de DIPEA dans 75 ml de DCM. On ajoute 8,84 g de BOP en solution dans du DCM et on maintient à pH7 par addition de DIPEA. On laisse 24 heures sous agitation à TA. On extrait le milieu au DCM, lave par une solution saturée de NaHCO₃, une solution saline, une solution KHSO₄ à 5%, à nouveau une solution saline. On chromatographie sur silice en éluant par un mélange DCM/MeOH (96/4 ; v/v). Le produit attendu
concrétise dans l'éther iso. Fc = 110°C
m = 7,3 g
α_{D} = -53,9°(c = 1 ; chloroforme)

### E) (2S)(Carbamoyl-2 pyrrolidinocarbonyl)-2 chloro-5 cyclohéxyl-3 (diméthoxy-3,4 phénylsulfonyl)-1 hydroxy-3 indoline, isomère cis (2 composés), isomère trans.

On place sous agitation à 0°C pendant 48 heures, 5,9 g du composé préparé à l'étape précédente et 1,67 g de DBU dans 60 ml de méthanol. On évapore le solvant sous vide, on ajoute de l'eau, extrait au DCM puis on lave par une solution de KHSO₄ à 5%. On chromatographie sur alumine en éluant par DCM/MeOH (98/2 ; v/v).
a) La fraction la moins polaire contient les 2 isomères cis. Cette fraction est recristallisée dans le méthanol. Le premier composé ainsi obtenu (cis 1) est pur en CLHP.
   Fc = 185°C

   Par recristallisation des eaux-mères dans le MeOH, on obtient un second composé (cis 2). Pureté CLHP: 75% (il contient 25% de cis 1).
   Fc = 132°C
b) La fraction la plus polaire contient l'isomère trans sous forme d'un composé apparemment unique obtenu par recristallisation dans le méthanol.
   Fc = 240°C
   α_{D} = -55, 1°(c = 1 ; chloroforme)

### EXEMPLE 89 bis

### ((2S)Carbamoyl-2 pyrrolidinocarbonyl)-2 chloro-5 cyclohéxyl-3 (diméthoxy-3,4 phénylsulfonyl)-1 hydroxy-3 indoline, isomère cis (2 composés), isomère trans.

En utilisant un mode opératoire semblable à celui décrit pour les exemples 87, 88, 89, on prépare un composé analogue en série (D) proline.

Le composé obtenu après cristallisation dans un mélange DCM/MeOH a la configuration trans.
Fc = 238°C
α_{D} = + 164°(c = 0,245 ; chloroforme/méthanol, 8/2, v/v).

Le spectre RMN de ce composé et de celui décrit à l'étape E b) de l'exemple précédent sont identiques.

### EXEMPLE 90

### Chloro-5 (chloro-2 phényl)-3 (diméthoxy-3,4 phénylsulphonyl)-1 [(2S)(N-méthylaminocarbonyl)-2 pyrrolidinocarbonyl]-2 hydroxy-3 indoline, isomère cis.

920 mg du composé préparé à l'exemple 28 sont placés sous agitation dans 20 ml de DCM contenant 371 mg de BOP, pendant 15 minutes puis on fait barbotter un courant de monométhylamine pendant 10 minutes et on maintient l'agitation pendant 30 minutes supplémentaires. On reprend par de l'eau, décante, lave au sulfate acide de potassium, au carbonate de sodium, sèche et concentre. Le résidu est chromatographié sur silice en éluant par DCM/méthanol (97,5/2,5 ; v/v). On recueille le produit attendu qui cristallise du mélange éther iso/DCM.
m = 750 mg
Fc = 158°C
α_{D} = -216°(c = 0,3 ; chloroforme)

En procédant comme dans les exemples précédemment décrits (exemples 27 à 31 et 90) et en utilisant des dérivés de la (L) proline (sauf indication contraire) on a préparé d'autres composés (VI) intermédiaires pour la synthèse des composés (I) selon l'invention.

Les composés (VI) préparés sont décrits dans le tableau 3 ci-après.

Les composés (I) préparés sont décrits dans le tableau 4 ci-après.

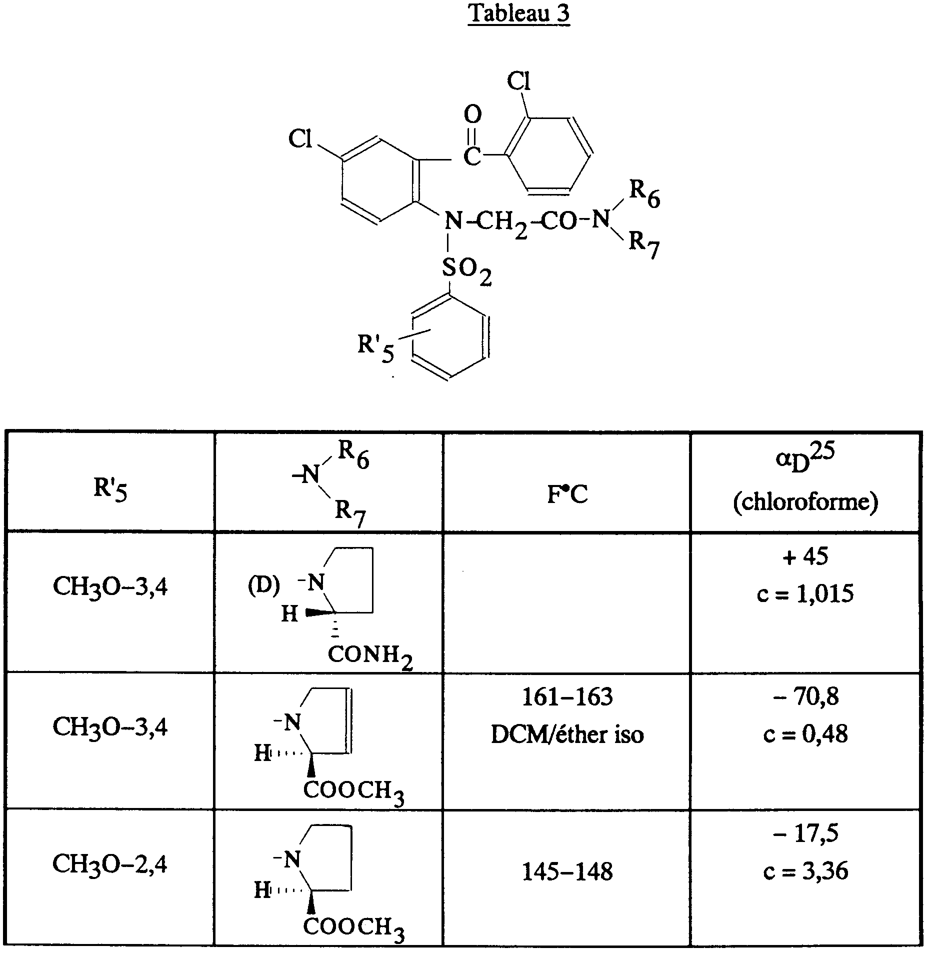

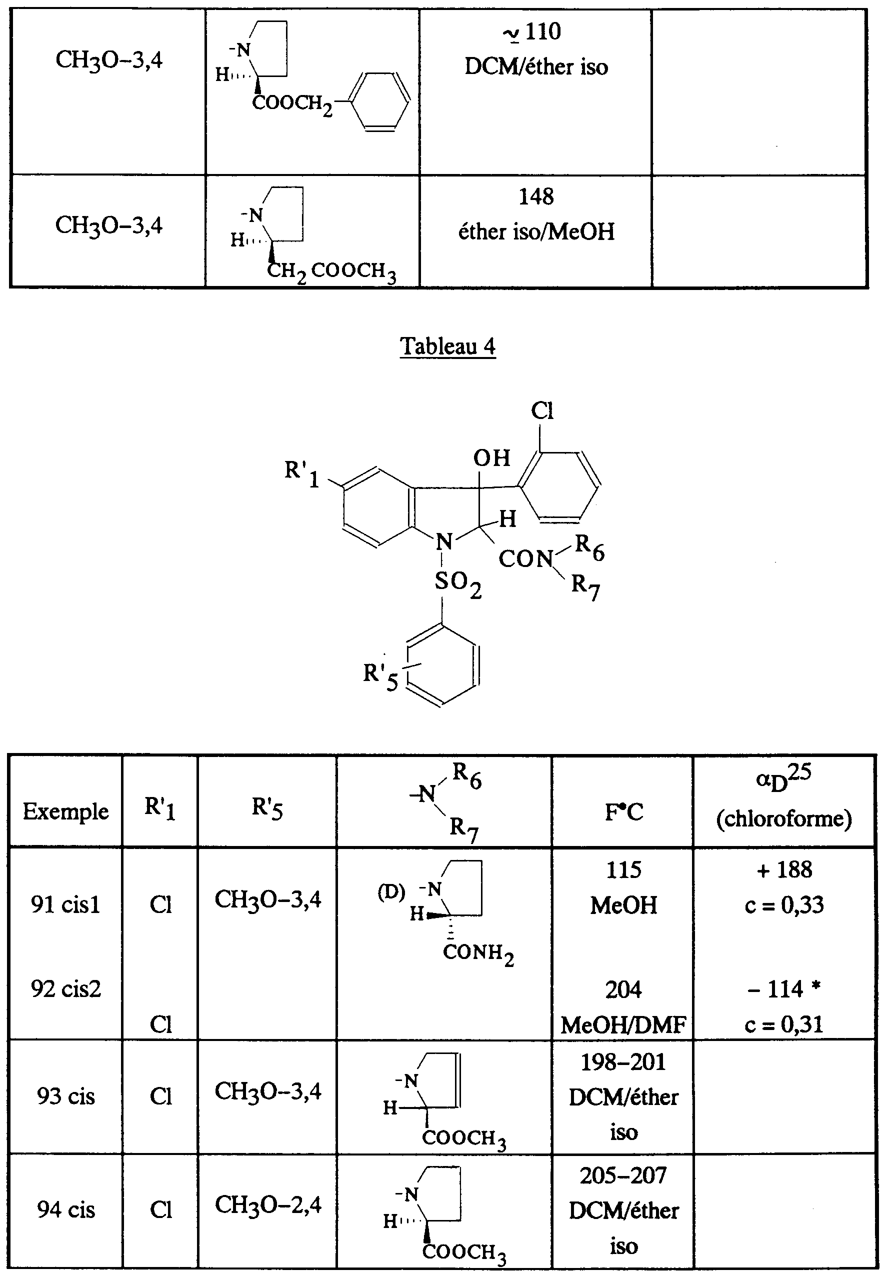

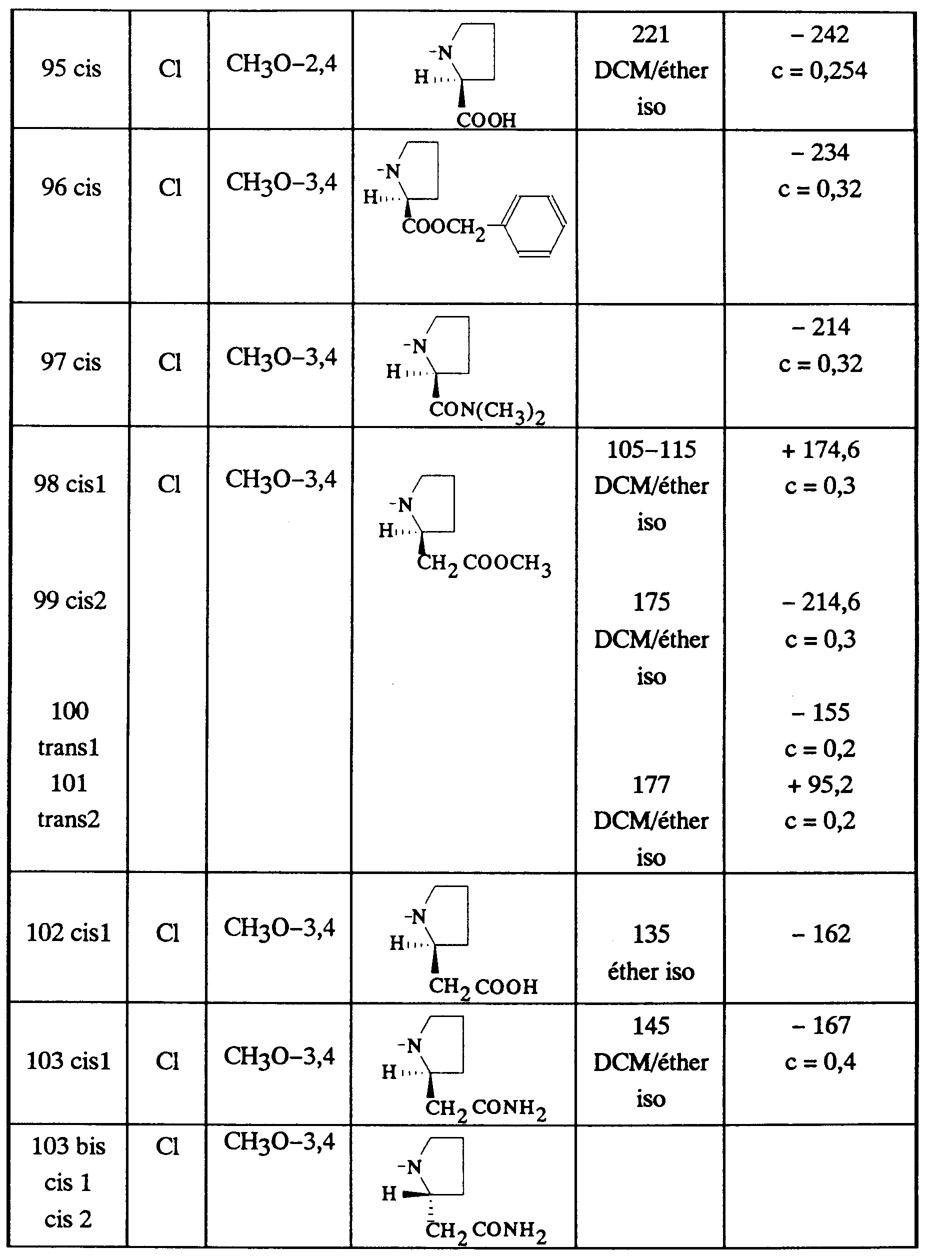

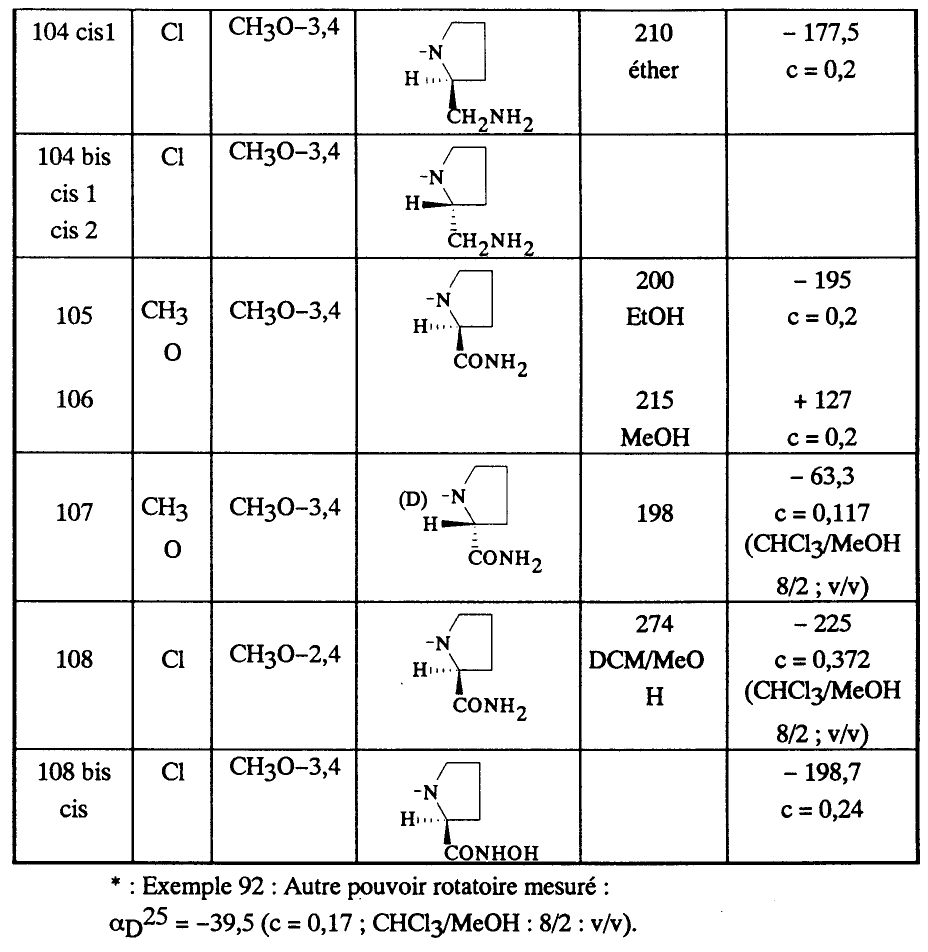

Le composé de l'exemple 107 est l'énantiomère de celui de l'exemple 106.

Le composé de l'exemple 108 bis a été préparé à partir du composé de l'exemple 28 par action du chlorhydrate d'hydroxylamine dans le DMF et activation par le réactif BOP en présence de DIPEA.

Certains composés selon l'invention, décrits dans le tableau 4 ci-dessus, sont utiles pour la préparation d'autres composés. Ainsi, le composé de l'exemple 99 permet d'obtenir le composé de l'exemple 101, puis celui de l'exemple 103 et enfin celui de l'exemple 104.

### EXEMPLE 109

### ((2S,4S)azido-4 méthoxycarbonyl-2 pyrrolidinocarbonyl)-2 chloro-5 (chloro-2 phényl)-3 (diméthoxy-3,4 phénylsulfonyl)-1 hydroxy-3 indoline, isomère cis.

### A) Dichloro-2′,5 [N-(diméthoxy-3,4 phénylsulfonyl)-N-((2S,4R),hydroxy-4 méthoxycarbonyl-2 pyrrolidinocarbonylméthyl)] amino-2 benzophénone.

On chauffe à 0°C dans 150 ml de DCM, 15 g de l'acide préparé à l'exemple 10-11, étape A) et 6,25 g de chlorhydrate de (2S,4R) hydroxy-4 prolinate de méthyle, en présence de 7,4 g de DIPEA. On ajoute goutte à goutte en 30 minutes une solution de 12,7 g de BOP dans 30 ml de DCM et on ajoute la quantité de DIPEA nécessaire pour neutraliser la solution. Après une nuit à TA, on extrait de la manière habituelle et chromatographie sur silice en éluant par un mélange DCM/AcOEt (60/40 ; v/v). Le produit attendu cristallise du mélange DCM/éther/éther iso.
Fc = 128-131°C
α_{D} = + 8,5° (c = 0,38 ; chloroforme)

### B) Dichloro-2′,5 [N-(diméthoxy-3,4 phénylsulfonyl)-N-((2S,4R)mesyloxy-4 méthoxycarbonyl-2 pyrrolidinocarbonylméthyl)] amino-2 benzophénone.

2 g du composé obtenu à l'étape précédente sont dissous à 0°C dans 10 ml de DCM. On ajoute 550 mg de triéthylamine puis 550 mg de chlorure de méthanesulfonyle et on laisse à 0°C pendant 20 heures. On ajoute de l'eau, lave par de l'eau chlorhydrique 0,5 N, par de l'eau, puis par une solution de bicarbonate de sodium, sèche sur sulfate de magnésium et évapore. L'huile obtenue est utilisée telle quelle à l'étape suivante.

### C) [N-((2S,4S)azido-4 méthoxycarbonyl-2 pyrrolidinocarbonylméthyl)-N-(diméthoxy-3,4 phénylsulfonyl)] amino-2 dichloro-2′,5 benzophénone.

On chauffe 11 g du produit préparé à l'étape précédente dans 60 ml de DMSO, à 80-90°C, en présence de 2,7 g d'azidure de sodium pendant 18 heures. On verse dans de l'eau, extrait à l'acétate d'éthyle, lave à l'eau, sèche et chromatographie sur silice en éluant par un mélange pentane/AcOEt (50/50 ; v/v. On obtient une huile (10 g).
α_{D} = -25,5 (c = 0,39 ; chloroforme, T = 26°C)

### D) ((2S,4S)azido-4 méthoxycarbonyl-2 pyrrolidinocarbonyl)-2 chloro-5 (chloro-2 phényl)-3 (diméthoxy-3,4 phénylsulfonyl)-1 hydroxy-3 indoline, isomère cis.

3,38 g du produit obtenu à l'étape précédente sont cyclisés dans les conditions habituelles en présence de DBU. On obtient le produit attendu qui est recristallisé dans DCM/éther iso.
m = 755 mg
Fc = 200-202°C
α_{D} = - 176° (c = 0,21 ; chloroforme, T = 26°C)

### EXEMPLE 110

### [(2S,4S)(N-benzyloxycarbonyl-N-méthyl) amino-4 méthoxycarbonyl-2 pyrrolidinocarbonyl]-2 chloro-5 (chloro-2 phényl)-3 (diméthoxy-3,4 phénylsulphonyl)-1 hydroxy-3 indoline, isomère cis.

### A) ester méthylique de N-Boc hydroxy-4 proline.

On part du chlorhydrate de l'ester méthylique de (2S, 4R) hydroxy-4 proline.

19 g de ce composé sont mis en suspension dans 100 ml de THF et l'on ajoute 22,9 g de (Boc)₂O puis on refroidit à 0°C. On ajoute goutte à goutte 21,2 g de triéthylamine dans 25 ml de THF puis on agite 12 heures à 0°C et 4 heures à 60°C. On ajoute de l'eau, extrait par de l'acétate d'éthyle, lave à l'eau, par une solution de sulfate acide de potassium (4 fois), par de l'eau, puis de l'eau saline. On évapore et isole une huile (21,6 g) contenant un peu de (Boc)₂O.

### B) Ester méthylique de (2S,4R)N-Boc mésyloxy-4 proline.

On refroidit à 0°C une solution de 22,9 g du produit préparé à l'étape précédente dans 250 ml de DCM. On ajoute goutte à goutte 22,9 g de chlorure de mésyle dans 10 ml de DCM, puis goutte à goutte 9,4 g de triéthylamine dans 100 ml de DCM et on laisse revenir à TA pendant une nuit. On évapore à siccité, ajoute de l'eau, extrait par AcOEt, lave par de l'eau, de l'eau saline et sèche sur sulfate de magnésium. Après une nouvelle évaporation, on obtient une huile utilisée telle quelle à l'étape suivante.

### C) Ester méthylique de (2S,4S)N-Boc azido-4 proline.

Ce composé est préparé à partir de celui obtenu à l'étape B. On dissout dans 70 ml de DMSO 15,2 g d'ester méthylique de N-Boc mésyloxy-4 proline et on chauffe à 90°C pendant 5 heures en présence de 3,05 g d'azidure de sodium. On refroidit, ajoute de l'eau, extrait par AcOEt, lave par de l'eau, de l'eau saline, sèche sur MgSO₄. L'huile obtenue est purifiée par chromatographie sur silice en éluant par le mélange AcOEt/hexane (40/60 ; v/v).
α_{D} = -37,8 (c = 3 ; chloroforme)
litt. α_{D} = - 36,6° (c= 2,8 ; chloroforme) D.J. Abraham et al., J. Med. Chem., 1983, 549, 26.

### D) Ester méthylique de (2S,4S)N-Boc amino-4 proline.

On dissout dans 100 ml de méthanol 8,45 g du composé obtenu à l'étape C, ajoute 500 mg de Pd/C à 10% et hydrogène à 40°C pendant 18 heures. On filtre le catalyseur, évapore la moitié du méthanol, ajoute 100 ml d'HCl 0,5 N puis évapore le reste du méthanol et extrait par AcOEt le produit de départ qui n'a pas réagi. La phase aqueuse est traitée par du carbonate de sodium et on extrait par AcOEt la fraction contenant le produit attendu (m = 4,35g).

### E) Ester méthylique de (2S,4S)N-Boc (N′-benzyloxycarbonylamino)-4 proline.

Le produit brut obtenu à l'étape précédente est dissous dans 15 ml d'éther et 15 ml de DCM à 0°C. On ajoute 2,3 g de DIPEA puis 3,03 g de chloroformiate de benzyle dans 5 ml de DCM, en70 minutes à 0°C. Après 3 heures, on évapore les solvants à TA sous vide ; on ajoute de l'eau et de l'acétate d'éthyle, lave la phase organique successivement par une solution de sulfate acide de potassium (3 fois), par de l'eau (3 fois), par une solution de carbonate de sodium (3 fois), par de l'eau (3 fois), de l'eau saline. On chromatographie sur silice en éluant par le mélange hexane/AcOEt (40/60 ; v/v) pour obtenir le produit attendu.
α_{D} = -16,4 (c = 0,3 ; chloroforme)

### F) Ester méthylique de (2S,4S)N-Boc (N′-benzyloxycarbonyl-N′-méthyl) amino-4 proline.

2 g du composé obtenu à l'étape précédente sont dissous dans 20 ml de DMF à 0°C, sous argon, en présence de 2,25 g d'iodure de méthyle. On ajoute par fractions 170 mg d'hydrure de sodium à 80% puis on agite à 0°C pendant 90 minutes. On extrait à l'eau et à l'acétate d'éthyle ; on lave la phase organique par de l'eau puis de l'eau saline. On chromatographie sur silice en éluant par un mélange hexane/AcOEt (50,50 ; v/v). On obtient 1,55 g du produit attendu.
α_{D} = -38,8 (c = 0,38 ; chloroforme)

### G) Dichloro-2′5 [(2S,4S)N-(diméthoxy-3,4 phénylsulfonyl) N-((N′-benzyloxycarbonyl-N′-méthyl)amino-4 méthoxycarbonyl-2) pyrrolidinocarbonylméthyl] amino-2 benzophénone.

Ce produit est obtenu par les méthodes habituelles.
α_{D} = -22,4 (c = 0,37 ; chloroforme)

### H) [((2S,4S)N-benzyloxycarbonyl-N-méthyl) amino-4 méthoxycarbonyl-2 pyrrolidinocarbonyl]-2 chloro-5 (chloro-2 phényl)-3 (diméthoxy-3,4 phénylsulfonyl)-1 hydroxy-3 indoline, isomère cis.

Ce produit est obtenu par cyclisation en présence de DBU selon les méthodes habituelles. Les cristaux formés sont cristallisés dans DCM/éther iso.
Fc = 129°C
α_{D} = - 129° (c = 0,321 ; chloroforme)

La pureté isomérique en CLHP est 99%

Les composés préparés aux exemples 109 et 110 sont utilisés pour préparer les composés selon l'invention décrits dans le tableau 5 ci-après :

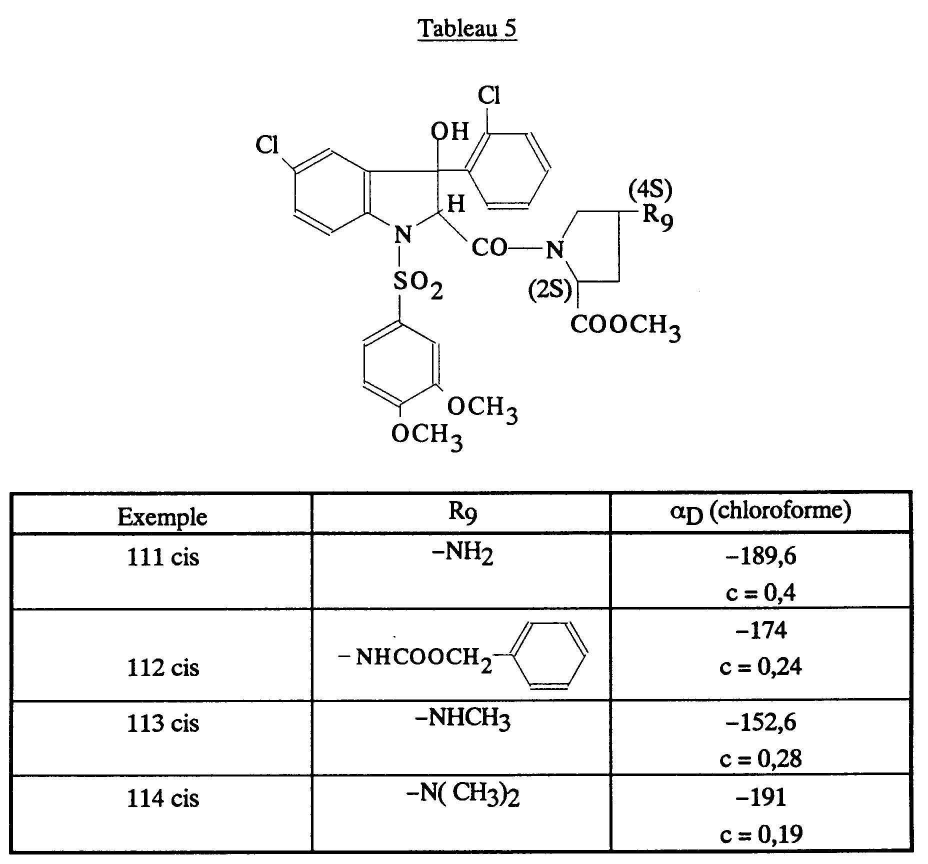

Le composé de l'exemple 112 permet de préparer successivement les composés des exemples 115 et 116 décrits dans le tableau 6 ci-après et le composé de l'exemple 114 permet de préparer le composé de l'exemple 116 bis.

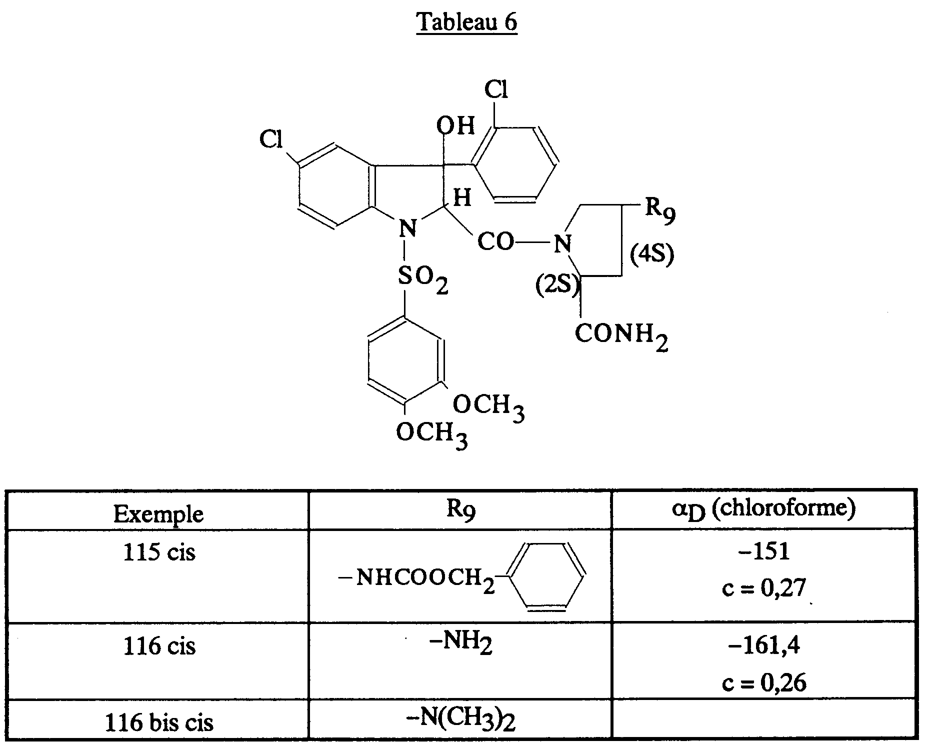

Le composé de l'exemple 116 bis peut être préparé soit par transformation du composé de l'exemple 114, soit à partir de la (2S, 4S) N-Boc diméthylamino-4 prolinamide dont la préparation est effectuée comme suit :
1) on prépare le (2S, 4S) N-Boc amino-4 prolinate de méthyle à partir du (2S, 4S) azido-4 prolinate de méthyle selon T.R. Webl dans J. Org. Chem., 1991, 56, 3009.
2) (2S, 4S) N-Boc diméthylamino-4 prolinate de méthyle.
   On dissout 4 g du composé préparé en 1) dans 50 ml d'acétonitrile, on ajoute 12,8 ml de formol à 30 % puis en 5 minutes 3 g de cyanoborohydrure de sodium. Après 2 heures de contact, on ajoute de l'acide acétique pour amener la solution vers pH 6. Après 3 heures, on évapore l'acétonitrile puis on ajoute de l'eau, du carbonate de potassium et du chlorure de sodium solide et on extrait par 4 volumes d'acétate d'éthyle. On évapore la phase organique, on dissout le résidu par de l'acide chlorhydrique 1 N et on extrait par AcOEt. On ajoute du carbonate de sodium solide puis du chlorure de sodium solide dans la phase aqueuse et on extrait par AcOEt. Après évaporation, on chromatographie le résidu sur gel de silice en éluant par le mélange DCM/MeOH (95/5, v/v), on isole une huile qui solidifie.
   m = 2,1 g
   IR (DCM) : 1755 cm⁻¹, 1695 cm⁻¹.
3) 534 mg de l'ester préparé en 2) sont dissous dans 4 ml de MeOH et traités par de la soude (116 mg) dans 1 ml d'eau pendant 48 heures à TA. On acidifie par de l'acide chlorhydrique 0,5 N à pH 3,5 et on évapore à siccité. On effectue un séchage azétropique du résidu en présence de benzène (5 fois) puis on sèche sous vide pendant 8 heures. On ajoute alors 2 ml de DMF et 3 ml de DCM et on refroidit à 0°C. On additionne 865 mg de BOP et de la DIPEA pour amener le milieu réactionnel à neutralité. Après 15 minutes, on fait barbotter 2 fois pendant 30 minutes un courant d'ammoniac gazeux. Après 2 heures à TA, on évapore le DCM, ajoute de l'eau carbonatée, du chlorure de sodium, puis on extrait par 4 volumes d'AcOEt. Après évaporation, le résidu est chromatographié sur silice. On élue par le mélange (DCM/MeOH/NH₄OH ; 84,5/15/0,5 ; v/v/v) un solide (m = 185 mg) que l'on recristallise dans le mélange DCM/éther iso.
   Fc = 183-186°C.
   α_{D}²⁵ = - 63,1° (c = 0,24 ; chloroforme).

### EXEMPLE 117

### Décarboxylation du N-(carboxy-2 éthyl)-N-éthyl (chloro-2 phényl)-3 chloro-5 (diméthoxy-3,4 phénylsulphonyl)-1 hydroxy-3 indoline-2 carboxamide, isomère cis.

On place en solution dans 20 ml deTHF, 630 mg du composé préparé à l'exemple 41 sous atmosphère d'argon puis on ajoute 101 mg de N-méthylmorpholine à -15°C et 118 mg de chloroformiate d'isobutyle. Après 5 minutes d'agitation, on ajoute 127 mg de N-hydroxypyridine-2 thione, 101 mg de TFA et l'on maintient à -15°C sous agitation pendant 15 minutes puis on ajoute 900 mg de *tert*-butylmercaptan et laisse revenir à TA. On irradie alors le milieu réactionnel pendant 1 heure 30 avec une lampe à filament de Tungstène (150 watts). On concentre le milieu, reprend par de l'eau, extrait au DCM, sèche et concentre. Le résidu est chromatographié sur silice en éluant par DCM/AcOEt (95/5 ; v/v). On obtient le produit attendu.
m = 300 mg
Fc = 215°C

Ce composé est semblable à celui de l'exemple 125, décrit dans la demande de brevet européen EP 469984. Il a la configuration cis autour de la liaison 2,3 de l'indoline comme le produit de départ.

### EXEMPLE 118

### Décarboxylation du ((2R)carboxyméthyl-2 pyrrolidinocarbonyl)-2 chloro-5 (chloro-2 phényl)-3 (diméthoxy-3,4 phénylsulfonyl)-1 hydroxy-3 indoline, isomère cis.

On procède comme dans l'exemple précédent à partir du composé décrit à l'exemple 102.

Le produit obtenu est recristallisé du mélange DCM/éther iso.
Fc = 215-220°C
α_{D} = -214,5° (c = 0,2 ; chloroforme)
Ce composé est le ((2S)méthyl-2 pyrrolidinocarbonyl)-2 chloro-5 (chloro-2 phényl)-3 (diméthoxy-3,4 phénylsulfonyl)-1 hydroxy-3 indoline, isomère cis.

### EXEMPLE 119

### Décarboxylation du (carboxy-2 pyrrolidinocarbonyl)-2 chloro-5 (chloro-2 phényl)-3 (diméthoxy-3,4 phénylsulfonyl)-1 hydroxy-3 indoline, isomère cis.

On procède comme à l'exemple précédent en utilisant comme produit de départ le composé préparé à l'exemple 28. Le produit obtenu est recristallisé du mélange éther iso/DCM.
Fc = 263°C
α_{D} = -2O1,5° (c = 0,2 ; chloroforme)

Ce composé est le pyrrolidinocarbonyl-2 chloro-5 (chloro-2 phényl)-3 (diméthoxy-3,4 phénylsulfonyl)-1 hydroxy-3 indoline, isomère cis.

## Revendications (Revendications pour l'(les) Etat(s) contractant(s) suivant(s): AT, BE, CH, DE, DK, FR, GB, IE, IT, LI, LU, NL, PT, SE)

1. Un composé de formule : 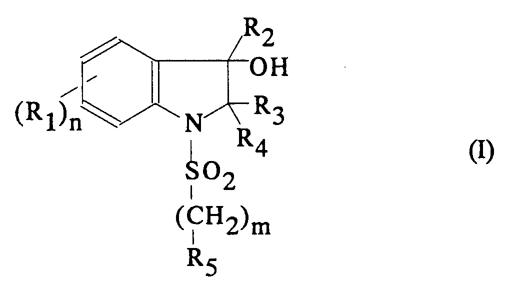 dans laquelle
- R₁ représente un atome d'halogène, un alkyle en C₁-C₄, un hydroxyle, un alcoxy en C₁-C₄, un groupe benzyloxy, un groupe cyano, un groupe trifluorométhyle, un groupe nitro ou un groupe amino ;
- R₂ représente un alkyle en C₁-C₆, un cycloalkyle en C₃-C₇, un cycloalcène en C₅-C₇, un phényle non substitué ou substitué une ou plusieurs fois par un alkyle en C₁-C₄, un alcoxy en C₁-C₄, un halogène, un groupe trifluorométhyle, un groupe amino, ou R₂ représente un nitrophényle non substitué ou substitué une fois par un groupe trifluorométhyle ou une ou plusieurs fois par un alkyle en C₁-C₄ ou un halogène ;
- R₃ représente un atome d'hydrogène ;
- R₄ représente un groupe carbamoyle de formule CONR₆R₇ ;
- R₅ représente un alkyle en C₁-C₄ ; un naphtyl-1 ; un naphtyl-2 ; un diméthylamino-5 naphtyl-1 ; un phényle non substitué ou substitué par un ou plusieurs substituants choisis parmi un halogène, un alkyle en C₁-C₄, un groupe trifluorométhyle, un groupe amino libre ou substitué par un ou 2 alkyles en C₁-C₄, un hydroxyle, un alcoxy en C₁-C₄, un alcénoxy en C₂-C₄, un alkylthio en C₁-C₄, un groupe trifluorométhoxy, un groupe benzyloxy, un groupe cyano, un groupe carboxy, un groupe alcoxycarbonyle en C₁-C₄, un groupe carbamoyle libre ou substitué par un ou deux alkyles en C₁-C₄ ou un groupe alkylamido en C₁-C₄, ou R₅ représente un nitrophényle non substitué ou substitué une fois par un groupe trifluorométhyle ou un alcénoxy en C₂-C₄ ou une ou plusieurs fois par un halogène, un alkyle en C₁-C₄, un alcoxy en C₁-C₄, un alkylthio en C₁-C₄, un groupe trifluorométhoxy ou un groupe benzyloxy ;
- R₆ représente un alkyle en C₁-C₆, ou R₆ est semblable à R₇ ;
- R₇ représente un groupe pipéridin-4-yl ; un groupe azétidin-3-yl, lesdits groupes étant substitués ou non sur l'azote par un alkyle en C₁-C₄, par un benzyloxycarbonyle ou par un alcoxycarbonyle en C₁-C₄ ; un groupe (CH₂)ᵣ lui-même substitué par un groupe pyridyl -2, -3 ou -4, par un groupe hydroxyle, par un groupe amino libre ou substitué par un ou deux alkyles en C₁-C₄, un groupe carboxyle, un groupe alcoxycarbonyle en C₁-C₄, un groupe benzyloxycarbonyle, un groupe carbamoyle libre ou substitué par un ou 2 alkyles en C₁-C₄ ;
- ou R₆ et R₇ ensemble constituent avec l'atome d'azote auquel ils sont liés un hétérocycle choisi parmi :
. la morpholine,
. la thiomorpholine,
. la thiazolidine ou la 2,2-diméthylthiazolidine non substituées ou substituées par R₈,
. la pipérazine non substituée ou substituée en 4 par un groupe R''₈,
. un cycle insaturé monoazoté à 5 chaînons substitué par R₈ ou un cycle saturé monoazoté à 3, 4, 5, 6 ou 7 chaînons substitué par R₈ et R₉ ;
- R₈ représente R′₈ ou un groupe (CH₂₎ᵣ lui-même substitué par un hydroxyle ou par un amino libre ou substitué par un ou deux alkyles en C₁-C₄ ;
- R′₈ représente un groupe (CH₂)_{q} lui-même substitué par un groupe carboxyle, un groupe alcoxycarbonyle en C₁-C₄, un groupe benzyloxycarbonyle, un groupe carbamoyle libre ou substitué par un hydroxyle ou par un ou 2 alkyles en C₁-C₄, ou un groupe aminocarbothioyle libre ou substitué par un ou 2 alkyles en C₁-C₄;
- R''₈ représente R′₈ ou un groupe (CH₂)₂NH₂ libre ou substitué par un ou deux alkyles en C₁-C₄;
- R₉ représente l'hydrogène, un halogène, un groupe (CH₂)ᵣ OR₁₀, un groupe (CH₂)ᵣNR₁₁R₁₂, un groupe (CH₂)ₛ CONR₁₁ R′₁₁, un groupe azido ;
- R₁₀ représente l'hydrogène, un alkyle en C₁-C₄, un mésyle ou un tosyle ;
- R₁₁, R′₁₁ et R₁₂ représentent chacun un hydrogène ou un alkyle en C₁-C₄ ou R₁₁ représente l'hydrogène et R₁₂ représente un benzyloxycarbonyle ou un alcoxycarbonyle en C₁-C₄ ;
- n représente 0, 1 ou 2 ;
- m représente 0, 1 ou 2 ;
- q représente 0, 1, 2 ou 3 ;
- r représente 0, 1, 2 ou 3, avec la limitation que r n'est pas nul lorsque R₈ ou R₉ est en position alpha de l'azote amidique intracyclique ;
- s représente 0 ou 1 ;
ainsi que ses sels éventuels.

2. Composé selon la revendication 1 dans lequel R₁ représente un atome de chlore, de brome ou un groupe méthoxy et n = 1.

3. Composé selon l'une quelconque des revendication1 ou 2 dans lequel R₂ représente un chlorophényle ou un méthoxyphényle ou un cyclohexyle.

4. Composé selon l'une quelconque des revendications 1 à 3 dans lequel R₅ représente un phényle substitué en position 3 et 4, ou en position 2 et 4 par un groupe méthoxy, ou bien R₅ représente un phényle substitué en position 4 par un méthyle.

5. Composé selon l'une quelconque des revendications 1 à 4 dans lequel m = 0.

6. Composé selon l'une quelconque des revendications 1 à 5 dans lequel R₄ représente CONR₆R₇ et NR₆R₇ représente un groupe pyrrolidino substitué en position 2 par un groupe (CH₂)_{q} lui-même substitué par un groupe carboxyle, ou un groupe carbamoyle avec q = 0,1,2, ou 3.

7. Composé selon l'une des revendications 1 à 5 dans lequel R₄ représente CONR₆R₇ et NR₆R₇ représente un groupe pipéridino substitué en position 4 par un groupe amino, un alkylamino en C₁-C₄ ou un dialkylamino en C₁-C₄.

8. Composé selon l'une quelconque des revendications 1 à 5 dans lequel R₄ représente CONR₆R₇ et NR₆R₇ représente un groupe thiazolidino substitué par un groupe (CH₂)_{q} lui-même substitué par un groupe carboxyle ou un groupe carbamoyle avec q = 0, 1, 2 ou 3.

9. Composé selon l'une quelconque des revendications 1 à 5 dans lequel R₄ représente CONR₆R₇ et NR₆R₇ représente un groupe pyrrolidino substitué en position 2 par un groupe (CH₂)_{q} lui-même substitué par un groupe carboxyle ou un groupe carbamoyle et substitué en position 4 par un groupe amino, un alkylamino en C₁-C₄ ou un dialkylamino en C₁-C₄.

10. Composé selon l'une quelconque des revendication 1 à 5 dans lequel R₄ représente CONR₆R₇, R₆ représentant un alkyle en C₁-C₄ et R₇ représentant un groupe (CH₂)ᵣ lui-même substitué par un groupe carboxyle ou un groupe carbamoyle avec r = 1,2 ou 3.

11. Composé selon l'une quelconque des revendications 1 à 10 sous forme d'isomère cis, dans lequel R₂ et R₄ sont du même côté du cycle indoline.

12. Un procédé pour la préparation d'un composé (I) selon la revendication 1 caractérisé en ce que :
a) on fait réagir sur un dérivé d'amino-2 phénone de formule : 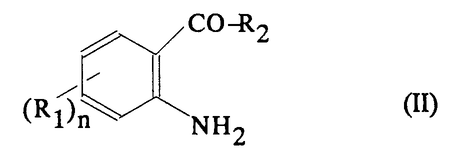 dans laquelle R₁, R₂ et n ont les significations indiquées ci-dessus pour (I) dans la revendication 1, un dérivé sulfonyle de formule :
Hal - SO₂ - (CH₂)ₘ - R₅ (III)
dans laquelle
- Hal représente un halogène, de préférence le chlore ou le brome,
- m et R₅ ont les significations indiquées ci-dessus pour (I) dans la revendication 1,
b) le composé ainsi obtenu de formule : 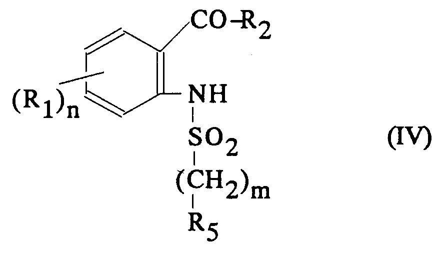 est traité par un dérivé halogéné de formule :
Hal′ - CH₂ COA (V)
dans laquelle
Hal′ représente un halogène préférentiellement le brome, et A représente soit le groupe NR₆R₇, soit le groupe OR dans lequel R est un tertiobutyle ou un benzyle ;
c) le cas échéant, lorsque A représente un groupe OR, l'ester ainsi obtenu de formule : 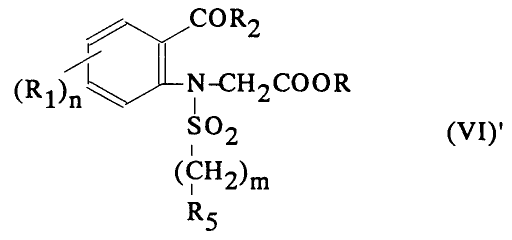 est déprotégé dans les conditions convenables ;
d) le cas échéant, l'acide ainsi obtenu à l'étape c) de formule: 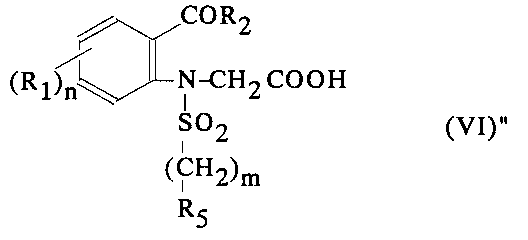 ou son chlorure d'acide de formule : 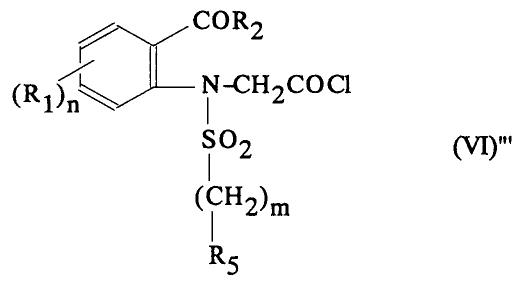 est traité par un composé HNR₆R₇ selon les techniques convenables du couplage amidique ;
e) le composé ainsi obtenu à l'étape b) ou à l'étape d), de formule : 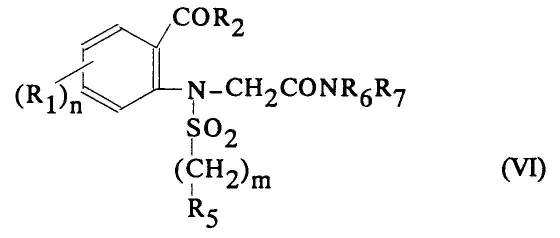 est cyclisé en milieu basique pour préparer le composé (I) selon la revendication 1 ;
f) on sépare éventuellement les isomères cis et trans du composé (I) selon la revendication 1, et, éventuellement on sépare les énantiomères.

13. Un composé de formule: 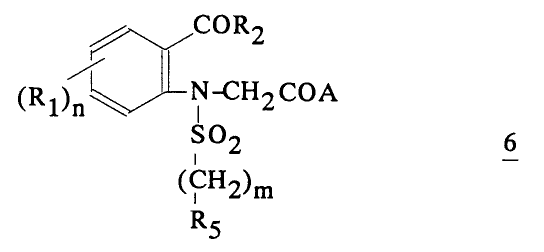 dans laquelle
A′ représente un groupe choisi parmi: NR₆R₇, OH, OtBu, OBz;
- R₁ représente un atome d'halogène, un alkyle en C₁-C₄, un hydroxyle, un alcoxy en C₁-C₄, un groupe benzyloxy, un groupe cyano, un groupe trifluorométhyle, un groupe nitro ou un groupe amino ;
- R₂ représente un alkyle en C₁-C₆; un cycloalkyle en C₃-C₇, un cycloalcène en C₅-C₇, un phényle non substitué ou substitué une ou plusieurs fois par un alkyle en C₁-C₄, un alcoxy en C₁-C₄, un halogène, un groupe trifluorométhyle, un groupe amino, ou R₂ représente un nitrophényle non substitué ou substitué une fois par un groupe trifluorométhyle ou une ou plusieurs fois par un alkyle en C₁-C₄, un alcoxy en C₁-C₄ ou un halogène ;
- R₅ représente un alkyle en C₁-C₄ ; un naphtyl-1 ; un naphtyl-2 ; un diméthylamino-5 naphtyl-1 ; un phényle non substitué ou substitué par un ou plusieurs substituants choisis parmi un halogène, un alkyle en C₁-C₄, un groupe trifluorométhyle, un groupe amino libre ou substitué par un ou 2 alkyles en C₁-C₄, un hydroxyle, un alcoxy en C₁-C₄, un alcénoxy en C₂-C₄, un alkylthio en C₁-C₄, un groupe trifluorométhoxy, un groupe benzyloxy, un groupe cyano, un groupe carboxy, un groupe alcoxycarbonyle en C₁-C₄, un groupe carbamoyle libre ou substitué par un ou deux alkyles en C₁-C₄ ou un groupe alkylamido en C₁-C₄, ou R₅ représente un nitrophényle non substitué ou substitué une fois par un groupe trifluorométhyle ou un alcénoxy en C₂-C₄, ou une ou plusieurs fois par un halogène, un alkyle en C₁-C₄, un alcoxy en C₁-C₄, un alkylthio en C₁-C₄, un groupe trifluorométhoxy ou un groupe benzyloxy ;
- R₆ représente un alkyle en C₁-C₆, ou R₆ est semblable à R₇ ;
- R₇ représente un groupe pipéridin-4-yl ; un groupe azétidin-3-yl, lesdits groupes étant substitués ou non sur l'azote par un alkyle en C₁-C₄, par un benzyloxycarbonyle ou par un alcoxycarbonyle en C₁-C₄ ; un groupe (CH₂)ᵣ lui-même substitué par un groupe pyridyl -2, -3 ou -4, par un groupe hydroxyle, par un groupe amino libre ou substitué par un ou deux alkyles en C₁-C₄, un groupe carboxyle, un groupe alcoxycarbonyle en C₁-C₄, un groupe benzyloxycarbonyle, un groupe carbamoyle libre ou substitué par un ou 2 alkyles en C₁-C₄ ;
- ou R₆ et R₇ ensemble constituent avec l'atome d'azote auquel ils sont liés un hétérocycle choisi parmi :
. la morpholine,
. la thiomorpholine,
. la thiazolidine ou la 2,2-diméthylthiazolidine substitué ou non par R₈,
. la pipérazine non substituée ou substituée en 4 par un groupe R''₈ ;
. un cycle insaturé monoazoté à 5 chaînons substitué par R₈ ou
. un cycle saturé monoazoté à 3, 4, 5, 6 ou 7 chaînons substitué par R₈ et R₉ ;
- R₈ représente R′₈ ou un groupe (CH₂)ᵣ lui-même substitué par un hydroxyle ou par un amino libre ou substitué par un ou deux alkyles en C₁-C₄ ;
- R′₈ représente un groupe (CH₂)_{q} lui-même substitué par un groupe carboxyle, un groupe alcoxycarbonyle en C₁-C₄, un groupe benzyloxycarbonyle, un groupe carbamoyle libre ou substitué par un hydroxyle ou par un ou 2 alkyles en C₁-C₄, ou un groupe aminocarbothioyle libre ou substitué par un ou 2 alkyles en C₁-C₄ ;
- R''₈ représente R′₈ ou un groupe (CH₂)₂NH₂ libre ou substitué par un ou deux alkyles en C₁-C₄;
- R₉ représente l'hydrogène, un halogène, un groupe (CH₂)ᵣ OR₁₀, un groupe (CH₂)ᵣNR₁₁R₁₂, un groupe (CH₂)ₛ CONR₁₁ R′₁₁, un groupe azido ;
- R₁₀ représente l'hydrogène, un alkyle en C₁-C₄, un mésyle ou un tosyle;
- R₁₁, R'₁₁ et R₁₂ représentent chacun un hydrogène ou un alkyle en C₁-C₄ ou R₁₁ représente l'hydrogène et R₁₂ représente un benzyloxycarbonyle ou un alcoxycarbonyle en C₁-C₄ ;
- n représente 0, 1 ou 2 ;
- m représente 0, 1 ou 2 ;
- q représente 0, 1, 2 ou 3 ;
- r représente 0, 1, 2 ou 3, avec la limitation que r n'est pas nul lorsque R₈ ou R₉ est en position alpha de l'azote amidique intracyclique ;
- s représente 0 ou 1.

14. Utilisation d'un composé de formule : 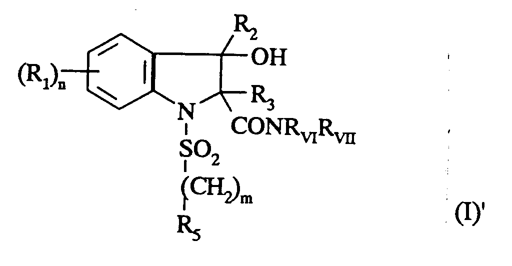 dans laquelle R₁, R₂, R₃, R₅, m et n ont les significations données pour les composés de formule (I) dans la revendication 1 et
- R_{VI} représente un alkyle en C₁-C₆,
- R_{VII} représente un groupe (CH₂)ᵣCOOH avec r = 1, 2 ou 3.
- ou R_{VI} et R_{VII} ensemble avec l'atome d'azote auquel ils sont liés constituent un hétérocycle choisi parmi :
. la thiazolidine ou la 2,2-diméthylthiazolidine substituées par un groupe (CH₂)_{q}COOH,
. la pipérazine substituée en 4 par un groupe (CH₂)_{q}COOH,
. un cycle insaturé monoazoté à 5 chaînons substitué par un groupe (CH₂)_{q}COOH,
. un cycle saturé monoazoté à 3, 4, 5, 6 ou 7 chaînons substitué par un groupe (CH₂)_{q}COOH,
avec q = 0, 1, 2 ou 3,
pour la préparation d'un composé de formule (I)'' ayant la même configuration autour de la liaison 2, 3 de l'indoline que le produit de départ 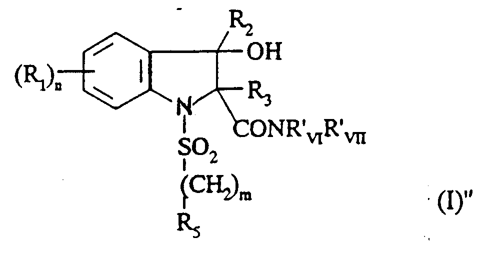 dans laquelle :
- R₁,R₂, R₃, R₅,m et n sont tels que définis ci-dessus,
- R'_{VI} représente un alkyle en C₁-C₆,
- R'_{VII} représente le groupe (CH₂)ᵣH,
ou R'_{VI} et R'_{VII} ensemble avec l'atome d'azote auquel ils sont liés constituent un hétérocycle choisi parmi :
. la thiazolidine ou la 2,2-diméthylthiazolidine substituée par un groupe (CH₂)_{q}H,
. la pipérazine substituée en 4 par un groupe (CH₂)_{q} H,
. un cycle insaturé monoazoté à 5 chaînons substitué par un groupe (CH₂)_{q}H,
. un cycle saturé monoazoté à 3, 4, 5, 6 ou 7 chaînons substitué par un groupe (CH₂)_{q}H.

15. Composition pharmaceutique contenant à titre de principe actif, un composé selon l'une des revendications 1 à 11.

16. Composition pharmaceutique contenant un composé selon l'une quelconque des revendications 1 à 11, en association avec un autre principe actif.

## Revendications (Revendications pour l'(les) Etat(s) contractant(s) suivant(s): ES)

1. Procédé pour la préparation d'un composé de formule (I) 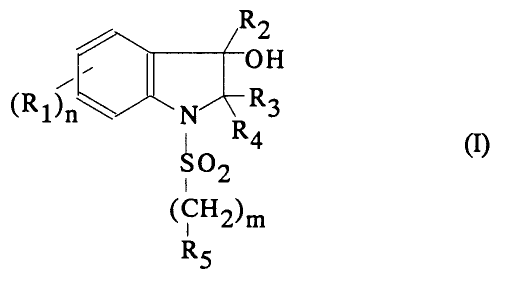 dans laquelle
- R₁ représente un atome d'halogène, un alkyle en C₁-C₄, un hydroxyle, un alcoxy en C₁-C₄, un groupe benzyloxy, un groupe cyano, un groupe trifluorométhyle, un groupe nitro ou un groupe amino ;
- R₂ représente un alkyle en C₁-C₆, un cycloalkyle en C₃-C₇, un cycloalcène en C₅-C₇, un phényle non substitué ou substitué une ou plusieurs fois par un alkyle en C₁-C₄, un alcoxy en C₁-C₄, un halogène, un groupe trifluorométhyle, un groupe amino, ou R₂ représente un nitrophényle non substitué ou substitué une fois par un groupe trifluorométhyle ou une ou plusieurs fois par un alkyle en C₁-C₄ ou un halogène ;
- R₃ représente un atome d'hydrogène ;
- R₄ représente un groupe carbamoyle de formule CONR₆R₇ ;
- R₅ représente un alkyle en C₁-C₄ ; un naphtyl-1 ; un naphtyl-2 ; un diméthylamino-5 naphtyl-1; un phényle non substitué ou substitué par un ou plusieurs substituants choisis parmi un halogène, un alkyle en C₁-C₄, un groupe trifluorométhyle, un groupe amino libre ou substitué par un ou 2 alkyles en C₁-C₄, un hydroxyle, un alcoxy en C₁-C₄, un alcénoxy en C₂-C₄, un alkylthio en C₁-C₄, un groupe trifluorométhoxy, un groupe benzyloxy, un groupe cyano, un groupe carboxy, un groupe alcoxycarbonyle en C₁-C₄, un groupe carbamoyle libre ou substitué par un ou deux alkyles en C₁-C₄ ou un groupe alkylamido en C₁-C₄, ou R₅ représente un nitrophényle non substitué ou substitué une fois par un groupe trifluorométhyle ou un alcénoxy en C₂-C₄ ou une ou plusieurs fois par un halogène, un alkyle en C₁-C₄, un alcoxy en C₁-C₄, un alkylthio en C₁-C₄, un groupe trifluorométhoxy ou un groupe benzyloxy ;
- R₆ représente un alkyle en C₁-C₆, ou R₆ est semblable à R₇ ;
- R₇ représente un groupe pipéridin-4-yl ; un groupe azétidin-3-yl, lesdits groupes étant substitués ou non sur l'azote par un alkyle en C₁-C₄, par un benzyloxycarbonyle ou par un alcoxycarbonyle en C₁-C₄ ; un groupe (CH₂)ᵣ lui-même substitué par un groupe pyridyl -2, -3 ou -4, par un groupe hydroxyle, par un groupe amino libre ou substitué par un ou deux alkyles en C₁-C₄, un groupe carboxyle, un groupe alcoxycarbonyle en C₁-C₄, un groupe benzyloxycarbonyle, un groupe carbamoyle libre ou substitué par un ou 2 alkyles en C₁-C₄;
- ou R₆ et R₇ ensemble constituent avec l'atome d'azote auquel ils sont liés un hétérocycle choisi parmi :
. la morpholine,
. la thiomorpholine,
. la thiazolidine ou la 2,2-diméthylthiazolidine non substituées ou substituées par R₈,
. la pipérazine non substituée ou substituée en 4 par un groupe R''₈,
. un cycle insaturé monoazoté à 5 chaînons substitué par R₈ ou un cycle saturé monoazoté à 3, 4, 5, 6 ou 7 chaînons substitué par R₈ et R₉ ;
- R₈ représente R′₈ ou un groupe (CH₂)ᵣ lui-même substitué par un hydroxyle ou par un amino libre ou substitué par un ou deux alkyles en C₁-C₄ ;
- R′₈ représente un groupe (CH₂)_{q} lui-même substitué par un groupe carboxyle, un groupe alcoxycarbonyle en C₁-C₄, un groupe benzyloxycarbonyle, un groupe carbamoyle libre ou substitué par un hydroxyle ou par un ou 2 alkyles en C₁-C₄, ou un groupe aminocarbothioyle libre ou substitué par un ou 2 alkyles en C₁-C₄ ;
- R''₈ représente R′₈ ou un groupe (CH₂)₂NH₂ libre ou substitué par un ou deux alkyles en C₁-C₄ ;
- R₉ représente l'hydrogène, un halogène, un groupe (CH₂)ᵣ OR₁₀, un groupe (CH₂)ᵣ NR₁₁ R₁₂, un groupe (CH₂)ₛ CONR₁₁ R′₁₁, un groupe azido;
- R₁₀ représente l'hydrogène, un alkyle en C₁-C₄, un mésyle ou un tosyle ;
- R₁₁, R′₁₁ et R₁₂ représentent chacun un hydrogène ou un alkyle en C₁-C₄ ou R₁₁ représente l'hydrogène et R₁₂ représente un benzyloxycarbonyle ou un alcoxycarbonyle en C₁-C₄ ;
- n représente 0, 1 ou 2 ;
- m représente 0, 1 ou 2 ;
- q représente 0, 1, 2 ou 3 ;
- r représente 0, 1, 2 ou 3, avec la limitation que r n'est pas nul lorsque R₈ ou R₉ est en position alpha de l'azote amidique intracyclique ;
- s représente 0 ou 1 ;
ainsi que de ses sels éventuels,
caractérisé en ce que :
a) on fait réagir sur un dérivé d'amino-2 phénone de formule : 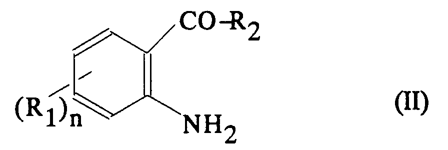 dans laquelle R₁, R₂ et n ont les significations indiquées ci-dessus, un dérivé sulfonyle de formule :
Hal - SO₂ - (CH₂)ₘ - R₅ (III)
dans laquelle
- Hal représente un halogène, de préférence le chlore ou le brome,
- m et R₅ ont les significations indiquées ci-dessus,
b) le composé ainsi obtenu de formule : 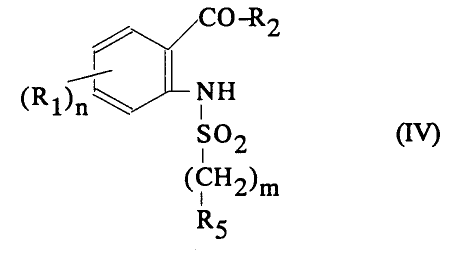 est traité par un dérivé halogéné de formule :
Hal′ - CH₂ COA (V)
dans laquelle
Hal′ représente un halogène préférentiellement le brome, et A représente soit le groupe NR₆R₇, soit le groupe OR dans lequel R est un tertiobutyle ou un benzyle ;
c) le cas échéant, lorsque A représente un groupe OR, l'ester ainsi obtenu de formule : 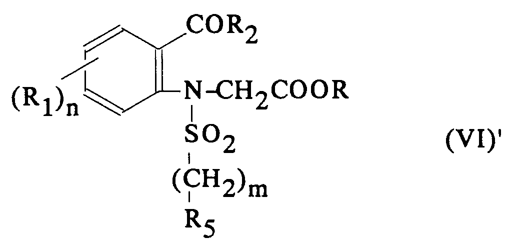 est déprotégé dans les conditions convenables ;
d) le cas échéant, l'acide ainsi obtenu à l'étape c) de formule : 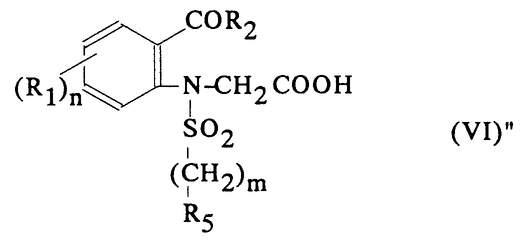 ou son chlorure d'acide de formule : 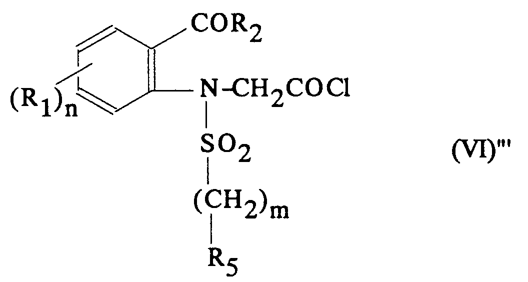 est traité par un composé HNR₆R₇ selon les techniques convenables du couplage amidique ;
e) le composé ainsi obtenu à l'étape b) ou à l'étape d), de formule : 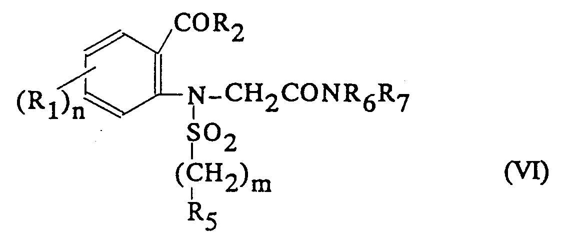 est cyclisé en milieu basique pour préparer le composé (I);
f) on sépare éventuellement les isomères cis et trans du composé (I) et,, éventuellement on sépare les énantiomères, les composés de formule (I) étant éventuellement transformés en leurs sels avec des acides minéraux ou organiques ou avec des bases minérales ou organiques.

2. Procédé selon la revendication 1 pour l'obtention d'un composé de formule (I) dans laquelle R₁ représente un atome de chlore, de brome ou un groupe méthoxy et n = 1,
caractérisé en ce qu'on utilise dans l'étape a) un composé de formule (II) dans laquelle R₁ et n sont tels que définis ci-dessus.

3. Procédé selon l'une des revendications 1 ou 2, pour la préparation d'un composé de formule (I) dans laquelle R₂ représente un chlorophényle ou un méthoxyphényle ou un cyclohexyle, caractérisé en ce qu'on utilise dans l'étape a) un composé de formule (II) dans laquelle R₂ est tel que défini ci-dessus.

4. Procédé selon l'une quelconque des revendications 1 à 3, pour la préparation d'un composé de formule (I) dans laquelle R₅ représente un phényle substitué en position 3 et 4, ou en position 2 et 4 par un groupe méthoxy, ou bien R₅ représente un phényle substitué en position 4 par un méthyle, caractérisé en ce qu'on utilise dans l'étape a) un composé de formule (III) dans laquelle R₅ est tel que défini ci-dessus.

5. Procédé selon l'une quelconque des revendications 1 à 4, pour la préparation d'un composé de formule (I) dans laquelle m = 0, caractérisé en ce qu'on utilise dans l'étape a) un composé de formule (III) dans laquelle m = 0.

6. Procédé selon l'une quelconque des revendications 1 à 5, pour la préparation d'un composé de formule (I) dans laquelle R₄ représente CONR₆R₇ et NR₆R₇ représente un groupe pyrrolidino substitué en position 2 par un groupe (CH₂)_{q} lui-même substitué par un groupe carboxyle, ou un groupe carbamoyle avec q = 0, 1, 2 ou 3, caractérisé en ce qu'on utilise à l'étape b) un composé de formule (V) dans laquelle A représente un groupe NR₆R₇ tel que défini ci-dessus.

7. Procédé selon l'une quelconque des revendications 1 à 5, pour la préparation d'un composé de formule (I) dans laquelle R₄ représente CONR₆R₇ et NR₆R₇ représente un groupe pipéridino substitué en position 4 par un groupe amino, un alkylamino en C₁-C₄ ou un dialkylamino en C₁-C₄, caractérisé en ce qu'on utilise à l'étape b) un composé de formule (V) dans laquelle A représente un groupe NR₆R₇ tel que défini ci-dessus.

8. Procédé selon l'une quelconque des revendications 1 à 5, pour la préparation d'un composé de formule (I) dans laquelle R₄ représente CONR₆R₇ et NR₆R₇ représente un groupe thiazolidino substitué par un groupe (CH₂)_{q} lui-même substitué par un groupe carboxyle ou un groupe carbamoyle avec q = 0, 1, 2 ou 3, caractérisé en ce qu'on utilise à l'étape b) un composé de formule (V) dans laquelle A représente un groupe NR₆R₇ tel que défini ci-dessus.

9. Procédé selon l'une quelconque des revendications 1 à 5, pour la préparation d'un composé de formule (I) dans laquelle R₄ représente CONR₆R₇ et NR₆R₇ représente un groupe pyrrolidino substitué en position 2 par un groupe (CH₂)_{q} lui-même substitué par un groupe carboxyle ou un groupe carbamoyle et substitué en position 4 par un groupe amino, un alkylamino en C₁-C₄ ou un dialkylamino en C₁-C₄, caractérisé en ce qu'on utilise à l'étape b) un composé de formule (V) dans laquelle A représente un groupe NR₆R₇ tel que défini ci-dessus.

10. Procédé selon l'une quelconque des revendications 1 à 5, pour la préparation d'un composé de formule (I) dans laquelle R₄ représente CONR₆R₇, R₆ représentant un alkyle en C₁-C₄ et R₇ représentant un groupe (CH₂)ᵣ lui-même substitué par un groupe carboxyle ou un groupe carbamoyle avec r = 1, 2 ou 3, caractérisé en ce qu'on utilise à l'étape b) un composé de formule (V) dans laquelle A représente un groupe NR₆R₇ tel que défini ci-dessus.

11. Procédé selon l'une quelconque des revendications 1 à 10, pour la préparation d'un composé de formule (I) sous forme d'isomère cis, dans lequel R₂ et R₄ sont du même côté du cycle indoline, caractérisé en ce qu'à l'étape f) on sépare l'isomère cis de l'isomère trans par chromatographie ou cristallisation fractionnée.

12. Procédé pour la préparation d'un composé de formule 6 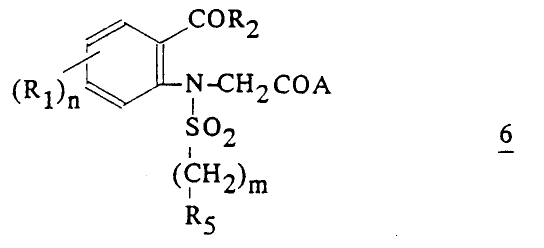 dans laquelle
A′ représente un groupe choisi parmi : NR₆R₇, OH, OtBu, OBz ;
- R₁ représente un atome d'halogène, un alkyle en C₁-C₄, un hydroxyle, un alcoxy en C₁-C₄, un groupe benzyloxy, un groupe cyano, un groupe trifluorométhyle, un groupe nitro ou un groupe amino ;
- R₂ représente un alkyle en C₁-C₆; un cycloalkyle en C₃-C₇, un cycloalcène en C₅-C₇, un phényle non substitué ou substitué une ou plusieurs fois par un alkyle en C₁-C₄, un alcoxy en C₁-C₄, un halogène, un groupe trifluorométhyle, un groupe amino, ou R₂ représente un nitrophényle non substitué ou substitué une fois par un groupe trifluorométhyle ou une ou plusieurs fois par un alkyle en C₁-C₄, un alcoxy en C₁-C₄ ou un halogène ;
- R₅ représente un alkyle en C₁-C₄ ; un naphtyl-1 ; un naphtyl-2 ; un diméthylamino-5 naphtyl-1 ; un phényle non substitué ou substitué par un ou plusieurs substituants choisis parmi un halogène, un alkyle en C₁-C₄, un groupe trifluorométhyle, un groupe amino libre ou substitué par un ou 2 alkyles en C₁-C₄, un hydroxyle, un alcoxy en C₁-C₄, un alcénoxy en C₂-C₄, un alkylthio en C₁-C₄, un groupe trifluorométhoxy, un groupe benzyloxy, un groupe cyano, un groupe carboxy, un groupe alcoxycarbonyle en C₁-C₄, un groupe carbamoyle libre ou substitué par un ou deux alkyles en C₁-C₄ ou un groupe alkylamido en C₁-C₄, ou R₅ représente un nitrophényle non substitué ou substitué une fois par un groupe trifluorométhyle ou un alcénoxy en C₂-C₄, ou une ou plusieurs fois par un halogène, un alkyle en C₁-C₄, un alcoxy en C₁-C₄, un alkylthio en C₁-C₄, un groupe trifluorométhoxy ou un groupe benzyloxy ;
- R₆ représente un alkyle en C₁-C₆, ou R₆ est semblable à R₇ ;
- R₇ représente un groupe pipéridin-4-yl ; un groupe azétidin-3-yl, lesdits groupes étant substitués ou non sur l'azote par un alkyle en C₁-C₄ par un benzyloxycarbonyle ou par un alcoxycarbonyle en C₁-C₄ ; un groupe (CH₂)ᵣ lui-même substitué par un groupe pyridyl -2, -3 ou -4, par un groupe hydroxyle, par un groupe amino libre ou substitué par un ou deux alkyles en C₁-C₄, un groupe carboxyle, un groupe alcoxycarbonyle en C₁-C₄, un groupe benzyloxycarbonyle, un groupe carbamoyle libre ou substitué par un ou 2 alkyles en C₁-C₄ ;
- ou R₆ et R₇ ensemble constituent avec l'atome d'azote auquel ils sont liés un hétérocycle choisi parmi :
. la morpholine,
. la thiomorpholine,
. la thiazolidine ou la 2,2-diméthylthiazolidine substitué ou non par R₈,
. la pipérazine non substituée ou substituée en 4 par un groupe R''₈ ;
. un cycle insaturé monoazoté à 5 chaînons substitué par R₈ ou
. un cycle saturé monoazoté à 3, 4, 5, 6 ou 7 chaînons substitué par R₈ et R₉ ;
- R₈ représente R'₈ ou un groupe (CH₂)ᵣ lui-même substitué par un hydroxyle ou par un amino libre ou substitué par un ou deux alkyles en C₁-C₄ ;
- R'₈ représente un groupe (CH₂)_{q} lui-même substitué par un groupe carboxyle, un groupe alcoxycarbonyle en C₁-C₄, un groupe benzyloxycarbonyle, un groupe carbamoyle libre ou substitué par un hydroxyle ou par un ou 2 alkyles en C₁-C₄, ou un groupe aminocarbothioyle libre ou substitué par un ou 2 alkyles en C₁-C₄ ;
- R''₈ représente R'₈ ou un groupe (CH₂)₂NH₂ libre ou substitué par un ou deux alkyles en C₁-C₄ ;
- R₉ représente l'hydrogène, un halogène, un groupe (CH₂)ᵣ OR₁₀, un groupe (CH₂)ᵣ NR₁₁ R₁₂, un groupe (CH₂)ₛ CONR₁₁ R'₁₁, un groupe azido ;
- R₁₀ représente l'hydrogène, un alkyle en C₁-C₄, un mésyle ou un tosyle ;
- R₁₁, R'₁₁ et R₁₂ représentent chacun un hydrogène ou un alkyle en C₁-C₄ ou R₁₁ représente l'hydrogène et R₁₂ représente un benzyloxycarbonyle ou un alcoxycarbonyle en C₁-C₄ ;
- n représente 0, 1 ou 2 ;
- m représente 0, 1 ou 2 ;
- q représente 0, 1, 2 ou 3 ;
- r représente 0, 1, 2 ou 3, avec la limitation que r n'est pas nul lorsque R₈ ou R₉ est en position alpha de l'azote amidique intracyclique ;
- s représente 0 ou 1,
caractérisé en ce que :
a) on fait réagir sur un dérivé d'amino-2 phénone de formule : 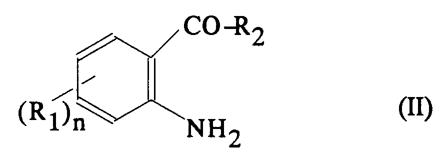 dans laquelle R₁, R₂ et n ont les significations indiquées ci-dessus pour (I) dans la revendication 1, un dérivé sulfonyle de formule :
Hal - SO₂ - (CH₂)ₘ - R₅ (III)
dans laquelle
- Hal représente un halogène, de préférence le chlore ou le brome,
- m et R₅ ont les significations indiquées ci-dessus pour (I) dans la revendication 1,
b) le composé ainsi obtenu de formule : 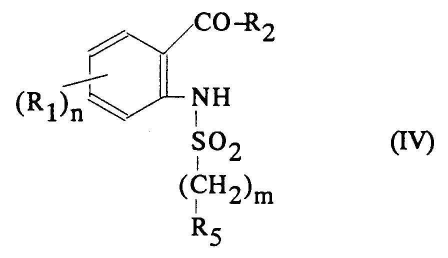 est traité par un dérivé halogéné de formule :
Hal′ - CH₂ COA (V)
dans laquelle
Hal′ représente un halogène préférentiellement le brome, et A représente soit le groupe NR₆R₇, soit le groupe OR dans lequel R est un tertiobutyle ou un benzyle ;
c) le cas échéant, lorsque A représente un groupe OR, l'ester ainsi obtenu de formule : 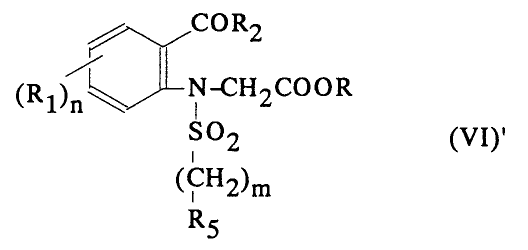 est isolé ou, le cas échéant, déprotégé dans les conditions convenables ,et l'acide ainsi obtenu de formule : 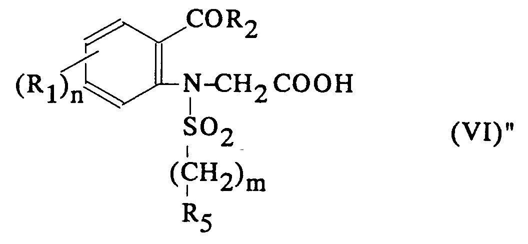 est isolé,
d) ou, le cas échéant, l'acide ainsi obtenu à l'étape c) est traité par un composé HNR₆R₇ selon les techniques convenables du couplage amidique pour obtenir un composé de formule

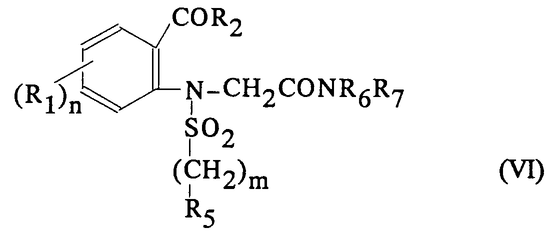

13. Procédé pour la préparation d'un composé de formule (I)'' ayant la même configuration autour de la liaison 2, 3 de l'indoline que le produit de départ 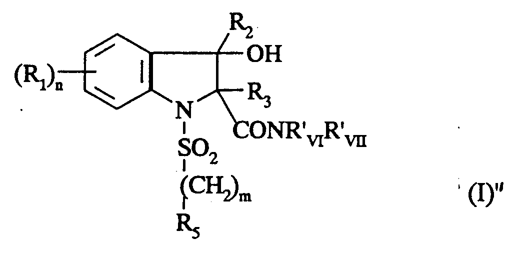 dans laquelle :
- R₁, R₂, R₃, R₅,m et n sont tels que définis pour les composés de formule (I) dans la revendication 1,
- R'_{VI} représente un alkyle en C₁-C₆,
- R'_{VII} représente le groupe (CH₂)ᵣH,
ou R'_{VI} et R'_{VII} ensemble avec l'atome d'azote auquel ils sont liés constituent un hétérocycle choisi parmi :
. la thiazolidine ou la 2,2-diméthylthiazolidine substituée par un groupe (CH₂)_{q}H,
. la pipérazine substituée en 4 par un groupe (CH₂)_{q} H,
. un cycle insaturé monoazoté à 5 chaînons substitué par un groupe (CH₂)_{q}H,
. un cycle saturé monoazoté à 3, 4, 5, 6 ou 7 chaînons substitué par un groupe (CH₂)_{q}H,
caractérisé en ce qu'on soumet un composé de formule (I)' 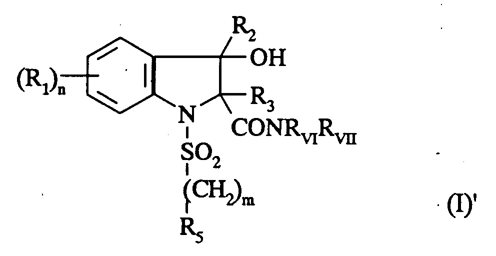 dans laquelle R₁, R₂, R₃, R₅, m et n ont les significations données pour les composés de formule (I) dans la revendication 1 et
- R_{VI} représente un alkyle en C₁-C₆,
- R_{VII} représente un groupe (CH₂)ᵣCOOH avec r = 1, 2 ou 3.
- ou R_{VI} et R_{VII} ensemble avec l'atome d'azote auquel ils sont liés constituent un hétérocycle choisi parmi :
. la thiazolidine ou la 2,2-diméthylthiazolidine substituées par un groupe (CH₂)_{q}COOH,
. la pipérazine substituée en 4 par un groupe (CH₂)_{q}COOH,
. un cycle insaturé monoazoté à 5 chaînons substitué par un groupe (CH₂)_{q}COOH,
. un cycle saturé monoazoté à 3, 4, 5, 6 ou 7 chaînons substitué par un groupe (CH₂)_{q}COOH,
avec q = 0, 1, 2 ou 3,
à une décarboxylation radicalaire.

14. Procédé pour la préparation d'une composition pharmaceutique, caractérisé en ce que l'on mélange, en tant que principe actif, un composé de formule (I) préparé par le procédé selon l'une quelconque des revendications 1 à 11 avec un véhicule pharmaceutiquement acceptable.

15. Procédé pour la préparation d'une composition pharmaceutique, caractérisé en ce que l'on mélange, en tant que principe actif, un composé de formule (I) préparé par le procédé selon l'une quelconque des revendications 1 à 11 en association avec un autre principe actif, avec un véhicule pharmaceutiquement acceptable.

## Revendications (Revendications pour l'(les) Etat(s) contractant(s) suivant(s): GR)

1. Un composé de formule : 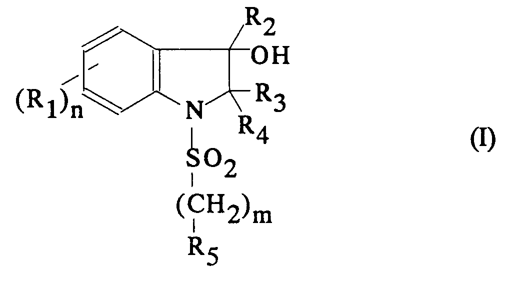 dans laquelle
- R₁ représente un atome d'halogène, un alkyle en C₁-C₄, un hydroxyle, un alcoxy en C₁-C₄, un groupe benzyloxy, un groupe cyano, un groupe trifluorométhyle, un groupe nitro ou un groupe amino ;
- R₂ représente un alkyle en C₁-C₆, un cycloalkyle en C₃-C₇, un cycloalcène en C₅-C₇, un phényle non substitué ou substitué une ou plusieurs fois par un alkyle en C₁-C₄, un alcoxy en C₁-C₄, un halogène, un groupe trifluorométhyle, un groupe amino, ou R₂ représente un nitrophényle non substitué ou substitué une fois par un groupe trifluorométhyle ou une ou plusieurs fois par un alkyle en C₁-C₄ ou un halogène ;
- R₃ représente un atome d'hydrogène ;
- R₄ représente un groupe carbamoyle de formule CONR₆R₇ ;
- R₅ représente un alkyle en C₁-C₄ ; un naphtyl-1 ; un naphtyl-2 ; un diméthylamino-5 naphtyl-1 ; un phényle non substitué ou substitué par un ou plusieurs substituants choisis parmi un halogène, un alkyle en C₁-C₄, un groupe trifluorométhyle, un groupe amino libre ou substitué par un ou 2 alkyles en C₁-C₄, un hydroxyle, un alcoxy en C₁-C₄, un alcénoxy en C₂-C₄, un alkylthio en C₁-C₄, un groupe trifluorométhoxy, un groupe benzyloxy, un groupe cyano, un groupe carboxy, un groupe alcoxycarbonyle en C₁-C₄, un groupe carbamoyle libre ou substitué par un ou deux alkyles en C₁-C₄ ou un groupe alkylamido en C₁-C₄, ou R₅ représente un nitrophényle non substitué ou substitué une fois par un groupe trifluorométhyle ou un alcénoxy en C₂-C₄ ou une ou plusieurs fois par un halogène, un alkyle en C₁-C₄, un alcoxy en C₁-C₄, un alkylthio en C₁-C₄, un groupe trifluorométhoxy ou un groupe benzyloxy ;
- R₆ représente un alkyle en C₁-C₆, ou R₆ est semblable à R₇ ;
- R₇ représente un groupe pipéridin-4-yl ; un groupe azétidin-3-yl, lesdits groupes étant substitués ou non sur l'azote par un alkyle en C₁-C₄, par un benzyloxycarbonyle ou par un alcoxycarbonyle en C₁-C₄ ; un groupe (CH₂)ᵣ lui-même substitué par un groupe pyridyl -2, -3 ou -4, par un groupe hydroxyle, par un groupe amino libre ou substitué par un ou deux alkyles en C₁-C₄, un groupe carboxyle, un groupe alcoxycarbonyle en C₁-C₄, un groupe benzyloxycarbonyle, un groupe carbamoyle libre ou substitué par un ou 2 alkyles en C₁-C₄ ;
- ou R₆ et R₇ ensemble constituent avec l'atome d'azote auquel ils sont liés un hétérocycle choisi parmi :
. la morpholine,
. la thiomorpholine,
. la thiazolidine ou la 2,2-diméthylthiazolidine non substituées ou substituées par R₈,
. la pipérazine non substituée ou substituée en 4 par un groupe R''₈,
. un cycle insaturé monoazoté à 5 chaînons substitué par R₈ ou un cycle saturé monoazoté à 3, 4, 5, 6 ou 7 chaînons substitué par R₈ et R₉ ;
- R₈ représente R′₈ ou un groupe (CH₂)ᵣ lui-même substitué par un hydroxyle ou par un amino libre ou substitué par un ou deux alkyles en C₁-C₄ ;
- R′₈ représente un groupe (CH₂)_{q} lui-même substitué par un groupe carboxyle, un groupe alcoxycarbonyle en C₁-C₄, un groupe benzyloxycarbonyle, un groupe carbamoyle libre ou substitué par un hydroxyle ou par un ou 2 alkyles en C₁-C₄, ou un groupe aminocarbothioyle libre ou substitué par un ou 2 alkyles en C₁-C₄ ;
- R''₈ représente R′₈ ou un groupe (CH₂)₂NH₂ libre ou substitué par un ou deux alkyles en C₁-C₄;
- R₉ représente l'hydrogène, un halogène, un groupe (CH₂)ᵣ OR₁₀, un groupe (CH₂)ᵣ NR₁₁ R₁₂, un groupe (CH₂)ₛ CONR₁₁ R′₁₁, un groupe azido;
- R₁₀ représente l'hydrogène, un alkyle en C₁-C₄, un mésyle ou un tosyle ;
- R₁₁, R′₁₁ et R₁₂ représentent chacun un hydrogène ou un alkyle en C₁-C₄ ou R₁₁ représente l'hydrogène et R₁₂ représente un benzyloxycarbonyle ou un alcoxycarbonyle en C₁-C₄ ;
- n représente 0, 1 ou 2 ;
- m représente 0, 1 ou 2 ;
- q représente 0, 1, 2 ou 3 ;
- r représente 0, 1, 2 ou 3, avec la limitation que r n'est pas nul lorsque R₈ ou R₉ est en position alpha de l'azote amidique intracyclique ;
- s représente 0 ou 1;
ainsi que ses sels éventuels.

2. Composé selon la revendication 1 dans lequel R₁ représente un atome de chlore, de brome ou un groupe méthoxy et n = 1.

3. Composé selon l'une quelconque des revendication1 ou 2 dans lequel R₂ représente un chlorophényle ou un méthoxyphényle ou un cyclohexyle.

4. Composé selon l'une quelconque des revendications 1 à 3 dans lequel R₅ représente un phényle substitué en position 3 et 4, ou en position 2 et 4 par un groupe méthoxy, ou bien R₅ représente un phényle substitué en position 4 par un méthyle.

5. Composé selon l'une quelconque des revendications 1 à 4 dans lequel m = 0.

6. Composé selon l'une quelconque des revendications 1 à 5 dans lequel R₄ représente CONR₆R₇ et NR₆R₇ représente un groupe pyrrolidino substitué en position 2 par un groupe (CH₂)q lui-même substitué par un groupe carboxyle, ou un groupe carbamoyle avec q = 0,1,2, ou 3.

7. Composé selon l'une des revendications 1 à 5 dans lequel R₄ représente CONR₆R₇ et NR₆R₇ représente un groupe pipéridino substitué en position 4 par un groupe amino, un alkylamino en C₁-C₄ ou un dialkylamino en C₁-C₄.

8. Composé selon l'une quelconque des revendications 1 à 5 dans lequel R₄ représente CONR₆R₇ et NR₆R₇ représente un groupe thiazolidino substitué par un groupe (CH₂)_{q} lui-même substitué par un groupe carboxyle ou un groupe carbamoyle avec q = 0, 1, 2 ou 3.

9. Composé selon l'une quelconque des revendications 1 à 5 dans lequel R₄ représente CONR₆R₇ et NR₆R₇ représente un groupe pyrrolidino substitué en position 2 par un groupe (CH₂)q lui-même substitué par un groupe carboxyle ou un groupe carbamoyle et substitué en position 4 par un groupe amino, un alkylamino en C₁-C₄ ou un dialkylamino en C₁-C₄.

10. Composé selon l'une quelconque des revendication 1 à 5 dans lequel R₄ représente CONR₆R₇, R₆ représentant un alkyle en C₁-C₄ et R₇ représentant un groupe (CH₂)ᵣ lui-même substitué par un groupe carboxyle ou un groupe carbamoyle avec r = 1,2 ou 3.

11. Composé selon l'une quelconque des revendications 1 à 10 sous forme d'isomère cis, dans lequel R₂ et R₄ sont du même côté du cycle indoline.

12. Un procédé pour la préparation d'un composé (I) selon la revendication 1
caractérisé en ce que :
a) on fait réagir sur un dérivé d'amino-2 phénone de formule : 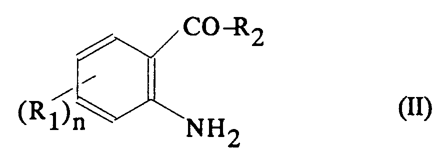 dans laquelle R₁, R₂ et n ont les significations indiquées ci-dessus pour (I) dans la revendication 1, un dérivé sulfonyle de formule :
Hal - SO₂ - (CH₂)ₘ - R₅ (III)
dans laquelle
- Hal représente un halogène, de préférence le chlore ou le brome,
- m et R₅ ont les significations indiquées ci-dessus pour (I) dans la revendication 1,
b) le composé ainsi obtenu de formule : 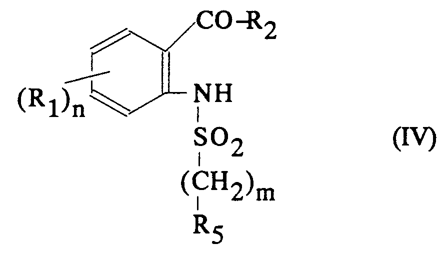 est traité par un dérivé halogéné de formule :
Hal′ - CH₂ COA (V)
dans laquelle
Hal′ représente un halogène préférentiellement le brome, et A représente soit le groupe NR₆R₇, soit le groupe OR dans lequel R est un tertiobutyle ou un benzyle ;
c) le cas échéant, lorsque A représente un groupe OR, l'ester ainsi obtenu de formule : 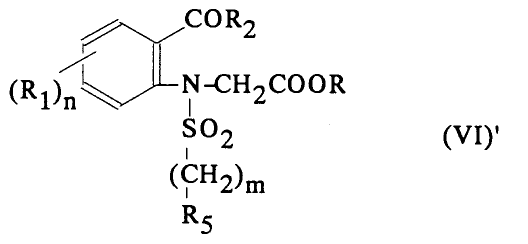 est déprotégé dans les conditions convenables ;
d) le cas échéant, l'acide ainsi obtenu à l'étape c) de formule : 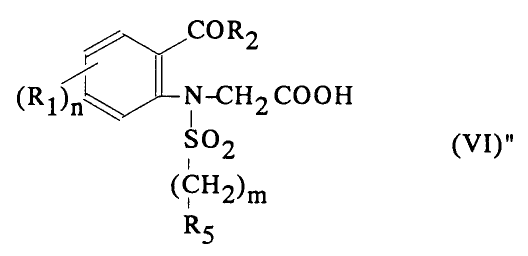 ou son chlorure d'acide de formule : 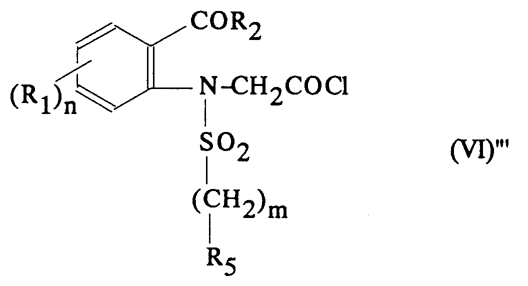 est traité par un composé HNR₆R₇ selon les techniques convenables du couplage amidique ;
e) le composé ainsi obtenu à l'étape b) ou à l'étape d), de formule : 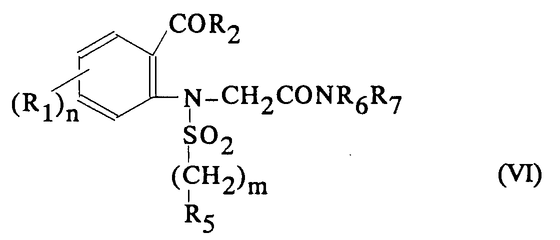 est cyclisé en milieu basique pour préparer le composé (I) selon la revendication 1 ;
f) on sépare éventuellement les isomères cis et trans du composé (I) selon la revendication 1, et, éventuellement on sépare les énantiomères.

13. Un composé de formule: 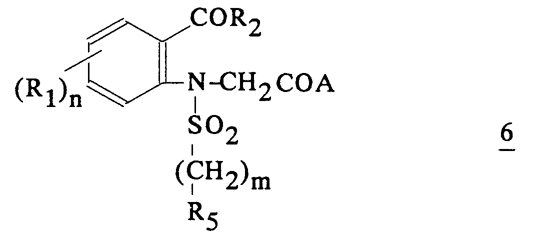 dans laquelle
A′ représente un groupe choisi parmi : NR₆R₇, OH, OtBu, OBz ;
- R₁ représente un atome d'halogène, un alkyle en C₁-C₄, un hydroxyle, un alcoxy en C₁-C₄, un groupe benzyloxy, un groupe cyano, un groupe trifluorométhyle, un groupe nitro ou un groupe amino;
- R₂ représente un alkyle en C₁-C₆; un cycloalkyle en C₃-C₇, un cycloalcène en C₅-C₇, un phényle non substitué ou substitué une ou plusieurs fois par un alkyle en C₁-C₄, un alcoxy en C₁-C₄, un halogène, un groupe trifluorométhyle, un groupe amino, ou R₂ représente un nitrophényle non substitué ou substitué une fois par un groupe trifluorométhyle ou une ou plusieurs fois par un alkyle en C₁-C₄, un alcoxy en C₁-C₄ ou un halogène ;
- R₅ représente un alkyle en C₁-C₄ ; un naphtyl-1 ; un naphtyl-2 ; un diméthylamino-5 naphtyl-1 ; un phényle non substitué ou substitué par un ou plusieurs substituants choisis parmi un halogène, un alkyle en C₁-C₄, un groupe trifluorométhyle, un groupe amino libre ou substitué par un ou 2 alkyles en C₁-C₄, un hydroxyle, un alcoxy en C₁-C₄, un alcénoxy en C₂-C₄, un alkylthio en C₁-C₄, un groupe trifluorométhoxy, un groupe benzyloxy, un groupe cyano, un groupe carboxy, un groupe alcoxycarbonyle en C₁-C₄, un groupe carbamoyle libre ou substitué par un ou deux alkyles en C₁-C₄ ou un groupe alkylamido en C₁-C₄, ou R₅ représente un nitrophényle non substitué ou substitué une fois par un groupe trifluorométhyle ou un alcénoxy en C₂-C₄, ou une ou plusieurs fois par un halogène, un alkyle en C₁-C₄, un alcoxy en C₁-C₄, un alkylthio en C₁-C₄, un groupe trifluorométhoxy ou un groupe benzyloxy ;
- R₆ représente un alkyle en C₁-C₆, ou R₆ est semblable à R₇ ;
- R₇ représente un groupe pipéridin-4-yl ; un groupe azétidin-3-yl, lesdits groupes étant substitués ou non sur l'azote par un alkyle en C₁-C₄ par un benzyloxycarbonyle ou par un alcoxycarbonyle en C₁-C₄ ; un groupe (CH₂)ᵣ lui-même substitué par un groupe pyridyl -2, -3 ou -4, par un groupe hydroxyle, par un groupe amino libre ou substitué par un ou deux alkyles en C₁-C₄, un groupe carboxyle, un groupe alcoxycarbonyle en C₁-C₄, un groupe benzyloxycarbonyle, un groupe carbamoyle libre ou substitué par un ou 2 alkyles en C₁-C₄ ;
- ou R₆ et R₇ ensemble constituent avec l'atome d'azote auquel ils sont liés un hétérocycle choisi parmi :
. la morpholine,
. la thiomorpholine,
. la thiazolidine ou la 2,2-diméthylthiazolidine substitué ou non par R₈,
. la pipérazine non substituée ou substituée en 4 par un groupe R''₈ ;
. un cycle insaturé monoazoté à 5 chaînons substitué par R₈ ou
. un cycle saturé monoazoté à 3, 4, 5, 6 ou 7 chaînons substitué par R₈ et R₉ ;
- R₈ représente R′₈ ou un groupe (CH₂)ᵣ lui-même substitué par un hydroxyle ou par un amino libre ou substitué par un ou deux alkyles en C₁-C₄ ;
- R′₈ représente un groupe (CH₂)_{q} lui-même substitué par un groupe carboxyle, un groupe alcoxycarbonyle en C₁-C₄, un groupe benzyloxycarbonyle, un groupe carbamoyle libre ou substitué par un hydroxyle ou par un ou 2 alkyles en C₁-C₄, ou un groupe aminocarbothioyle libre ou substitué par un ou 2 alkyles en C₁-C₄ ;
- R''₈ représente R′₈ ou un groupe (CH₂)₂NH₂ libre ou substitué par un ou deux alkyles en C₁-C₄;
- R₉ représente l'hydrogène, un halogène, un groupe (CH₂)ᵣ OR₁₀, un groupe (CH₂)ᵣ NR₁₁ R₁₂, un groupe (CH₂)ₛ CONR₁₁ R′₁₁, un groupe azido;
- R₁₀ représente l'hydrogène, un alkyle en C₁-C₄, un mésyle ou un tosyle;
- R₁₁, R'₁₁ et R₁₂ représentent chacun un hydrogène ou un alkyle en C₁-C₄ ou R₁₁ représente l'hydrogène et R₁₂ représente un benzyloxycarbonyle ou un alcoxycarbonyle en C₁-C₄;
- n représente 0, 1 ou 2 ;
- m représente 0, 1 ou 2 ;
- q représente 0, 1, 2 ou 3 ;
- r représente 0, 1, 2 ou 3, avec la limitation que r n'est pas nul lorsque R₈ ou R₉ est en position alpha de l'azote amidique intracyclique ;
- s représente 0 ou 1.

14. Utilisation d'un composé de formule : 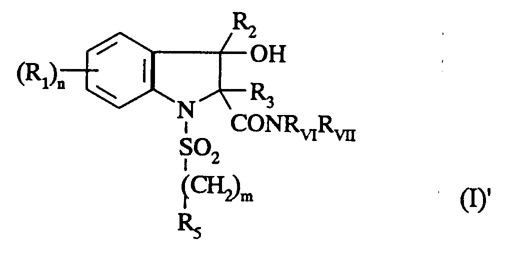 dans laquelle R₁, R₂, R₃, R₅, m et n ont les significations données pour les composés de formule (I) dans la revendication 1 et
- R_{VI} représente un alkyle en C₁-C₆,
- R_{VII} représente un groupe (CH₂)ᵣCOOH avec r = 1, 2 ou 3.
- ou R_{VI} et R_{VII} ensemble avec l'atome d'azote auquel ils sont liés constituent un hétérocycle choisi parmi :
. la thiazolidine ou la 2,2-diméthylthiazolidine substituées par un groupe (CH₂)_{q}COOH,
. la pipérazine substituée en 4 par un groupe (CH₂)_{q}COOH,
. un cycle insaturé monoazoté à 5 chaînons substitué par un groupe (CH₂)_{q}COOH,
. un cycle saturé monoazoté à 3, 4, 5, 6 ou 7 chaînons substitué par un groupe (CH₂)_{q}COOH,
avec q = 0, 1, 2 ou 3,
pour la préparation d'un composé de formule (I)'' ayant la même configuration autour de la liaison 2, 3 de l'indoline que le produit de départ 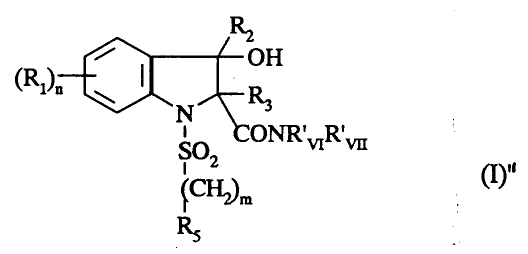 dans laquelle :
- R₁,R₂, R₃, R₅,m et n sont tels que définis ci-dessus,
- R'_{VI} représente un alkyle en C₁-C₆,
- R'_{VII} représente le groupe (CH₂)ᵣH,
ou R'_{VI} et R'_{VII} ensemble avec l'atome d'azote auquel ils sont liés constituent un hétérocycle choisi parmi :
. la thiazolidine ou la 2,2-diméthylthiazolidine substituée par un groupe (CH₂)_{q}H,
. la pipérazine substituée en 4 par un groupe (CH₂)_{q} H,
. un cycle insaturé monoazoté à 5 chaînons substitué par un groupe (CH₂)_{q}H,
. un cycle saturé monoazoté à 3, 4, 5, 6 ou 7 chaînons substitué par un groupe (CH₂)_{q}H.

15. Procédé pour la préparation d'une composition pharmaceutique, caractérisé en ce que l'on mélange, en tant que principe actif, un composé de formule (I) selon l'une quelconque des revendications 1 à 11 avec un véhicule pharmaceutiquement acceptable.

16. Procédé pour la préparation d'une composition pharmaceutique, caractérisé en ce que l'on mélange, en tant que principe actif, un composé de formule (I) selon l'une quelconque des revendications 1 à 11 en association avec un autre principe actif, avec un véhicule pharmaceutiquement acceptable.

## Claims (Claims for the following Contracting State(s): AT, BE, CH, DE, DK, FR, GB, IE, IT, LI, LU, NL, PT, SE)

1. A compound of formula : 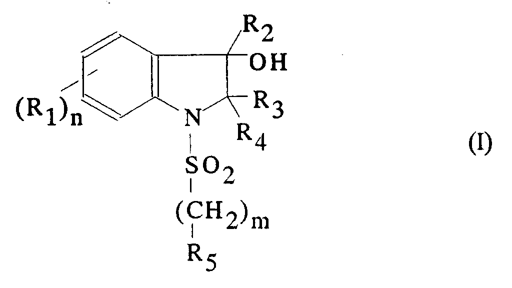 in which
- R₁ is a halogen atom, a C₁-C₄ alkyl, a hydroxyl, a C₁-C₄ alkoxy, a benzyloxy group, a cyano group, a trifluoromethyl group, a nitro group or an amino group ;
- R₂ is a C₁-C₆ alkyl, a C₃-C₇ cycloalkyl, a C₅-C₇ cycloalkene or a phenyl which is unsubstituted, monosubstituted or polysubstituted by a C₁-C₄ alkyl, a C₁-C₄ alkoxy, a halogen, a trifluoromethyl group or an amino group, or R₂ is a nitrophenyl which is unsubstituted or monosubstituted by a trifluoromethyl group or monosubstituted or polysubstituted by a C₁-C₄ alkyl or a halogen ;
- R₃ is a hydrogen atom;
- R₄ is a carbamoyl group of formula CONR₆R₇ ;
- R₅ is a C₁-C₄ alkyl, a 1-naphthyl, a 2-naphthyl, a 5-dimethylamino-1-naphthyl, a phenyl which is unsubstituted or substituted by one or more substituents selected from a halogen, a C₁-C₄ alkyl, a trifluoromethyl group, an amino group which is free or substituted by one or two C₁-C₄ alkyls, a hydroxyl, a C₁-C₄ alkoxy, a C₂-C₄ alkenoxy, a C₁-C₄ alkylthio, a trifluoromethoxy group, a benzyloxy group, a cyano group, a carboxyl group, a C₁-C₄ alkoxycarbonyl group, a carbamoyl group which is free or substituted by one or two C₁-C₄ alkyls or a C₁-C₄ alkylamido group, or R₅ is a nitrophenyl which is unsubstituted or monosubstituted by a trifluoromethyl group or a C₂-C₄ alkenoxy or mono- or polysubstituted by a halogen, a C₁-C₄ alkyl, a C₁-C₄ alkoxy, a C₁-C₄ alkythio, a trifluoromethoxy group or a benzyloxy group ;
- R₆ is a C₁-C₆ alkyl or R₆ is similar to R₇ ;
- R₇ is a 4-piperidinyl group or a 3-azetidinyl group, the said groups being substituted or unsubstituted on the nitrogen by a C₁-C₄ alkyl, by a benzyloxycarbonyl or by a C₁-C₄ alkoxycarbonyl ; a (CH₂)ᵣ group which is itself substituted by a 2-, 3- or 4-pyridyl group, by a hydroxyl group or by an amino group which is free or substituted by one or two C₁-C₄ alkyls, a carboxyl group, a C₁-C₄ alkoxycarbonyl group, a benzyloxycarbonyl group or a carbamoyl group which is free or substituted by one or two C₁-C₄ alkyls.
- or R₆ and R₇ together with the nitrogen atom to which they are bonded, form a heterocycle selected from :
. morpholine,
. thiomorpholine,
. thiazolidine or 2,2-dimethylthiazolidine, unsubstituted or substituted by R₈,
. piperazine, unsubstituted or substituted at the 4-position by a R''₈ group,
. an unsaturated, 5-membered ring containing a single nitrogen atom and substituted by R₈ or a saturated, 3-, 4-, 5-, 6- or 7-membered ring containing a single nitrogen atom and substituted by R₈ and R₉ ;
- R₈ is R'₈ or a (CH₂₎ᵣ group which is itself substituted by a hydroxyl or by an amino which is free or substituted by one or two C₁-C₄ alkyls ;
- R'₈ is a (CH₂)_{q} group which is itself substituted by a carboxyl group, a C₁-C₄ alkoxycarbonyl group, a benzyloxycarbonyl group, a carbamoyl group which is free or substituted by a hydroxyl or by one or two C₁-C₄ alkyls or an aminocarbothioyl group which is free or substituted by one or two C₁-C₄ alkyls ;
- R''₈ is R'₈ or a (CH₂)₂NH₂ group which is free or substituted by one or two C₁-C₄ alkyls ;
- R₉ is hydrogen, a halogen, a (CH₂)ᵣOR₁₀ group, a (CH₂)ᵣNR₁₁R₁₂ group, a (CH₂)ₛCONR₁₁R'₁₁ group or an azido group ;
- R₁₀ is hydrogen, a C₁-C₄ alkyl, a mesyl or a tosyl ;
- R_{11,} R'₁₁ and R₁₂ are each a hydrogen or a C₁-C₄ alkyl or R₁₁ is hydrogen and R₁₂ is a benzyloxycarbonyl or a C₁-C₄ alkoxycarbonyl ;
- n is 0, 1 or 2 ;
- m is 0, 1 or 2 ;
- q is 0, 1, 2 or 3 ;
- r is 0, 1, 2 or 3, with the limitation that r is not zero when R₈ or R₉ is at the alpha-position of the intracyclic amide nitrogen ;
- s is 0 or 1 ;
as well as its possible salts.

2. A compound according to claim 1 in which R₁ is a chlorine or bromine atom or a methoxy group and n = 1.

3. A compound according to any one of claims 1 or 2 in which R₂ is a chlorophenyl, a methoxyphenyl or a cyclohexyl.

4. A compound according to any one of claims 1 to 3 in which R₅ is a phenyl substituted at the 3- and 4-positions or at the 2- and 4-positions by a methoxy group, or else R₅ is a phenyl substituted at the 4-position by a methyl.

5. A compound according to any one of claims 1 to 4 in which m = 0.

6. A compound according to any one of claims 1 to 5 in which R₄ is CONR₆R₇ and NR₆R₇ is a pyrrolidino group substituted at the 2-position by a (CH₂)_{q} group which is itself substituted by a carboxyl group, or a carbamoyl group with q = 0, 1, 2 or 3.

7. A compound according to one of claims 1 to 5 in which R₄ is CONR₆R₇ and NR₆R₇ is a piperidino group substituted at the 4-position by an amino group, a C₁-C₄ alkylamino or a C₁-C₄ dialkylamino.

8. A compound according to any one of claims 1 to 5 in which R₄ is CONR₆R₇ and NR₆R₇ is a thiazolidino group substituted by a (CH₂)_{q} group which is itself substituted by a carboxyl group or a carbamoyl group with q = 0, 1, 2, or 3.

9. A compound according to any one of claims 1 to 5 in which R₄ is CONR₆R₇ and NR₆R₇ is a pyrrolidino group substituted at the 2-position by a (CH₂)_{q} group which is itself substituted by a carboxyl group or a carbamoyl group and substituted at the 4-position by an amino group, a C₁-C₄ alkylamino or a C₁-C₄ dialkylamino.

10. A compound according to any one of claims 1 to 5 in which R₄ is CONR₆R₇, R₆ being a C₁-C₄ alkyl and R₇ being a (CH₂)ᵣ group which is itself substituted by a carboxyl group or a carbamoyl group with r = 1, 2 or 3.

11. A compound according to any one of claims 1 to 10 in the form of a cis isomer in which R₂ and R₄ are on the same side of the indoline ring.

12. A process for preparing a compound (I) according to claim 1, characterised in that
a) a 2-aminophenone derivative of formula : 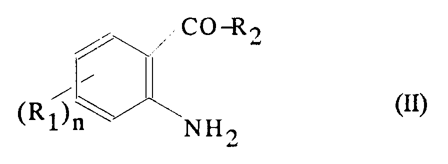 in which R₁, R₂ and n have the meanings indicated above for I in claim 1, is reacted with a sulfonyl derivative of formula :
Hal-SO₂-(CH₂)ₘ-R₅ (III)
in which
- Hal is a halogen, preferably chlorine or bromine,
- m and R₅ have the meanings indicated above for (I) in claim 1 ;
b) the thus obtained compound of formula : 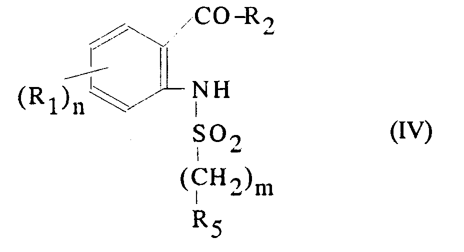 is treated with a halogenated derivative of formula :
Hal'-CH₂COA (V)
in which
Hal' is a halogen, preferably bromine, and A represents either the NR₆R₇ group or the OR group in which R is a tert-butyl or a benzyl ;
c) the thus obtained ester of formula : 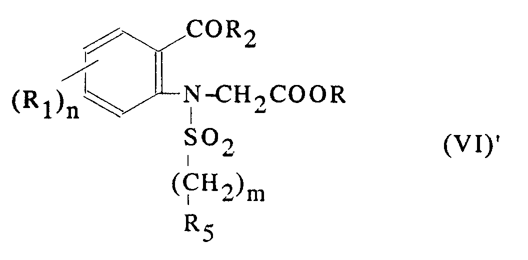 is deprotected under suitable conditions, if applicable, when A is an OR group ;
d) if applicable, the resulting acid from Step c) of formula : 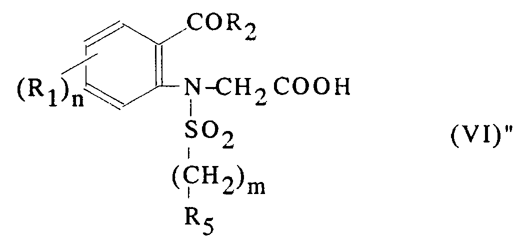 or its acid chloride of formula : 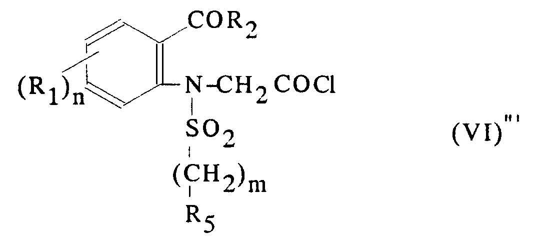 is treated with an HNR₆R₇ compound according to suitable amide coupling techniques ;
e) the thus obtained compound from Step b) or from Step d) of formula : 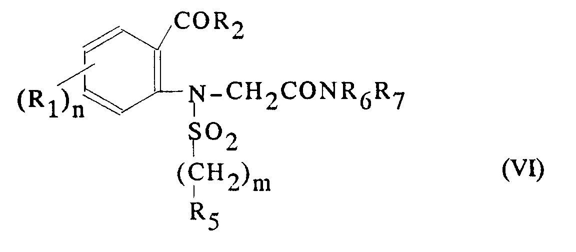 is cyclised in a basic medium in order to prepare compound (I) according to claim 1 ;
f) if appropriate, the cis and trans isomers of compound (I) according to claim 1 are separated and, if appropriate, the enantiomers are separated.

13. A compound of formula : 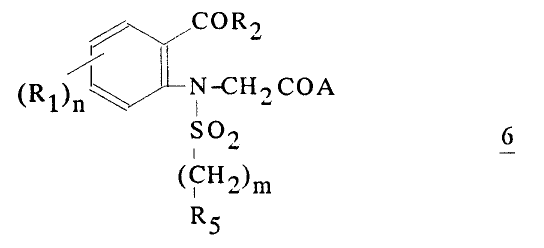 in which
A' is a group selected from ; NR₆R₇ , OH, OtBu or OBz;
- R₁ is a halogen atom, a C₁-C₄ alkyl, a hydroxyl, a C₁-C₄ alkoxy, a benzyloxy group, a cyano group, a trifluoromethyl group, a nitro group or an amino group ;
- R₂ is a C₁-C₆ alkyl, a C₃-C₇ cycloalkyl, a C₅-C₇ cycloalkene or a phenyl which is unsubstituted, monosubstituted or polysubstituted by a C₁-C₄ alkyl, a C₁-C₄ alkoxy, a halogen, a trifluoromethyl group or an amino group, or R₂ is a nitrophenyl which is unsubstituted or monosubstituted by a trifluoromethyl group or monosubstituted or polysubstituted by a C₁-C₄ alkyl, a C₁-C₄ alkoxy or a halogen ;
- R₅ is a C₁-C₄ alkyl, a 1-naphthyl, a 2-naphthyl, a 5-dimethylamino-1-naphthyl, a phenyl which is unsubstituted or substituted by one or more substituents selected from a halogen, a C₁-C₄ alkyl, a trifluoromethyl group, an amino group which is free or substituted by one or two C₁-C₄ alkyls, a hydroxyl, a C₁-C₄ alkoxy, a C₂-C₄ alkenoxy, a C₁-C₄ alkylthio, a trifluoromethoxy group, a benzyloxy group, a cyano group, a carboxyl group, a C₁-C₄ alkoxycarbonyl group, a carbamoyl group which is free or substituted by one or two C₁-C₄ alkyls or a C₁-C₄ alkylamido group, or R₅ is a nitrophenyl which is unsubstituted or monosubstituted by a trifluoromethyl group or a C₂-C₄ alkenoxy or mono- or polysubstituted by a halogen, a C₁-C₄ alkyl, a C₁-C₄ alkoxy, a C₁-C₄ alkylthio, a trifluoromethoxy group or a benzyloxy group;
- R₆ is a C₁-C₆ alkyl or R₆ is similar to R₇ ;
- R₇ is a 4-piperidinyl group or a 3-azetidinyl group, the said groups being substituted or unsubstituted on the nitrogen by a C₁-C₄ alkyl, by a benzyloxycarbonyl or by a C₁-C₄ alkoxycarbonyl ; a (CH₂)ᵣ group which is itself substituted by a 2-, 3- or 4-pyridyl group, by a hydroxyl group or by an amino group which is free or substituted by one or two C₁-C₄ alkyls, a carboxyl group, a C₁-C₄ alkoxycarbonyl group, a benzyloxycarbonyl group or a carbamoyl group which is free or substituted by one or two C₁-C₄ alkyls ;
- or R₆ and R₇ together with the nitrogen atom to which they are bonded, form a heterocycle selected from :
. morpholine,
. thiomorpholine,
. thiazolidine or 2,2-dimethylthiazolidine, unsubstituted or substituted by R₈,
. piperazine, unsubstituted or substituted at the 4-position by a R''₈ group,
. an unsaturated, 5-membered ring containing a single nitrogen atom and substituted by R₈ or
. a saturated, 3-, 4-, 5-, 6- or 7-membered ring containing a single nitrogen atom and substituted by R₈ and R₉ ;
- R₈ is R'₈ or a (CH₂)ᵣ group which is itself substituted by a hydroxyl or by an amino which is free or substituted by one or two C₁-C₄ alkyls ;
- R'₈ is a (CH₂)_{q} group which is itself substituted by a carboxyl group, a C₁-C₄ alkoxycarbonyl group, a benzyloxycarbonyl group, a carbamoyl group which is free or substituted by a hydroxyl or by one or two C₁-C₄ alkyls or an aminocarbothioyl group which is free or substituted by one or two C₁-C₄ alkyls ;
- R''₈ is R'₈ or a (CH₂)₂NH₂ group which is free or substituted by one or two C₁-C₄ alkyls ;
- R₉ is hydrogen, a halogen, a (CH₂)ᵣOR₁₀ group, a (CH₂)ᵣNR₁₁R₁₂ group, a (CH₂)ₛCONR₁₁R'₁₁ group or an azido group ;
- R₁₀ is hydrogen, a C₁-C₄ alkyl, a mesyl or a tosyl ;
- R₁₁, R'₁₁ and R₁₂ are each a hydrogen or a C₁-C₄ alkyl or R₁₁ is hydrogen and R₁₂ is a benzyloxycarbonyl or a C₁-C₄ alkoxycarbonyl ;
- n is 0, 1 or 2 ;
- m is 0, 1 or 2 ;
- q is 0, 1, 2 or 3 ;
- r is 0, 1, 2 or 3, with the limitation that r is not zero when R₈ or R₉ is at the alpha-position of the intracyclic amide nitrogen ;
- s is 0 or 1.

14. The use of a compound of formula : 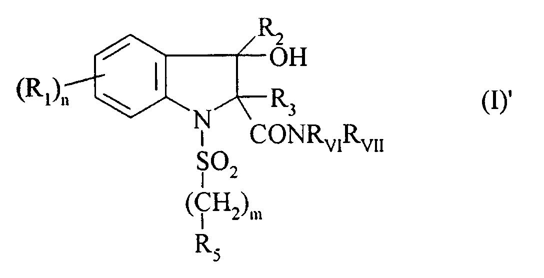 in which R₁, R₂, R₃, R₅, m and n have the meanings given for the compounds of formula (I) in claim 1, and
- R_{VI} is a C₁-C₆ alkyl,
- R_{VII} is a (CH₂)ᵣCOOH group with r = 1, 2 or 3,
- or R_{VI} and R_{VII} together with the nitrogen atom to which they are bonded, form a heterocycle selected from :
. thiazolidine or 2,2-dimethylthiazolidine substituted by a (CH₂)_{q}COOH group,
. piperazine substituted at the 4-position by a (CH₂)_{q}COOH group,
. an unsaturated, 5-membered ring containing a single nitrogen atom and substituted by a (CH₂)_{q}COOH group or
. a saturated, 3-, 4-, 5-, 6- or 7-membered ring containing a single nitrogen atom and substituted by a (CH₂)_{q}COOH group,
with q = 0, 1, 2 or 3
for the preparation of a compound of formula (I)'' having the same configuration around the 2,3 bond of the indoline as the starting product 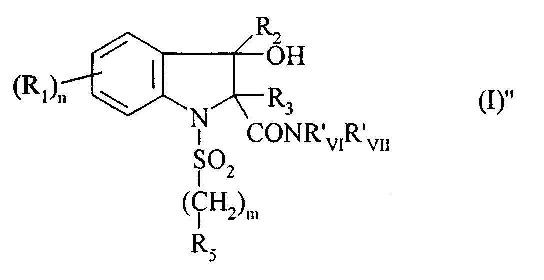 in which
- R₁, R₂, R₃, R₅, m and n are as defined above,
- R'_{VI} is a C₁-C₆ alkyl,
- R'_{VII} is a (CH₂)ᵣH group,
- or R'_{VI} and R'_{VII} together with the nitrogen atom to which they are bonded, form a heterocycle selected from :
. thiazolidine or 2,2-dimethylthiazolidine substituted by a (CH₂)_{q}H group,
. piperazine substituted at the 4-position by a (CH₂)_{q}H group,
. an unsaturated, 5-membered ring containing a single nitrogen atom and substituted by a (CH₂)_{q}H group or
. a saturated 3-, 4-, 5-, 6- or 7-membered ring containing a single nitrogen atom and substituted by a (CH₂)_{q}H group.

15. A pharmaceutical composition in which a compound according to one of claims 1 to 11 is present as the active principle.

16. A pharmaceutical composition in which a compound according to any one of claims 1 to 11 is present in association with another active principle.

## Claims (Claims for the following Contracting State(s): ES)

1. A process for preparing a compound of formula (I): 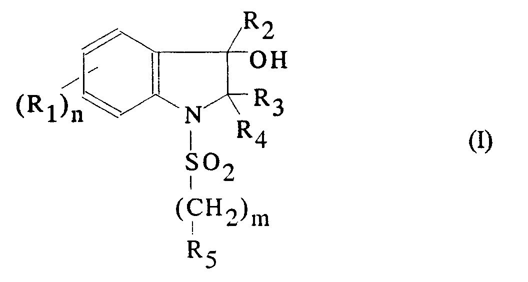 in which
- R₁ is a halogen atom, a C₁-C₄ alkyl, a hydroxyl, a C₁-C₄ alkoxy, a benzyloxy group, a cyano group, a trifluoromethyl group, a nitro group or an amino group ;
- R₂ is a C₁-C₆ alkyl, a C₃-C₇ cycloalkyl, a C₅-C₇ cycloalkene or a phenyl which is unsubstituted, monosubstituted or polysubstituted by a C₁-C₄ alkyl, a C₁-C₄ alkoxy, a halogen, a trifluoromethyl group or an amino group, or R₂ is a nitrophenyl which is unsubstituted or monosubstituted by a trifluoromethyl group or monosubstituted or polysubstituted by a C₁-C₄ alkyl or a halogen ;
- R₃ is a hydrogen atom;
- R₄ is a carbamoyl group of formula CONR₆R₇ ;
- R₅ is a C₁-C₄ alkyl, a 1-naphthyl, a 2-naphthyl, a 5-dimethylamino-1-naphthyl, a phenyl which is unsubstituted or substituted by one or more substituents selected from a halogen, a C₁-C₄ alkyl, a trifluoromethyl group, an amino group which is free or substituted by one or two C₁-C₄ alkyls, a hydroxyl, a C₁-C₄ alkoxy, a C₂-C₄ alkenoxy, a C₁-C₄ alkylthio, a trifluoromethoxy group, a benzyloxy group, a cyano group, a carboxyl group, a C₁-C₄ alkoxycarbonyl group, a carbamoyl group which is free or substituted by one or two C₁-C₄ alkyls or a C₁-C₄ alkylamido group, or R₅ is a nitrophenyl which is unsubstituted or monosubstituted by a trifluoromethyl group or a C₂-C₄ alkenoxy or mono- or polysubstituted by a halogen, a C₁-C₄ alkyl, a C₁-C₄ alkoxy, a C₁-C₄ alkythio, a trifluoromethoxy group or a benzyloxy group ;
- R₆ is a C₁-C₆ alkyl or R₆ is similar to R₇ ;
- R₇ is a 4-piperidinyl group or a 3-azetidinyl group, the said groups being substituted or unsubstituted on the nitrogen by a C₁-C₄ alkyl, by a benzyloxycarbonyl or by a C₁-C₄ alkoxycarbonyl ; a (CH₂)ᵣ group which is itself substituted by a 2-, 3- or 4-pyridyl group, by a hydroxyl group or by an amino group which is free or substituted by one or two C₁-C₄ alkyls, a carboxyl group, a C₁-C₄ alkoxycarbonyl group, a benzyloxycarbonyl group or a carbamoyl group which is free or substituted by one or two C₁-C₄ alkyls.
- or R₆ and R₇ together with the nitrogen atom to which they are bonded, form a heterocycle selected from :
. morpholine,
. thiomorpholine,
. thiazolidine or 2,2-dimethylthiazolidine, unsubstituted or substituted by R₈,
. piperazine, unsubstituted or substituted at the 4-position by an R''₈ group,
. an unsaturated, 5-membered ring containing a single nitrogen atom and substituted by R₈ or a saturated, 3-, 4-, 5-, 6- or 7-membered ring containing a single nitrogen atom and substituted by R₈ and R₉ ;
- R₈ is R'₈ or a (CH₂)ᵣ group which is itself substituted by a hydroxyl or by an amino which is free or substituted by one or two C₁-C₄ alkyls ;
- R'₈ is a (CH₂)_{q} group which is itself substituted by a carboxyl group, a C₁-C₄ alkoxycarbonyl group, a benzyloxycarbonyl group, a carbamoyl group which is free or substituted by a hydroxyl or by one or two C₁-C₄ alkyls or an aminocarbothioyl group which is free or substituted by one or two C₁-C₄ alkyls ;
- R''₈ is R'₈ or a (CH₂)₂NH₂ group which is free or substituted by one or two C₁-C₄ alkyls ;
- R₉ is hydrogen, a halogen, a (CH₂)ᵣOR₁₀ group, a (CH₂)ᵣNR₁₁R₁₂ group, a (CH₂)ₛCONR₁₁R'₁₁ group or an azido group ;
- R₁₀ is hydrogen, a C₁-C₄ alkyl, a mesyl or a tosyl ;
- R_{11,} R'₁₁ and R₁₂ are each a hydrogen or a C₁-C₄ alkyl or R₁₁ is hydrogen and R₁₂ is a benzyloxycarbonyl or a C₁-C₄ alkoxycarbonyl ;
- n is 0, 1 or 2 ;
- m is 0, 1 or 2 ;
- q is 0, 1, 2 or 3 ;
- r is 0, 1, 2 or 3, with the limitation that r is not zero when R₈ or R₉ is at the alpha-position of the intracyclic amide nitrogen ;
- s is 0 or 1 ;
as well as its possible salts,
characterised in that :
a) a 2-aminophenone derivative of formula : 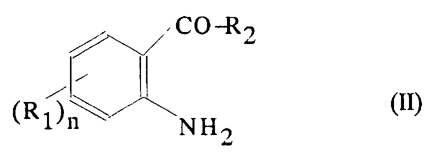 in which R₁, R₂ and n have the meanings indicated above, is reacted with a sulfonyl derivative of formula :
Hal-SO₂-(CH₂)ₘ-R₅ (III)
in which
- Hal is a halogen, preferably chlorine or bromine,
- m and R₅ have the meanings indicated above ;
b) the thus obtained compound of formula : 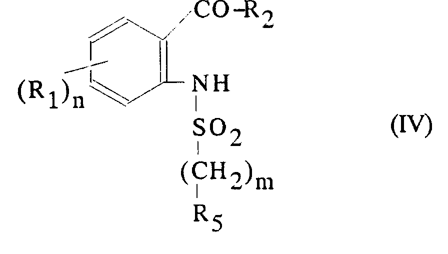 is treated with a halogenated derivative of formula :
Hal'-CH₂COA (V)
in which
Hal' is a halogen, preferably bromine, and A represents either the NR₆R₇ group or the OR group in which R is a tert-butyl or a benzyl ;
c) the thus obtained ester of formula : 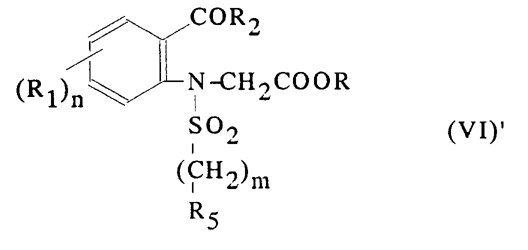 is deprotected under suitable conditions, if applicable, when A is an OR group ;
d) if applicable, the thus obtained acid from Step c) of formula : 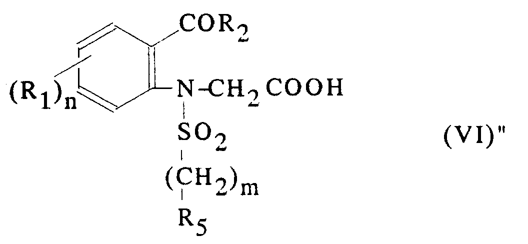 or its acid chloride of formula : 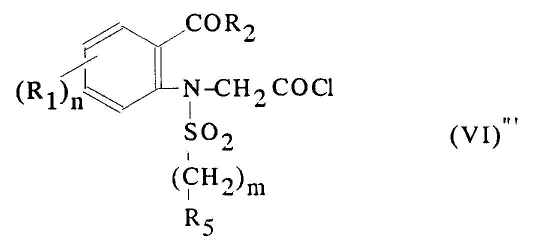 is treated with a HNR₆R₇ compound according to suitable amide coupling techniques ;
e) the thus obtained compound from Step b) or from Step d) of formula : 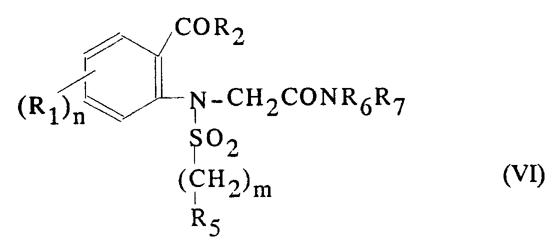 is cyclised in a basic medium in order to prepare compound (I);
f) if appropriate, the cis and trans isomers of compound (I) are separated and, if appropriate, the enantiomers are separated, the compounds of formula (I) being optionally converted into their salts with mineral or organic acids or with mineral or organic bases.

2. A process according to claim 1 for obtaining a compound of formula (I) in which R₁ is a chlorine or bromine atom or a methoxy group and n = 1, characterised in that a compound of formula (II) in which R₁ and n are as defined above is used in step a).

3. A process according to one of claims 1 or 2, for preparing a compound of formula (I), in which R₂ is a chlorophenyl, a methoxyphenyl or a cyclohexyl, characterised in that a compound of formula (II) in which R₂ is as defined above is used in step a).

4. A process according to any one of claims 1 to 3, for preparing a compound of formula (I), in which R₅ is a phenyl substituted at the 3- and 4-positions or at the 2- and 4-positions by a methoxy group, or else R₅ is a phenyl substituted at the 4-position by a methyl, characterised in that a compound of formula (III) in which R₅ is as defined above is used in step a).

5. A process according to any one of claims 1 to 4, for preparing a compound of formula (I), in which m = 0, characterised in that a compound of formula (III) in which m = 0 is used in step a).

6. A process according to any one of claims 1 to 5, for preparing a compound of formula (I), in which R₄ is CONR₆R₇ and NR₆R₇ is a pyrrolidino group substituted at the 2-position by a (CH₂)_{q} group which is itself substituted by a carboxyl group, or a carbamoyl group with q = 0, 1, 2 or 3, characterised in that a compound of formula (V) in which A is a NR₆R₇ group as defined above is used in step b).

7. A process according to any one of claims 1 to 5, for preparing a compound of formula (I), in which R₄ is CONR₆R₇ and NR₆R₇ is a piperidino group substituted at the 4-position by an amino group, a C₁-C₄ alkylamino or a C₁-C₄ dialkylamino, characterised in that a compound of formula (V) in which A is a NR₆R₇ group as defined above is used in step b).

8. A process according to any one of claims 1 to 5, for preparing a compound of formula (I), in which R₄ is CONR₆R₇ and NR₆R₇ is a thiazolidino group substituted by a (CH₂)_{q} group which is itself substituted by a carboxyl group or a carbamoyl group with q = 0, 1, 2, or 3, characterised in that a compound of formula (V) in which A is a NR₆R₇ group as defined above is used in step b).

9. A process according to any one of claims 1 to 5, for preparing a compound of formula (I), in which R₄ is CONR₆R₇ and NR₆R₇ is a pyrrolidino group substituted at the 2-position by a (CH₂)_{q} group which is itself substituted by a carboxyl group or a carbamoyl group and substituted at the 4-position by an amino group, a C₁-C₄ alkylamino or a C₁-C₄ dialkylamino, characterised in that a compound of formula (V) in which A is a NR₆R₇ group as defined above is used in step b).

10. A process according to any one of claims 1 to 5, for preparing a compound of formula (I), in which R₄ is CONR₆R₇, R₆ being a C₁-C₄ alkyl and R₇ being a (CH₂)ᵣ group which is itself substituted by a carboxyl group or a carbamoyl group with r = 1, 2 or 3, characterised in that a compound of formula (V) in which A is a NR₆R₇ group as defined above is used in step b).

11. A process according to any one of claims 1 to 10, for preparing a compound of formula (I), in the form of a cis isomer in which R₂ and R₄ are on the same side of the indoline ring, characterised in that the cis isomer is separated from the trans isomer by chromatography or fractionated crystallization in step f).

12. A process for preparing a compound of formula 6: 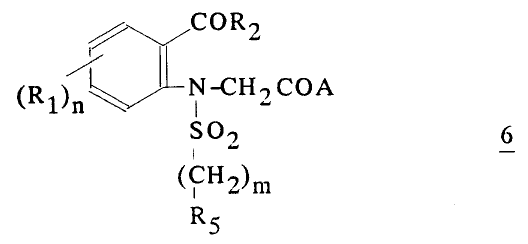 in which
A' is a group selected from : NR₆R₇ , OH, OtBu or OBz;
- R₁ is a halogen atom, a C₁-C₄ alkyl, a hydroxyl, a C₁-C₄ alkoxy, a benzyloxy group, a cyano group, a trifluoromethyl group, a nitro group or an amino group ;
- R₂ is a C₁-C₆ alkyl, a C₃-C₇ cycloalkyl, a C₅-C₇ cycloalkene or a phenyl which is unsubstituted, monosubstituted or polysubstituted by a C₁-C₄ alkyl, a C₁-C₄ alkoxy, a halogen, a trifluoromethyl group or an amino group, or R₂ is a nitrophenyl which is unsubstituted or monosubstituted by a trifluoromethyl group or monosubstituted or polysubstituted by a C₁-C₄ alkyl, a C₁-C₄ alkoxy or a halogen ;
- R₅ is a C₁-C₄ alkyl, a 1-naphthyl, a 2-naphthyl, a 5-dimethylamino-1-naphthyl, a phenyl which is unsubstituted or substituted by one or more substituents selected from a halogen, a C₁-C₄ alkyl, a trifluoromethyl group, an amino group which is free or substituted by one or two C₁-C₄ alkyls, a hydroxyl, a C₁-C₄ alkoxy, a C₂-C₄ alkenoxy, a C₁-C₄ alkylthio, a trifluoromethoxy group, a benzyloxy group, a cyano group, a carboxyl group, a C₁-C₄ alkoxycarbonyl group, a carbamoyl group which is free or substituted by one or two C₁-C₄ alkyls or a C₁-C₄ alkylamido group, or R₅ is a nitrophenyl which is unsubstituted or monosubstituted by a trifluoromethyl group or a C₂-C₄ alkenoxy or mono- or polysubstituted by a halogen, a C₁-C₄ alkyl, a C₁-C₄ alkoxy, a C₁-C₄ alkylthio, a trifluoromethoxy group or a benzyloxy group ;
- R₆ is a C₁-C₆ alkyl or R₆ is similar to R₇ ;
- R₇ is a 4-piperidinyl group or a 3-azetidinyl group, the said groups being substituted or unsubstituted on the nitrogen by a C₁-C₄ alkyl, by a benzyloxycarbonyl or by a C₁-C₄ alkoxycarbonyl ; a (CH₂)ᵣ group which is itself substituted by a 2-, 3- or 4-pyridyl group, by a hydroxyl group or by an amino group which is free or substituted by one or two C₁-C₄ alkyls, a carboxyl group, a C₁-C₄ alkoxycarbonyl group, a benzyloxycarbonyl group or a carbamoyl group which is free or substituted by one or two C₁-C₄ alkyls ;
- or R₆ and R₇ together with the nitrogen atom to which they are bonded, form a heterocycle selected from ;
. morpholine,
. thiomorpholine,
. thiazolidine or 2,2-dimethylthiazolidine, unsubstituted or substituted by R₈,
. piperazine, unsubstituted or substituted at the 4-position by an R''₈ group,
. an unsaturated, 5-membered ring containing a single nitrogen atom and substituted by R₈ or
. a saturated, 3-, 4-, 5-, 6- or 7-membered ring containing a single nitrogen atom and substituted by R₈ and R₉ ;
- R₈ is R'₈ or a (CH₂)ᵣ group which is itself substituted by a hydroxyl or by an amino which is free or substituted by one or two C₁-C₄ alkyls ;
- R'₈ is a (CH₂)_{q} group which is itself substituted by a carboxyl group, a C₁-C₄ alkoxycarbonyl group, a benzyloxycarbonyl group, a carbamoyl group which is free or substituted by a hydroxyl or by one or two C₁-C₄ alkyls or an aminocarbothioyl group which is free or substituted by one or two C₁-C₄ alkyls ;
- R''₈ is R'₈ or a (CH₂)₂NH₂ group which is free or substituted by one or two C₁-C₄ alkyls ;
- R₉ is hydrogen, a halogen, a (CH₂)ᵣOR₁₀ group, a (CH₂)ᵣNR₁₁R₁₂ group, a (CH₂)ₛCONR₁₁R'₁₁ group or an azido group ;
- R₁₀ is hydrogen, a C₁-C₄ alkyl, a mesyl or a tosyl ;
- R₁₁, R'₁₁ and R₁₂ are each a hydrogen or a C₁-C₄ alkyl or R₁₁ is hydrogen and R₁₂ is a benzyloxycarbonyl or a C₁-C₄ alkoxycarbonyl ;
- n is 0, 1 or 2 ;
- m is 0, 1 or 2 ;
- q is 0, 1, 2 or 3 ;
- r is 0, 1, 2 or 3, with the limitation that r is not zero when R₈ or R₉ is at the alpha-position of the intracyclic amide nitrogen ;
- s is 0 or 1,
characterised in that:
a) a 2-aminophenone derivative of formula : 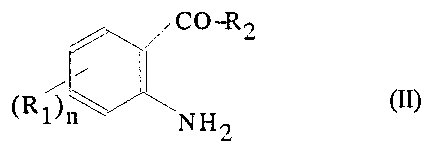 in which R₁, R₂ and n have the meanings indicated above for I, in claim 1, is reacted with a sulfonyl derivative of formula :
Hal-SO₂-(CH₂)ₘ-R₅ (III)
in which
- Hal is a halogen, preferably chlorine or bromine,
- m and R₅ have the meanings indicated above for (I) in claim 1 ;
b) the thus obtained compound of formula : 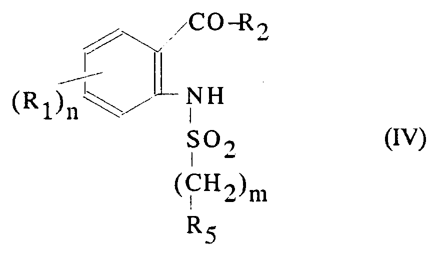 is treated with a halogenated derivative of formula :
Hal'-CH₂COA (V)
in which
Hal' is a halogen, preferably bromine, and A represents either the group NR₆R₇ or the group OR in which R is a tert-butyl or a benzyl;
c) if applicable, the thus obtained ester of formula : 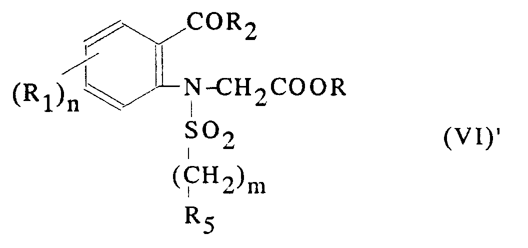 is isolated or, if applicable, deprotected under suitable conditions, when A is an OR group and the thus obtained acid of formula : 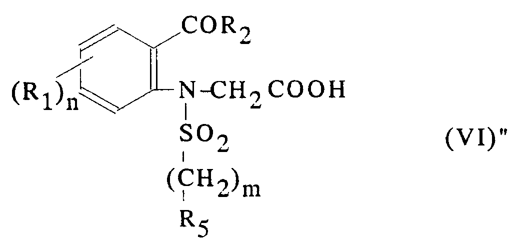 is isolated ;
d) or, if applicable, the thus obtained acid from step c) is treated with a HNR₆R₇ compound according to suitable amide coupling techniques to obtain a compound of formula

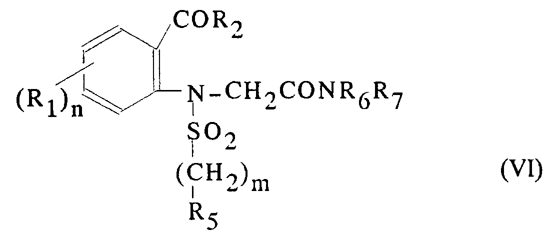

13. A process for preparing a compound of formula (I)'' having the same configuration around the 2,3 bond of the indoline as the starting product 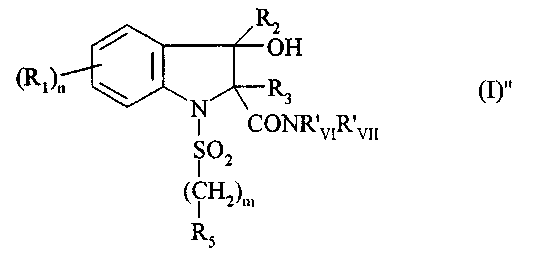 in which
- R₁, R₂, R₃, R₅, m and n are as defined for the compounds of formula (I) in claim 1,
- R'_{VI} is a C₁-C₆ alkyl_{,}
- R'_{VII} is a (CH₂)ᵣH group,
- or R'_{VI} and R'_{VII} together with the nitrogen atom to which they are bonded, form a heterocycle selected from :
. thiazolidine or 2,2-dimethylthiazolidine substituted by a (CH₂)_{q}H group,
. piperazine substituted at the 4-position by a (CH₂)_{q}H group,
. an unsaturated, 5-membered ring containing a single nitrogen atom and substituted by a (CH₂)_{q}H group or
. a saturated, 3-, 4-, 5-, 6- or 7- membered ring containing a single nitrogen atom and substituted by a (CH₂)_{q}H group,
characterised in that a compound of formula (I)' 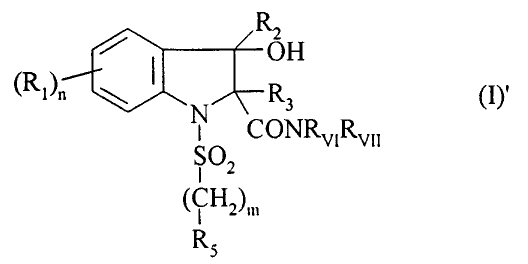 in which R₁, R₂, R₃, R₅, m and n have the meanings given for the compounds of formula (I), in claim 1, and
- R_{VI} is a C₁-C₆ alkyl,
- R_{VII} is a (CH₂)ᵣCOOH group wherein r = 1, 2 or 3,
- or R_{VI} and R_{VII} together, with the nitrogen atom to which they are bonded, form a heterocycle selected from :
. thiazolidine or 2,2-dimethylthiazolidine substituted by a (CH₂)_{q}COOH group,
. piperazine substituted at the 4-position by a (CH₂)_{q}COOH group,
. an unsaturated, 5-membered ring containing a single nitrogen atom and substituted by a (CH₂)_{q}COOH group or
. a saturated, 3-, 4-, 5-, 6- or 7-membered ring containing a single nitrogen atom and substituted by a (CH₂)_{q}COOH group,
with q = 0, 1, 2 or 3,
is subjected to radical decarboxylation.

14. Process for preparing a pharmaceutical composition, characterised in that a compound of formula (I) prepared by the process according to any one of claims 1 to 11 is mixed as an active principle with a pharmaceutically acceptable vehicle.

15. Process for preparing a pharmaceutical composition, characterised in that a compound of formula (I) prepared by the process according to any one of claims 1 to 11 in association with another active principle is mixed as an active principle with a pharmaceutically acceptable vehicle.

## Claims (Claims for the following Contracting State(s): GR)

1. A compound of formula : 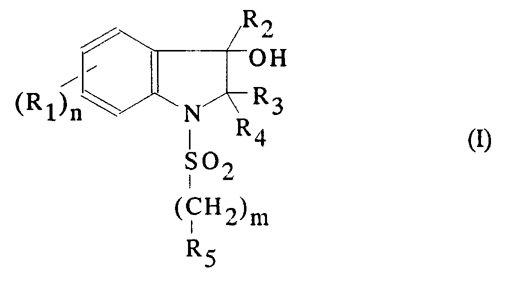 in which
- R₁ is a halogen atom, a C₁-C₄ alkyl, a hydroxyl, a C₁-C₄ alkoxy, a benzyloxy group, a cyano group, a trifluoromethyl group, a nitro group or an amino group ;
- R₂ is a C₁-C₆ alkyl, a C₃-C₇ cycloalkyl, a C₅-C₇ cycloalkene or a phenyl which is unsubstituted, monosubstituted or polysubstituted by a C₁-C₄ alkyl, a C₁-C₄ alkoxy, a halogen, a trifluoromethyl group or an amino group, or R₂ is a nitrophenyl which is unsubstituted or monosubstituted by a trifluoromethyl group or monosubstituted or polysubstituted by a C₁-C₄ alkyl or a halogen ;
- R₃ is a hydrogen atom;
- R₄ is a carbamoyl group of formula CONR₆R₇ ;
- R₅ is a C₁-C₄ alkyl, a 1-naphthyl, a 2-naphtyl, a 5-dimethylamino-1-naphthyl, a phenyl which is unsubstituted or substituted by one or more substituents selected from a halogen, a C₁-C₄ alkyl, a trifluoromethyl group, an amino group which is free or substituted by one or two C₁-C₄ alkyls, a hydroxyl, a C₁-C₄ alkoxy, a C₂-C₄ alkenoxy, a C₁-C₄ alkylthio, a trifluoromethoxy group, a benzyloxy group, a cyano group, a carboxyl group, a C₁-C₄ alkoxycarbonyl group, a carbamoyl group which is free or substituted by one or two C₁-C₄ alkyls or a C₁-C₄ alkylamido group, or R₅ is a nitrophenyl which is unsubstituted or monosubstituted by a trifluoromethyl group or a C₂-C₄ alkenoxy or mono- or polysubstituted by a halogen, a C₁-C₄ alkyl, a C₁-C₄ alkoxy, a C₁-C₄ alkythio, a trifluoromethoxy group or a benzyloxy group ;
- R₆ is a C₁-C₆ alkyl or R₆ is similar to R₇ ;
- R₇ is a 4-piperidinyl group or a 3-azetidinyl group, the said groups being substituted or unsubstituted on the nitrogen by a C₁-C₄ alkyl, by a benzyloxycarbonyl or by a C₁-C₄ alkoxycarbonyl, a (CH₂)ᵣ group which is itself substituted by a 2-, 3- or 4-pyridyl group, by a hydroxyl group or by an amino group which is free or substituted by one or two C₁-C₄ alkyls, a carboxyl group, a C₁-C₄ alkoxycarbonyl group, a benzyloxycarbonyl group or a carbamoyl group which is free or substituted by one or two C₁-C₄ alkyls ;
- or R₆ and R₇ together with the nitrogen atom to which they are bonded, form a heterocycle selected from :
. morpholine,
. thiomorpholine,
. thiazolidine or 2,2-dimethylthiazolidine unsubstituted or substituted by R₈,
. piperazine unsubstituted or substituted at the 4-position by an R''₈ group,
. an unsaturated, 5-membered ring containing a single nitrogen atom and substituted by R₈ or
. a saturated, 3-, 4-, 5-, 6- or 7-membered ring containing a single nitrogen atom and substituted by R₈ and R₉ ;
- R₈ is R'₈ or a (CH₂₎ᵣ group which is itself substituted by a hydroxyl or by an amino which is free or substituted by one or two C₁-C₄ alkyls ;
- R'₈ is a (CH₂)_{q} group which is itself substituted by a carboxyl group, a C₁-C₄ alkoxycarbonyl group, a benzyloxycarbonyl group, a carbamoyl group which is free or substituted by a hydroxyl or by one or two C₁-C₄ alkyls or an aminocarbothioyl group which is free or substituted by one or two C₁-C₄ alkyls ;
- R''₈ is R'₈ or a (CH₂)₂NH₂ group which is free or substituted by one or two C₁-C₄ alkyls ;
- R₉ is hydrogen, a halogen, a (CH₂)ᵣOR₁₀ group, a (CH₂)ᵣNR₁₁R₁₂ group, a (CH₂)ₛCONR₁₁R'₁₁ group or an azido group ;
- R₁₀ is hydrogen, a C₁-C₄ alkyl, a mesyl or a tosyl ;
- R_{11,} R'₁₁ and R₁₂ are each a hydrogen or a C₁-C₄ alkyl or R₁₁ is hydrogen and R₁₂ is a benzyloxycarbonyl or a C₁-C₄ alkoxycarbonyl ;
- n is 0, 1 or 2 ;
- m is 0, 1 or 2 ;
- q is 0, 1, 2 or 3 ;
- r is 0, 1, 2 or 3, with the limitation that r is not zero when R₈ or R₉ is at the alpha-position of the intracyclic amide nitrogen ;
- s is 0 or 1 ;
as well as its possible salts.

2. A compound according to claim 1 in which R₁ is a chlorine or bromine atom or a methoxy group and n = 1.

3. A compound according to any one of claims 1 or 2 in which R₂ is a chlorophenyl, a methoxyphenyl or a cyclohexyl.

4. A compound according to any one of claims 1 to 3 in which R₅ is a phenyl substituted at the 3- and 4-positions or at the 2- and 4-positions by a methoxy group, or else R₅ is a phenyl substituted at the 4-position by a methyl.

5. A compound according to any one of claims 1 to 4 in which m = 0.

6. A compound according to any one of claims 1 to 5 in which R₄ is CONR₆R₇ and NR₆R₇ is a pyrrolidino group substituted at the 2-position by a (CH₂)_{q} group which is itself substituted by a carboxyl group, or a carbamoyl group with q = 0, 1, 2 or 3.

7. A compound according to one of claims 1 to 5 in which R₄ is CONR₆R₇ and NR₆R₇ is a piperidino group substituted at the 4-position by an amino group, a C₁-C₄ alkylamino or a C₁-C₄ dialkylamino.

8. A compound according to any one of claims 1 to 5 in which R₄ is CONR₆R₇ and NR₆R₇ is a thiazolidino group substituted by a (CH₂)_{q} group which is itself substituted by a carboxyl group or a carbamoyl group with q = 0, 1, 2, or 3.

9. A compound according to any one of claims 1 to 5 in which R₄ is CONR₆R₇ and NR₆R₇ is a pyrrolidino group substituted at the 2-position by a (CH₂)_{q} group which is itself substituted by a carboxyl group or a carbamoyl group and substituted at the 4-position by an amino group, a C₁-C₄ alkylamino or a C₁-C₄ dialkylamino.

10. A compound according to any one of claims 1 to 5 in which R₄ is CONR₆R_{7,} R₆ being a C₁-C₄ alkyl and R₇ being a (CH₂)ᵣ group which is itself substituted by a carboxyl group or a carbamoyl group with r = 1, 2 or 3.

11. A compound according to any one of claims 1 to 10 in the form of a cis isomer in which R₂ and R₄ are on the same side of the indoline ring.

12. A process for preparing a compound (I) according to claim 1, characterised in that
a) a 2-aminophenone derivative of formula : 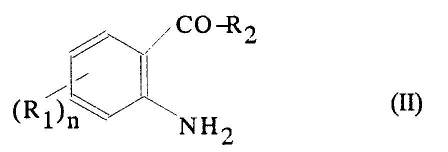 in which R₁, R₂ and n have the meanings indicated above for I in claim 1, is reacted with a sulfonyl derivative of formula :
Hal-SO₂-(CH₂)ₘ-R₅ (III)
in which
- Hal is a halogen, preferably chlorine or bromine,
- m and R₅ have the meanings indicated above for (I) in claim 1 ;
b) the thus obtained compound of formula : 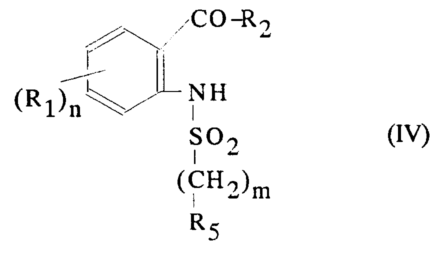 is treated with a halogenated derivative of formula :
Hal'-CH₂COA (V)
in which
Hal' is a halogen, preferably bromine, and A represents either the NR₆R₇ group or the OR group in which R is a tert-butyl or a benzyl;
c) the thus obtained ester of formula : 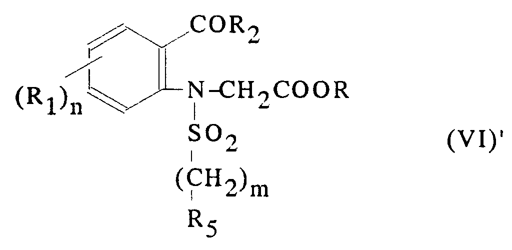 is deprotected under suitable conditions, if applicable, when A is an OR group;
d) if applicable, the resulting acid from Step c) of formula : 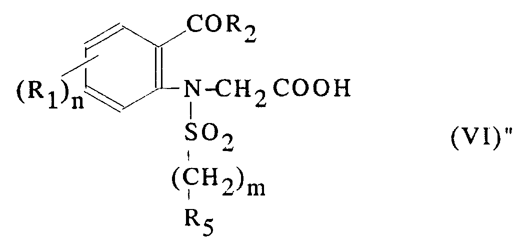 or its acid chloride of formula : 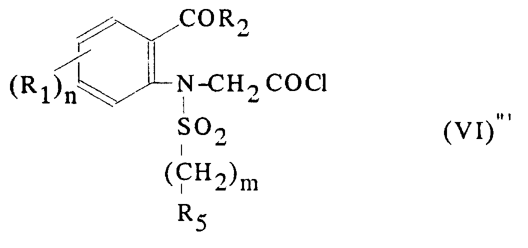 is treated with HNR₆R₇ compound according to suitable amide coupling techniques ;
e) the thus obtained compound from Step b) or from Step d) of formula : 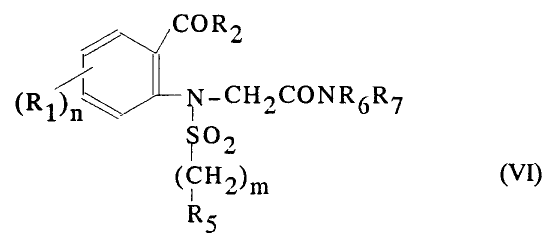 is cyclised in a basic medium in order to prepare compound I according to claim 1 ;
f) if appropriate, the cis and trans isomers of compound (I) according to claim 1 are separated and, if appropriate, the enantiomers are separated.

13. A compound of formula: 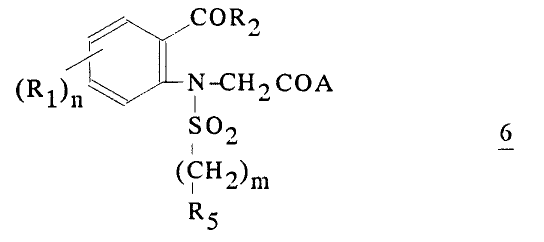 in which
A' is a group selected from : NR₆R₇ , OH, OtBu or OBz;
- R₁ is a halogen atom, a C₁-C₄ alkyl, a hydroxyl, a C₁-C₄ alkoxy, a benzyloxy group, a cyano group, a trifluoromethyl group, a nitro group or an amino group ;
- R₂ is a C₁-C₆ alkyl, a C₃-C₇ cycloalkyl, a C₅-C₇ cycloalkene or a phenyl which is unsubstituted, monosubstituted or polysubstituted by a C₁-C₄ alkyl, a C₁-C₄ alkoxy, a halogen, a trifluoromethyl group or an amino group, or R₂ is a nitrophenyl which is unsubstituted or monosubstituted by a trifluoromethyl group or monosubstituted or polysubstituted by a C₁-C₄ alkyl, a C₁-C₄ alkoxy or a halogen ;
- R₅ is a C₁-C₄ alkyl, a 1-naphthyl, a 2-naphthyl, a 5-dimethylamino-1-naphthyl, a phenyl which is unsubstituted or substituted by one or more substituents selected from a halogen, a C₁-C₄ alkyl, a trifluoromethyl group, an amino group which is free or substituted by one or two C₁-C₄ alkyls, a hydroxyl, a C₁-C₄ alkoxy, a C₂-C₄ alkenoxy, a C₁-C₄ alkylthio, a trifluoromethoxy group, a benzyloxy group, a cyano group, a carboxyl group, a C₁-C₄ alkoxycarbonyl group, a carbamoyl group which is free or substituted by one or two C₁-C₄ alkyls or a C₁-C₄ alkylamido group, or R₅ is a nitrophenyl which is unsubstituted or monosubstituted by a trifluoromethyl group or a C₂-C₄ alkenoxy or mono- or polysubstituted by a halogen, a C₁-C₄ alkyl, a C₁-C₄ alkoxy, a C₁-C₄ alkylthio, a trifluoromethoxy group or a benzyloxy group ;
- R₆ is a C₁-C₆ alkyl or R₆ is similar to R₇ ;
- R₇ is a 4-piperidinyl group or a 3-azetidinyl group, the said groups being substituted or unsubstituted on the nitrogen by a C₁-C₄ alkyl, by a benzyloxycarbonyl or by a C₁-C₄ alkoxycarbonyl, a (CH₂)ᵣ group which is itself substituted by a 2-, 3- or 4-pyridyl group, by a hydroxyl group or by an amino group which is free or substituted by one or two C₁-C₄ alkyls, a carboxyl group, a C₁-C₄ alkoxycarbonyl group, a benzyloxycarbonyl group or a carbamoyl group which is free or substituted by one or 2 C₁-C₄ alkyls ;
- or R₆ and R₇ together with the nitrogen atom to which they are bonded, form a heterocycle selected from ;
. morpholine,
. thiomorpholine,
. thiazolidine or 2,2-dimethylthiazolidine unsubstituted or substituted by R₈,
. piperazine unsubstituted or substituted at the 4-position by an R''₈ group,
. an unsaturated, 5-membered ring containing a single nitrogen atom and substituted by R₈ or
. a saturated, 3-, 4-, 5-, 6- or 7-membered ring containing a single nitrogen atom and substituted by R₈ and R₉ ;
- R₈ is R'₈ or a (CH₂)ᵣ group which is itself substituted by a hydroxyl or by an amino which is free or substituted by one or two C₁-C₄ alkyls ;
- R'₈ is a (CH₂)_{q} group which is itself substituted by a carboxyl group, a C₁-C₄ alkoxycarbonyl group, a benzyloxycarbonyl group, a carbamoyl group which is free or substituted by a hydroxyl or by one or 2 C₁-C₄ alkyls or an aminocarbothioyl group which is free or substituted by one or two C₁-C₄ alkyls ;
- R''₈ is R'₈ or a (CH₂)₂NH₂ group which is free or substituted by one or two C₁-C₄ alkyls ;
- R₉ is hydrogen, a halogen, a (CH₂)ᵣOR₁₀ group, a (CH₂)ᵣNR₁₁R₁₂ group, a (CH₂)_{S}CONR₁₁R'₁₁ group or an azido group ;
- R₁₀ is hydrogen, a C₁-C₄ alkyl, a mesyl or a tosyl ;
- R₁₁, R'₁₁ and R₁₂ are each a hydrogen or a C₁-C₄ alkyl or R₁₁ is hydrogen and R₁₂ is a benzyloxycarbonyl or a C₁-C₄ alkoxycarbonyl ;
- n is 0, 1 or 2 ;
- m is 0, 1 or 2 ;
- q is 0, 1, 2 or 3 ;
- r is 0, 1, 2 or 3, with the limitation that r is not zero when R₈ or R₉ is at the alpha-position of the intracyclic amide nitrogen ;
- s is 0 or 1.

14. The use of a compound of formula : 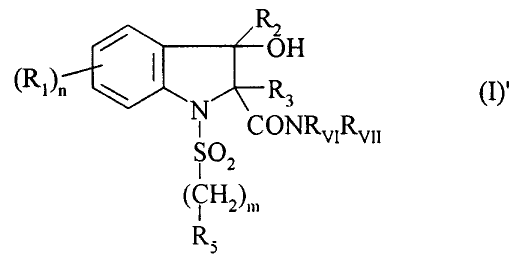 in which R₁, R₂, R₃, R₅, m and n have the meanings given for the compounds of formula (I) in claim 1, and
- R_{VI} is a C₁-C₆ alkyl,
- R_{VII} is a (CH₂)ᵣCOOH group with r = 1, 2 or 3,
- or R_{VI} and R_{VII} together with the nitrogen atom to which they are bonded, form a heterocycle selected from :
. thiazolidine or 2,2-dimethylthiazolidine, substituted by a (CH₂)_{q}COOH group,
. piperazine substituted at the 4-position by a (CH₂)_{q}COOH group,
. an unsaturated, 5-membered ring containing a single nitrogen atom and substituted by a (CH₂)_{q}COOH group or
. a saturated, 3-, 4-, 5-, 6- or 7-membered ring containing a single nitrogen atom and substituted by a (CH₂)_{q}COOH group,
with q = 0, 1, 2 or 3
for preparing a compound of formula (I)'' having the same configuration around the 2,3 bond of the indoline as the starting product 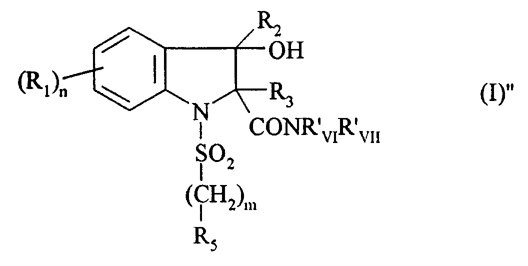 in which
- R₁, R₂, R₃, R₅, m and n are as defined above,
- R'_{VI} is a C₁-C₆ alkyl_{,}
- R'_{VII} is a (CH₂)ᵣH group,
- or R'_{VI} and R'_{VII} together with the nitrogen atom to which they are bonded, form a heterocycle selected from :
. thiazolidine or 2,2-dimethylthiazolidine, substituted by a (CH₂)_{q}H group,
. piperazine substituted at the 4-position by a (CH₂)_{q}H group,
. an unsaturated, 5-membered ring containing a single nitrogen atom and substituted by a (CH₂)_{q}H group or
. a saturated 3-, 4-, 5-, 6- or 7-membered ring containing a single nitrogen atom and substituted by a (CH₂)_{q}H group.

15. Process for preparing a pharmaceutical composition, characterised in that a compound of formula (I) according to any one of claims 1 to 11 is mixed as an active principle with a pharmaceutically acceptable vehicle.

16. Process for preparing a pharmaceutical composition, characterised in that a compound of formula (I) according to any one of claims 1 to 11 in association with another active principle is mixed as an active principle with a pharmaceutically acceptable vehicle.

## Patentansprüche (Patentansprüche für folgende(n) Vertragsstaat(en): AT, BE, CH, DE, DK, FR, GB, IE, IT, LI, LU, NL, PT, SE)

1. Verbindungen der Formel 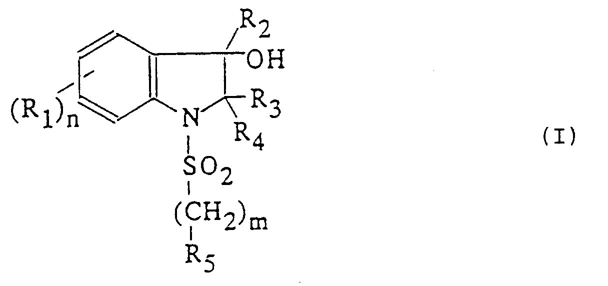 worin bedeuten:
- R₁ Halogen, C₁₋₄-Alkyl, Hydroxy, C₁₋₄-Alkoxy, Benzyloxy, Cyano, Trifluormethyl, Nitro oder Amino;
- R₂ C₁₋₆-Alkyl, C₃₋₇-Cycloalkyl, C₅₋₇-Cycloalkenyl, Phenyl, das unsubstituiert vorliegt oder ein- oder mehrfach mit C₁₋₄-Alkyl, C₁₋₄-Alkoxy, Halogen, Trifluormethyl oder Amino substituiert ist, oder Nitrophenyl, das unsubstituiert vorliegt oder mit einer Trifluormethylgruppe oder ein- oder mehrfach mit C₁₋₄-Alkyl oder Halogen substituiert ist;
- R₃ Wasserstoff;
- R₄ eine Carbamoylgruppe der Formel CONR₆R₇;
- R₅ C₁₋₄-Alkyl, Naphth-1-yl, Naphth-2-yl, 5-Dimethylaminonaphth-1-yl, Phenyl, das unsubstituiert vorliegt oder mit einem oder mehreren Substituenten substituiert ist, die unter Halogen, C₁₋₄-Alkyl, Trifluormethyl, Amino, das unsubstituiert vorliegt oder mit einer oder zwei C₁₋₄-Alkylgruppen substituiert ist, Hydroxy, C₁₋₄-Alkoxy, C₂₋₄-Alkenoxy, C₁₋₄-Alkylthio, Trifluormethoxy, Benzyloxy, Cyano, Carboxy, C₁₋₄-Alkoxycarbonyl, Carbamoyl, das unsubstituiert vorliegt oder mit einer oder zwei C₁₋₄-Alkylgruppen substituiert ist, oder C₁₋₄-Alkylamido ausgewählt sind, oder Nitrophenyl, das unsubstituiert vorliegt oder mit einer Trifluormethyl- oder C₂₋₄-Alkenoxygruppe oder ein- oder mehrfach mit Halogen, C₁₋₄-Alkyl, C₁₋₄-Alkoxy, C₁₋₄-Alkylthio, Trifluormethoxy oder Benzyloxygruppen substituiert ist;
- R₆ C₁₋₆-Alkyl oder R₇;
- R₇ Piperidin-4-yl oder Azetidin-3-yl, wobei die Gruppen gegebenenfalls am Stickstoffatom mit C₁₋₄-Alkyl, Benzyloxycarbonyl oder C₁₋₄-Alkoxycarbonyl substituiert sind, eine Gruppe (CH₂)ᵣ, die mit 2-Piperidyl, 3-Piperidyl oder 4-Piperidyl, Hydroxy, Amino, das unsubstituiert vorliegt oder mit einer oder zwei C₁₋₄-Alkylgruppen substituiert ist, Carboxy, C₁₋₄-Alkoxycarbonyl, Benzyloxycarbonyl oder Carbamoyl, das unsubstituiert vorliegt oder mit einer oder zwei C₁₋₄-Alkylgruppen substituiert ist, substituiert ist;
- oder R₆ und R₇ bilden gemeinsam mit dem Stickstoffatom, an das sie gebunden sind, einen Heterocyclus, der ausgewählt ist unter:
. Morpholin,
. Thiomorpholin,
. Thiazolidin oder 2,2-Dimethylthiazolidin, die gegebenenfalls mit R₈ substituiert sind,
. Piperazin, das gegebenenfalls in 4-Stellung mit R''₈ substituiert ist,
. einem 5-gliedrigen ungesättigten Ring mit einem Stickstoffatom, der mit R₈ substituiert ist, oder einem gesättigten 3-, 4-, 5-, 6- oder 7-gliedrigen Ring mit einem Stickstoffatom, der mit R₈ und R₉ substituiert ist;
- R₈ R'₈ oder eine Gruppe (CH₂)ᵣ, die mit Hydroxy oder Amino substituiert ist, welches unsubstituiert vorliegt oder mit einer oder zwei C₁₋₄-Alkylgruppen substituiert ist;
- R'₈ eine Gruppe (CH₂)_{q}, die substituiert ist mit Carboxy, C₁₋₄-Alkoxycarbonyl, Benzyloxycarbonyl, Carbamoyl, das unsubstituiert vorliegt oder mit Hydroxy oder einer oder zwei C₁₋₄-Alkylgruppen substituiert ist, oder Aminocarbothioyl, das unsubstituiert vorliegt oder mit einer oder zwei C₁₋₄-Alkylgruppen substituiert ist;
- R''₈ R'₈ oder eine Gruppe (CH₂)₂NH₂, die unsubstituiert vorliegt oder mit einer oder zwei C₁₋₄-Alkylgruppen substituiert ist;
- R₉ Wasserstoff, Halogen, eine Gruppe (CH₂)ᵣOR₁₀, eine Gruppe (CH₂)ᵣNR₁₁R₁₂, eine Gruppe (CH₂)ₛCONR₁₁R'₁₁ oder eine Gruppe Azido;
- R₁₀ Wasserstoff, C₁₋₄-Alkyl, Mesyl oder Tosyl;
- R₁₁, R'₁₁ und R₁₂ jeweils Wasserstoff oder C₁₋₄-Alkyl oder R₁₁ Wasserstoff und R₁₂ Benzyloxycarbonyl oder C₁₋₄-Alkoxycarbonyl;
- n Null, 1 oder 2;
- m Null, 1 oder 2;
- q Null, 1, 2 oder 3;
- r Null, 1, 2 oder 3, mit der Maßgabe, daß r nicht Null ist, wenn sich R₈ oder R₉ in α-Stellung zum Amidstickstoff im Ring befinden;
- s Null oder 1;
sowie gegebenenfalls die Salze dieser Verbindungen.

2. Verbindungen nach Anspruch 1, worin R₁ Chlor, Brom oder eine Methoxygruppe bedeutet und n 1 ist.

3. Verbindungen nach einem der Ansprüche 1 oder 2, worin R₂ Chlorphenyl, Methoxyphenyl oder Cyclohexyl bedeutet.

4. Verbindungen nach einem der Ansprüche 1 bis 3, worin R₅ Phenyl, das in 3- und 4-Stellung oder in 2- und 4-Stellung mit Methoxy substituiert ist, oder Phenyl bedeutet, das in 4-Stellung mit Methyl substituiert ist.

5. Verbindungen nach einem der Ansprüche 1 bis 4, worin m Null ist.

6. Verbindungen nach einem der Ansprüche 1 bis 5, worin R₄ CONR₆R₇ bedeutet, wobei NR₆R₇ eine Pyrrolidinogruppe ist, die in 2-Stellung mit einer Gruppe (CH₂)_{q} substituiert ist, welche mit einer Carboxygruppe oder einer Carbamoylgruppe substituiert ist, und wobei q Null, 1, 2 oder 3 bedeutet.

7. Verbindung nach einem der Ansprüche 1 bis 5, worin R₄ CONR₆R₇ bedeutet, wobei NR₆R₇ eine Piperidinogruppe ist, die in 4-Stellung mit Amino, C₁₋₄-Alkylamino oder C₁₋₄-Dialkylamino substituiert ist.

8. Verbindungen nach einem der Ansprüche 1 bis 5, worin R₄ CONR₆R₇ bedeutet, wobei NR₆R₇ eine Thiazolidinogruppe ist, die mit einer (CH₂)_{q}-Gruppe substituiert ist, welche mit einer Carboxygruppe oder einer Carbamoylgruppe substituiert ist, und wobei q Null, 1, 2 oder 3 bedeutet.

9. Verbindungen nach einem der Ansprüche 1 bis 5, worin R₄ CONR₆R₇ bedeutet, wobei NR₆R₇ eine Pyrrolidinogruppe ist, die in 2-Stellung mit einer Gruppe (CH₂)_{q} substituiert ist, welche mit einer Carboxygruppe oder einer Carbamoylgruppe substituiert ist, und in 4-Stellung mit einer Aminogruppe, C₁₋₄-Alkylaminogruppe oder C₁₋₄-Dialkylaminogruppe substituiert ist.

10. Verbindungen nach einem der Ansprüche 1 bis 5, worin R₄ CONR₆R₇ bedeutet, wobei R₆ eine C₁₋₄-Alkylgruppe und R₇ eine Gruppe (CH₂)ᵣ bedeutet, die mit einer Carboxygruppe oder einer Carbamoylgruppe substituiert ist, und wobei r 1, 2 oder 3 ist.

11. Verbindungen nach einem der Ansprüche 1 bis 10 in Form der cis-Isomere, worin sich R₂ und R₄ auf der gleichen Seite des Indolinrings befinden.

12. Verfahren zur Herstellung einer Verbindung (I) nach Anspruch 1, dadurch gekennzeichnet, daß:
a) ein 2-Aminophenonderivat der Formel 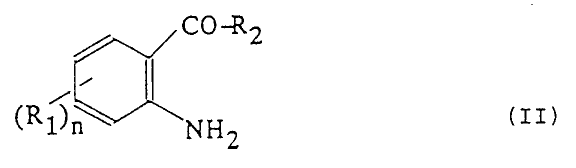 worin R₁, R₂ und n die oben in Anspruch 1 für Formel (I) angegebenen Bedeutungen aufweisen, mit einem Sulfonylderivat der Formel:
Hal - SO₂ - (CH₂)ₘ - R₅ (III)
umgesetzt wird, worin bedeuten:
- Hal Halogen, vorzugsweise Chlor oder Brom,
- m und R₅ die oben in Anspruch 1 für (I) angegebenen Bedeutungen;
b) die so hergestellte Verbindung der Formel 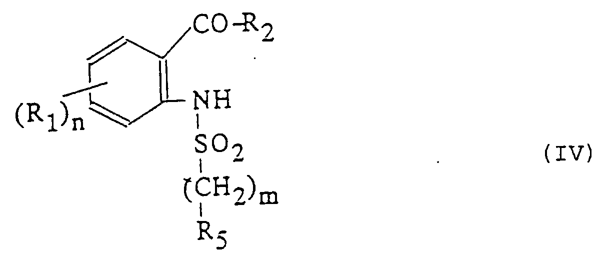 mit einem Halogenderivat der Formel:
Hal' - CH₂ COA (V)
behandelt wird, worin bedeuten:
Hal' Halogen, vorzugsweise Brom,
A entweder die Gruppe NR₆R₇ oder die Gruppe OR, worin R *tert*.-Butyl oder Benzyl ist;
c) falls A eine Gruppe OR bedeutet, gegebenenfalls die Schutzgruppe von dem so hergestellten Ester der Formel 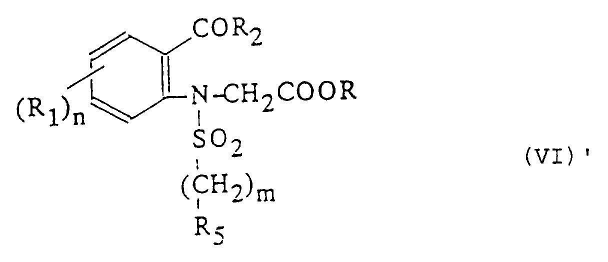 unter geeignet gewählten Bedingungen entfernt wird;
d) gegebenenfalls die in Schritt c) hergestellte Säure der Formel 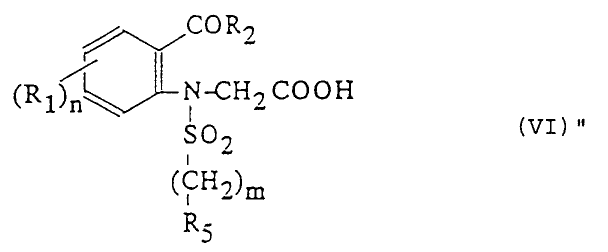 oder ihr Säurechlorid der Formel 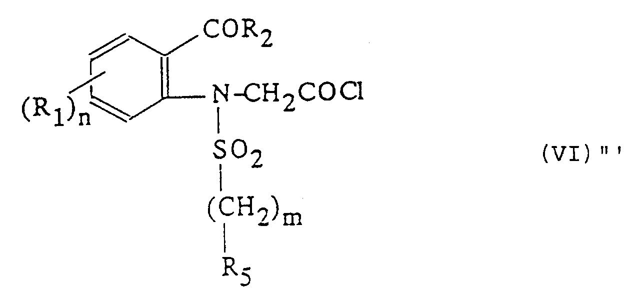 mit einer Verbindung HNR₆R₇ nach geeignet gewählten Verfahren für die Amidbindung behandelt wird;
e) die in Schritt b) oder Schritt d) hergestellte Verbindung der Formel 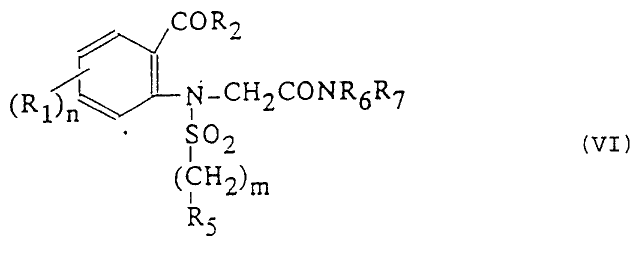 in basischem Medium zur Herstellung der Verbindung (I) nach Anspruch 1 cyclisiert wird; und
f) gegebenenfalls die cis- und trans-Isomere der Verbindung (I) nach Anspruch 1 und gegebenenfalls die Enantiomere getrennt werden.

13. Verbindungen der Formel 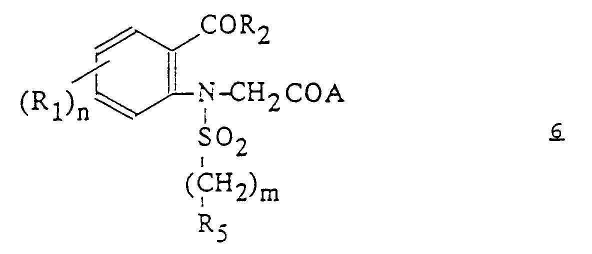 worin bedeuten:
- A' eine Gruppe, die ausgewählt ist unter: NR₆R₇, OH, OtBu, OBz;
- R₁ Halogen, C₁₋₄-Alkyl, Hydroxy, C₁₋₄-Alkoxy, Benzyloxy, Cyano, Trifluormethyl, Nitro oder Amino;
- R₂ C₁₋₆-Alkyl, C₃₋₇-Cycloalkyl, C₅₋₇-Cycloalkenyl, Phenyl, das unsubstituiert vorliegt oder ein- oder mehrfach mit C₁₋₄-Alkyl, C₁₋₄-Alkoxy, Halogen, Trifluormethyl oder Amino substituiert ist, oder Nitrophenyl, das unsubstituiert vorliegt oder mit einer Trifluormethylgruppe oder einer oder mehreren C₁₋₄-Alkylgruppen, C₁₋₄-Alkoxygruppen oder Halogengruppen substituiert ist;
- R₅ C₁₋₄-Alkyl, Naphth-1-yl, Naphth-2-yl, 5-Dimethylaminonaphth-1-yl, Phenyl, das unsubstituiert vorliegt oder mit einem oder mehreren Substituenten substituiert ist, die unter Halogen, C₁₋₄-Alkyl, Trifluormethyl, Amino, das unsubstituiert vorliegt oder mit einer oder zwei C₁₋₄-Alkylgruppen substituiert ist, Hydroxy, C₁₋₄-Alkoxy, C₂₋₄-Alkenoxy, C₁₋₄-Alkylthio, Trifluormethoxy, Benzyloxy, Cyano, Carboxy, C₁₋₄-Alkoxycarbonyl, Carbamoyl, das unsubstituiert vorliegt oder mit einer oder zwei C₁₋₄-Alkylgruppen substituiert ist, oder C₁₋₄-Alkylamido ausgewählt sind, oder Nitrophenyl, das unsubstituiert vorliegt oder mit einer Trifluormethylgruppe oder C₂₋₄-Alkenoxygruppe oder ein- oder mehrfach mit Halogen, C₁₋₄-Alkyl, C₁₋₄-Alkoxy, C₁₋₄-Alkylthio, Trifluormethoxy oder Benzyloxy substituiert ist;
- R₆ C₁₋₆-Alkyl oder R₇;
- R₇ Piperidin-4-yl oder Azetidin-3-yl, wobei die Gruppen gegebenenfalls am Stickstoffatom mit C₁₋₄-Alkyl, Benzyloxycarbonyl oder C₁₋₄-Alkoxycarbonyl substituiert sind, eine Gruppe (CH₂)ᵣ, die mit 2-Piperidyl, 3-Piperidyl oder 4-Piperidyl, Hydroxy, Amino, das unsubstituiert vorliegt oder mit einer oder zwei C₁₋₄-Alkylgruppen substituiert ist, Carboxy, C₁₋₄-Alkoxycarbonyl, Benzyloxycarbonyl oder Carbamoyl, das unsubstituiert vorliegt oder mit einer doer zwei C₁₋₄-Alkylgruppen substituiert ist, substituiert ist;
- oder R₆ und R₇ bilden gemeinsam mit dem Stickstoffatom, an das sie gebunden sind, einen Heterocyclus, der ausgewählt ist unter:
. Morpholin,
. Thiomorpholin,
. Thiazolidin oder 2,2-Dimethylthiazolidin, die gegebenenfalls mit R₈ substituiert sind,
. Piperazin, das gegebenenfalls in 4-Stellung mit R''₈ substituiert ist,
. einem 5-gliedrigen ungesättigten Ring mit einem Stickstoffatom, der mit R₈ substituiert ist, oder
. einem gesättigten 3-, 4-, 5-, 6- oder 7-gliedrigen Ring mit einem Stickstoffatom, der mit R₈ und R₉ substituiert ist;
- R₈ R'₈ oder eine Gruppe (CH₂)ᵣ, die mit Hydroxy oder Amino substituiert ist, welches unsubstituiert vorliegt oder mit einer oder zwei C₁₋₄-Alkylgruppen substituiert ist;
- R'₈ eine Gruppe (CH₂)_{q}, die substituiert ist mit Carboxy, C₁₋₄-Alkoxycarbonyl, Benzyloxycarbonyl, Carbamoyl, das unsubstituiert vorliegt oder mit Hydroxy oder einer oder zwei C₁₋₄-Alkylgruppen substituiert ist, oder Aminocarbothioyl, das unsubstituiert vorliegt oder mit einer oder zwei C₁₋₄-Alkylgruppen substituiert ist;
- R''₈ R'₈ oder eine Gruppe (CH₂)₂NH₂, die unsubstituiert vorliegt oder mit einer oder zwei C₁₋₄-Alkylgruppen substituiert ist;
- R₉ Wasserstoff, Halogen, eine Gruppe (CH₂)ᵣOR₁₀, eine Gruppe (CH₂)ᵣNR₁₁R₁₂, eine Gruppe (CH₂)ₛCONR₁₁R'₁₁ oder eine Gruppe Azido;
- R₁₀ Wasserstoff, C₁₋₄-Alkyl, Mesyl oder Tosyl;
- R₁₁, R'₁₁ und R₁₂ jeweils Wasserstoff oder C₁₋₄-Alkyl oder R₁₁ Wasserstoff und R₁₂ Benzyloxycarbonyl oder C₁₋₄-Alkoxycarbonyl;
- n Null, 1 oder 2;
- m Null, 1 oder 2;
- q Null, 1, 2 oder 3;
- r Null, 1, 2 oder 3, mit der Maßgabe, daß r nicht Null ist, wenn sich R₈ oder R₉ in α-Stellung zum Amidstickstoff im Ring befinden;
- s Null oder 1;

14. Verwendung von Verbindungen der Formel 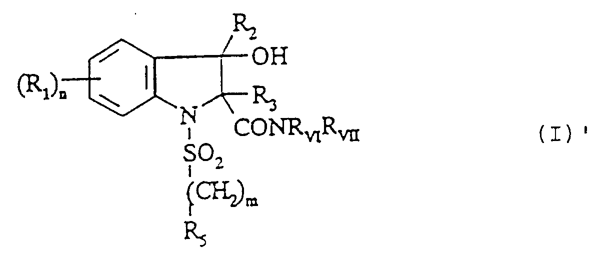 worin R₁, R₂, R₃, R₅, m und n die in Anspruch 1 für die Verbindungen der Formel (I) angegebenen Bedeutungen aufweisen und ferner bedeuten:
- R_{VI} C₁₋₆-Alkyl,
- R_{VII} (CH₂)ᵣCOOH mit r = 1, 2 oder 3,
- oder R_{VI} und R_{VII} bilden gemeinsam mit dem Stickstoffatom, an das sie gebunden sind, einen Heterocyclus, der ausgewählt ist unter:
. Thiazolidin oder 2,2-Dimethylthiazolidin, die mit einer Gruppe (CH₂)_{q}COOH substituiert sind,
. Piperazin, das in 4-Stellung mit einer Gruppe (CH₂)_{q}COOH substituiert ist,
. einem 5-gliedrigen ungesättigten Ring mit einem Stickstoffatom, der mit einer Gruppe (CH₂)_{q}COOH substituiert ist,
. einem 3-, 4-, 5-, 6-, oder 7-gliedrigen gesättigten Ring, der mit einer Gruppe (CH₂)_{q}COOH substituiert ist,
mit q = 0, 1, 2 oder 3
zur Herstellung einer Verbindung der Formel (I)'', die an den Bindungen 2,3 des Indolins die gleiche Konfiguration wie das Ausgangsprodukt aufweist: 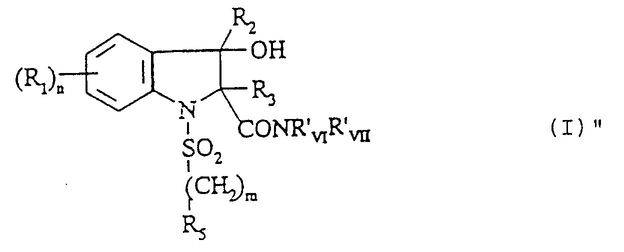 worin bedeuten:
- R₁, R₂, R₃, R₅, m und n die oben angegebenen Bedeutungen,
- R'_{VI} C₁₋₆-Alkyl,
- R'_{VII} die Gruppe (CH₂)ᵣH
oder R'_{VI} und R'_{VII} bilden gemeinsam, mit dem Stickstoffatom, an das sie gebunden sind, einen Heterocyclus, der ausgewählt ist unter:
. Thiazolidin oder 2,2-Dimethylthiazolidin, die mit einer Gruppe (CH₂)_{q}H substituiert sind,
. Piperazin, das in 4-Stellung mit einer Gruppe (CH₂)_{q}H substituiert ist,
. einem 5-gliedrigen ungesättigten Ring mit einem Stickstoffatom, der mit einer Gruppe (CH₂)_{q}H substituiert ist,
. einem 3-, 4-, 5-, 6- oder 7-gliedrigen gesättigten Ring mit einem Stickstoffatom, der mit einer Gruppe (CH₂)_{q}H substituiert ist.

15. Pharmazeutische Zusammensetzung, die als Wirkstoff eine Verbindung nach einem der Ansprüche 1 bis 11 enthält.

16. Pharmazeutische Zusammensetzung, die eine Verbindung nach einem der Ansprüche 1 bis 11 in Kombination mit einem weiteren Wirkstoff enthält.

## Patentansprüche (Patentansprüche für folgende(n) Vertragsstaat(en): ES)

1. Verfahren zur Herstellung einer Verbindung der Formel (I) 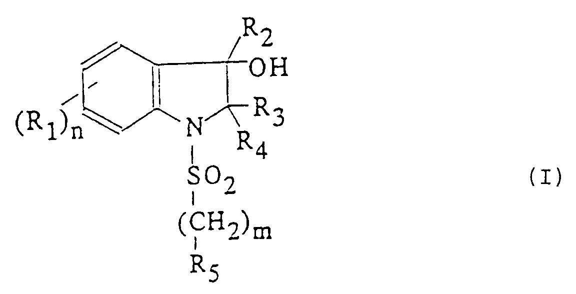 worin bedeuten:
- R₁ Halogen, C₁₋₄-Alkyl, Hydroxy, C₁₋₄-Alkoxy, Benzyloxy, Cyano, Trifluormethyl, Nitro oder Amino;
- R₂ C₁₋₆-Alkyl, C₃₋₇-Cycloalkyl, C₅₋₇-Cycloalkenyl, Phenyl, das unsubstituiert vorliegt oder ein- oder mehrfach mit C₁₋₄-Alkyl, C₁₋₄-Alkoxy, Halogen, Trifluormethyl oder Amino substituiert ist, oder Nitrophenyl, das unsubstituiert vorliegt oder mit einer Trifluormethylgruppe oder ein- oder mehrfach mit C₁₋₄-Alkyl oder Halogen substituiert ist;
- R₃ Wasserstoff;
- R₄ eine Carbamoylgruppe der Formel CONR₆R₇;
- R₅ C₁₋₄-Alkyl, Naphth-1-yl, Naphth-2-yl, 5-Dimethylaminonaphth-1-yl, Phenyl, das unsubstituiert vorliegt oder mit einem oder mehreren Substituenten substituiert ist, die unter Halogen, C₁₋₄-Alkyl, Trifluormethyl, Amino, das unsubstituiert vorliegt oder mit einer oder zwei C₁₋₄-Alkylgruppen substituiert ist, Hydroxy, C₁₋₄-Alkoxy, C₂₋₄-Alkenoxy, C₁₋₄-Alkylthio, Trifluormethoxy, Benzyloxy, Cyano, Carboxy, C₁₋₄-Alkoxycarbonyl, Carbamoyl, das unsubstituiert vorliegt oder mit einer oder zwei C₁₋₄-Alkylgruppen substituiert ist, oder C₁₋₄-Alkylamido ausgewählt sind, oder Nitrophenyl, das unsubstituiert vorliegt oder mit einer Trifluormethyl- oder C₂₋₄-Alkenoxygruppe oder ein- oder mehrfach mit Halogen, C₁₋₄-Alkyl, C₁₋₄-Alkoxy, C₁₋₄-Alkylthio, Trifluormethoxy oder Benzyloxy substituiert ist;
- R₆ C₁₋₆-Alkyl oder R₇;
- R₇ Piperidin-4-yl oder Azetidin-3-yl, wobei die Gruppen gegebenenfalls am Stickstoffatom mit C₁₋₄-Alkyl, Benzyloxycarbonyl oder C₁₋₄-Alkoxycarbonyl substituiert sind, eine Gruppe (CH₂)ᵣ, die mit 2-Piperidyl, 3-Piperidyl oder 4-Piperidyl, Hydroxy, Amino, das unsubstituiert vorliegt oder mit einer oder zwei C₁₋₄-Alkylgruppen substituiert ist, Carboxy, C₁₋₄-Alkoxycarbonyl, Benzyloxycarbonyl oder Carbamoyl, das unsubstituiert vorliegt oder mit einer oder zwei C₁₋₄-Alkylgruppen substituiert ist, substituiert ist;
- oder R₆ und R₇ bilden gemeinsam mit dem Stickstoffatom, an das sie gebunden sind, einen Heterocyclus, der ausgewählt ist unter:
. Morpholin,
. Thiomorpholin,
. Thiazolidin oder 2,2-Dimethylthiazolidin, die gegebenenfalls mit R₈ substituiert sind,
. Piperazin, das gegebenenfalls in 4-Stellung mit R''₈ substituiert ist,
. einem 5-gliedrigen ungesättigten Ring mit einem Stickstoffatom, der mit R₈ substituiert ist, oder einem gesättigten 3-, 4-, 5-, 6- oder 7-gliedrigen Ring mit einem Stickstoffatom, der mit R₈ und R₉ substituiert ist;
- R₈ R'₈ oder eine Gruppe (CH₂)ᵣ, die mit Hydroxy oder Amino substituiert ist, welches unsubstituiert vorliegt oder mit einer oder zwei C₁₋₄-Alkylgruppen substituiert ist;
- R'₈ eine Gruppe (CH₂)_{q}, die substituiert ist mit Carboxy, C₁₋₄-Alkoxycarbonyl, Benzyloxycarbonyl, Carbamoyl, das unsubstituiert vorliegt oder mit Hydroxy oder einer oder zwei C₁₋₄-Alkylgruppen substituiert ist, oder Aminocarbothioyl, das unsubstituiert vorliegt oder mit einer oder zwei C₁₋₄-Alkylgruppen substituiert ist;
- R''₈ R'₈ oder eine Gruppe (CH₂)₂NH₂, die unsubstituiert vorliegt oder mit einer oder zwei C₁₋₄-Alkylgruppen substituiert ist;
- R₉ Wasserstoff, Halogen, eine Gruppe (CH₂)ᵣOR₁₀, eine Gruppe (CH₂)ᵣNR₁₁R₁₂, eine Gruppe (CH₂)ₛCONR₁₁R'₁₁ oder eine Gruppe Azido;
- R₁₀ Wasserstoff, C₁₋₄-Alkyl, Mesyl oder Tosyl;
- R₁₁, R'₁₁ und R₁₂ jeweils Wasserstoff oder C₁₋₄-Alkyl oder R₁₁ Wasserstoff und R₁₂ Benzyloxycarbonyl oder C₁₋₄-Alkoxycarbonyl;
- n Null, 1 oder 2;
- m Null, 1 oder 2;
- q Null, 1, 2 oder 3;
- r Null, 1, 2 oder 3, mit der Maßgabe, daß r nicht Null ist, wenn sich R₈ oder R₉ in α-Stellung zum Amidstickstoff im Ring befinden;
- s Null oder 1;
sowie gegebenenfalls die Salze dieser Verbindungen
dadurch gekennzeichnet, daß
a) ein 2-Aminophenonderivat der Formel 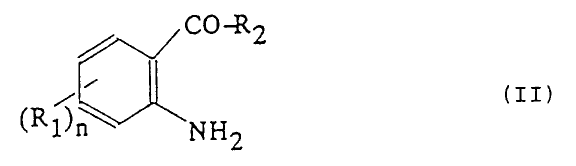 worin R₁, R₂ und n die oben angegebenen Bedeutungen aufweisen, mit einem Sulfonylderivat der Formel:
Hal - SO₂ - (CH₂)ₘ - R₅ (III)
umgesetzt wird, worin bedeuten:
- Hal Halogen, vorzugsweise Chlor oder Brom,
- m und R₅ die oben angegebenen Bedeutungen;
b) die so hergestellte Verbindung der Formel 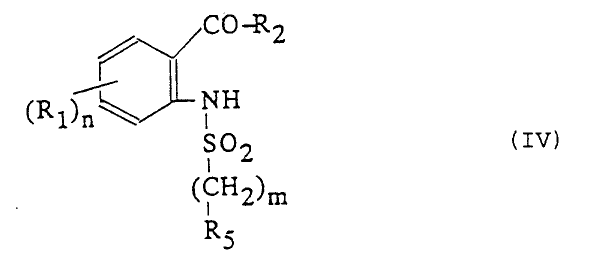 mit einem Halogenderivat der Formel:
Hal' - CH₂ COA (V)
behandelt wird, worin bedeuten:
Hal' Halogen, vorzugsweise Brom,
A entweder die Gruppe NR₆R₇ oder die Gruppe OR, worin R *tert*.-Butyl oder Benzyl ist;
c) falls A eine Gruppe OR bedeutet, gegebenenfalls die Schutzgruppe von dem so hergestellten Ester der Formel 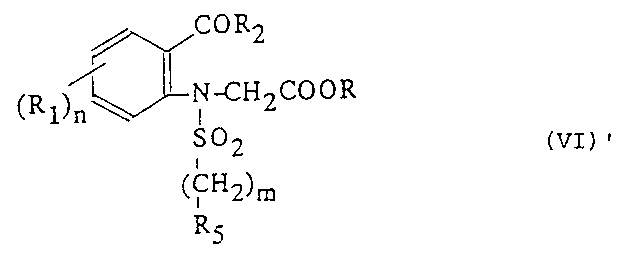 unter geeignet gewählten Bedingungen entfernt wird;
d) gegebenenfalls die in Schritt c) hergestellte Säure der Formel 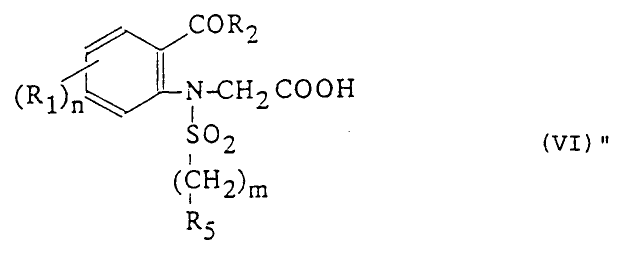 oder ihr Säurechlorid der Formel 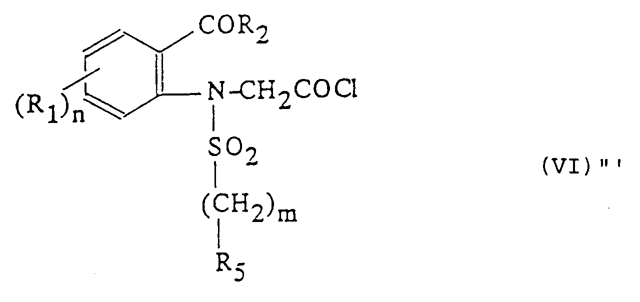 mit einer Verbindung HNR₆R₇ nach geeignet gewählten Verfahren für die Amidbindung behandelt wird;
e) die in Schritt b) oder Schritt d) hergestellte Verbindung der Formel 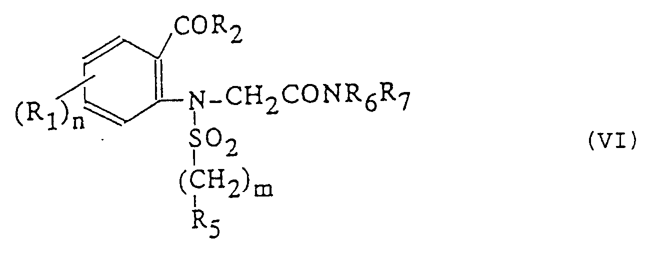 in basischem Medium zur Herstellung der Verbindung (I) cyclisiert wird; und
f) gegebenenfalls die cis- und trans-Isomere der Verbindung (I) und gegebenenfalls die Enantiomere getrennt werden, wobei die Verbindungen der Formel (I) gegebenenfalls mit anorganischen oder organischen Säuren oder mit anorganischen oder organischen Basen in ihre Salze übergeführt werden.

2. Verfahren nach Anspruch 1 zur Herstellung einer Verbindung der Formel (I), worin R₁ Chlor, Brom oder Methoxy bedeutet und n 1 ist, dadurch gekennzeichnet, daß in Schritt a) eine Verbindung der Formel (II) verwendet wird, worin R₁ und n die oben angegebenen Bedeutungen aufweisen.

3. Verfahren nach einem der Ansprüche 1 oder 2 zur Herstellung einer Verbindung der Formel (I), worin R₂ Chlorphenyl, Methoxyphenyl oder Cyclohexyl bedeutet, dadurch gekennzeichnet, daß in Schritt a) eine Verbindung der Formel (II) verwendet wird, worin R₂ die oben angegebenen Bedeutungen aufweist.

4. Verfahren nach einem der Ansprüche 1 bis 3 zur Herstellung einer Verbindung der Formel (I), worin R₅ Phenyl bedeutet, das in 3- und 4-Stellung oder in 2- und 4-Stellung mit einer Methoxygruppe substituiert ist, oder R₅ Phenyl bedeutet, das in 4-Stellung mit Methyl substituiert ist, dadurch gekennzeichnet, daß in Schritt a) eine Verbindung der Formel (III) verwendet wird, worin R₅ die oben angegebene Bedeutung aufweist.

5. Verfahren nach einem der Ansprüche 1 bis 4 zur Herstellung einer Verbindung der Formel (I), worin m Null ist, dadurch gekennzeichnet, daß in Schritt a) eine Verbindung der Formel (III) verwendet wird, worin m Null ist.

6. Verfahren nach einem der Ansprüche 1 bis 5 zur Herstellung einer Verbindung der Formel (I), worin R₄ CONR₆R₇ bedeutet, wobei NR₆R₇ eine Pyrrolidinogruppe ist, die in 2-Stellung mit einer Gruppe (CH₂)_{q} substituiert ist, welche mit einer Carboxygruppe oder einer Carbamoylgruppe substituiert ist, und wobei q Null, 1, 2 oder 3 ist, dadurch gekennzeichnet, daß in Schritt b) eine Verbindung der Formel (V) verwendet wird, worin A eine oben definierte Gruppe NR₆R₇ bedeutet.

7. Verfahren nach einem der Ansprüche 1 bis 5 zur Herstellung einer Verbindung der Formel (I), worin R₄ CONR₆R₇ bedeutet, wobei NR₆R₇ eine Piperidinogruppe ist, die in 4-Stellung mit Amino, C₁₋₄-Alkylamino oder C₁₋₄-Dialkylamino substituiert ist, dadurch gekennzeichnet, daß in Schritt b) eine Verbindung der Formel (V) verwendet wird, worin A eine oben definierte NR₆R₇-Gruppe bedeutet.

8. Verfahren nach einem der Ansprüche 1 bis 5 zur Herstellung einer Verbindung der Formel (I) worin R₄ CONR₆R₇ bedeutet, wobei NR₆R₇ eine Thiazolidinogruppe ist, die mit einer Gruppe (CH₂)_{q} substituiert ist, welche mit einer Carboxygruppe oder einer Carbamoylgruppe substituiert ist, und wobei q Null, 1, 2 oder 3 bedeutet, dadurch gekennzeichnet, daß in Schritt b) eine Verbindung der Formel (V) verwendet wird, worin A eine oben definierte NR₆R₇-Gruppe bedeutet.

9. Verfahren nach einem der Ansprüche 1 bis 5 zur Herstellung einer Verbindung der Formel (I), worin R₄ CONR₆R₇ bedeutet, wobei NR₆R₇ eine Pyrrolidinogruppe ist, die in 2-Stellung mit einer Gruppe (CH₂)_{q} substituiert ist, die mit einer Carboxygruppe oder einer Carbamoylgruppe substituiert ist, und in 4-Stellung mit einer Amino-, C₁₋₄-Alkylamino oder C₁₋₄-Dialkylaminogruppe substituiert ist, dadurch gekennzeichnet, daß in Schritt b) eine Verbindung der Formel (V) verwendet wird, worin A eine oben definierte NR₆R₇-Gruppe bedeutet.

10. Verfahren nach einem der Ansprüche 1 bis 5 zur Herstellung einer Verbindung der Formel (I) worin R₄ CONR₆R₇ bedeutet, wobei R₆ C₁₋₄-Alkyl und R₇ eine Gruppe (CH₂)ᵣ bedeutet, welche mit einer Carboxygruppe oder einer Carbamoylgruppe substituiert ist, und wobei r 1, 2 oder 3 bedeutet, dadurch gekennzeichnet, daß in Schritt b) eine Verbindung der Formel (V) verwendet wird, worin A eine oben definierte NR₆R₇-Gruppe bedeutet.

11. Verfahren nach einem der Ansprüche 1 bis 10 zur Herstellung einer Verbindung der Formel (I) in Form der cis-Isomere, worin sich R₂ und R₄ auf der gleichen Seite des Indolinrings befinden, dadurch gekennzeichnet, daß in Schritt f) die cis-Isomere von den trans-Isomeen durch Chromatographie oder fraktionierte Kristallisation getrennt werden.

12. Verfahren zur Herstellung einer Verbindung der Formel 6 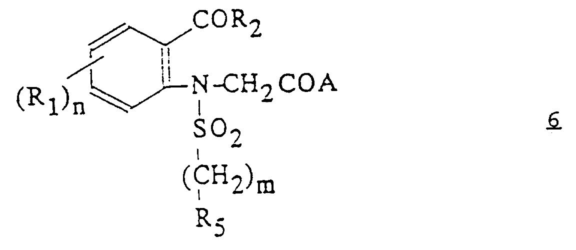 worin bedeuten:
- A' eine Gruppe, die ausgewählt ist unter: NR₆R₇, OH, OtBu, OBz;
- R₁ Halogen, C₁₋₄-Alkyl, Hydroxy, C₁₋₄-Alkoxy, Benzyloxy, Cyano, Trifluormethyl, Nitro oder Amino;
- R₂ C₁₋₆-Alkyl, C₃₋₇-Cycloalkyl, C₅₋₇-Cycloalkenyl, Phenyl, das unsubstituiert vorliegt oder ein- oder mehrfach mit C₁₋₄-Alkyl, C₁₋₄-Alkoxy, Halogen, Trifluormethyl oder Amino substituiert ist, oder Nitrophenyl, das unsubstituiert vorliegt oder mit einer Trifluormethylgruppe oder ein- oder mehrfach mit C₁₋₄-Alkyl, C₁₋₄-Alkoxy oder Halogen substituiert ist;
- R₅ C₁₋₄-Alkyl, Naphth-1-yl, Naphth-2-yl, 5-Dimethylaminonaphth-1-yl, Phenyl, das unsubstituiert vorliegt oder mit einem oder mehreren Substituenten substituiert ist, die unter Halogen, C₁₋₄-Alkyl, Trifluormethyl, Amino, das unsubstituiert vorliegt oder mit einer oder zwei C₁₋₄-Alkylgruppen substituiert ist, Hydroxy, C₁₋₄-Alkoxy, C₂₋₄-Alkenoxy, C₁₋₄-Alkylthio, Trifluormethoxy, Benzyloxy, Cyano, Carboxy, C₁₋₄-Alkoxycarbonyl, Carbamoyl, das unsubstituiert vorliegt oder mit einer oder zwei C₁₋₄-Alkylgruppen substituiert ist, oder C₁₋₄-Alkylamido ausgewählt sind, oder Nitrophenyl, das unsubstituiert vorliegt oder mit einer Trifluormethyl- oder C₂₋₄-Alkenoxygruppe oder ein- oder mehrfach mit Halogen, C₁₋₄-Alkyl, C₁₋₄-Alkoxy, C₁₋₄-Alkylthio, Trifluormethoxy oder Benzyloxy substituiert ist;
- R₆ C₁₋₆-Alkyl oder R₇;
- R₇ Piperidin-4-yl oder Azetidin-3-yl, wobei die Gruppen gegebenenfalls am Stickstoffatom mit C₁₋₄-Alkyl, Benzyloxycarbonyl oder C₁₋₄-Alkoxycarbonyl substituiert sind, eine Gruppe (CH₂)ᵣ, die mit 2-Piperidyl, 3-Piperidyl oder 4-Piperidyl, Hydroxy, Amino, das unsubstituiert vorliegt oder mit einer oder zwei C₁₋₄-Alkylgruppen substituiert ist, Carboxy, C₁₋₄-Alkoxycarbonyl, Benzyloxycarbonyl oder Carbamoyl, das unsubstituiert vorliegt oder mit einer oder zwei C₁₋₄-Alkylgruppen substituiert ist, substituiert ist;
- oder R₆ und R₇ bilden gemeinsam mit dem Stickstoffatom, an das sie gebunden sind, einen Heterocyclus, der ausgewählt ist unter:
. Morpholin,
. Thiomorpholin,
. Thiazolidin oder 2,2-Dimethylthiazolidin, die gegebenenfalls mit R₈ substituiert sind,
. Piperazin, das gegebenenfalls in 4-Stellung mit R''₈ substituiert ist,
. einem 5-gliedrigen ungesättigten Ring mit einem Stickstoffatom, der mit R₈ substituiert ist, oder einem gesättigten 3-, 4-, 5-, 6- oder 7-gliedrigen Ring mit einem Stickstoffatom, der mit R₈ und R₉ substituiert ist;
- R₈ R'₈ oder eine Gruppe (CH₂)ᵣ, die mit Hydroxy oder Amino substituiert ist, welches unsubstituiert vorliegt oder mit einer oder zwei C₁₋₄-Alkylgruppen substituiert ist;
- R'₈ eine Gruppe (CH₂)_{q}, die substituiert ist mit Carboxy, C₁₋₄-Alkoxycarbonyl, Benzyloxycarbonyl, Carbamoyl, das unsubstituiert vorliegt oder mit Hydroxy oder einer oder zwei C₁₋₄-Alkylgruppen substituiert ist, oder Aminocarbothioyl, das unsubstituiert vorliegt oder mit einer oder zwei C₁₋₄-Alkylgruppen substituiert ist;
- R''₈ R'₈ oder eine Gruppe (CH₂)₂NH₂, die unsubstituiert vorliegt oder mit einer oder zwei C₁₋₄-Alkylgruppen substituiert ist;
- R₉ Wasserstoff, Halogen, eine Gruppe (CH₂)ᵣOR₁₀, eine Gruppe (CH₂)ᵣNR₁₁R₁₂, eine Gruppe (CH₂)ₛCONR₁₁R'₁₁ oder eine Gruppe Azido;
- R₁₀ Wasserstoff, C₁₋₄-Alkyl, Mesyl oder Tosyl;
- R₁₁, R'₁₁ und R₁₂ jeweils Wasserstoff oder C₁₋₄-Alkyl oder R₁₁ Wasserstoff und R₁₂ Benzyloxycarbonyl oder C₁₋₄-Alkoxycarbonyl;
- n Null, 1 oder 2;
- m Null, 1 oder 2;
- q Null, 1, 2 oder 3;
- r Null, 1, 2 oder 3, mit der Maßgabe, daß r nicht Null ist, wenn sich R₈ oder R₉ in α-Stellung zum Amidstickstoff im Ring befinden;
- s Null oder 1,
dadurch gekennzeichnet, daß
a) ein 2-Aminophenonderivat der Formel 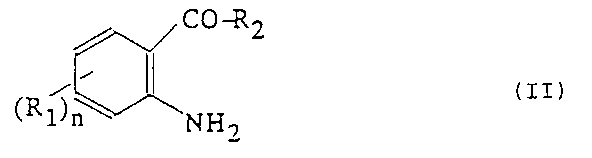 worin R₁, R₂ und n die oben in Anspruch 1 für Formel (I) angegebenen Bedeutungen aufweisen, mit einem Sulfonylderivat der Formel:
Hal - SO₂ - (CH₂)ₘ - R₅ (III)
umgesetzt wird, worin bedeuten:
- Hal Halogen, vorzugsweise Chlor oder Brom,
- m und R₅ die oben in Anspruch 1 für (I) angegebenen Bedeutungen;
b) die so hergestellte Verbindung der Formel 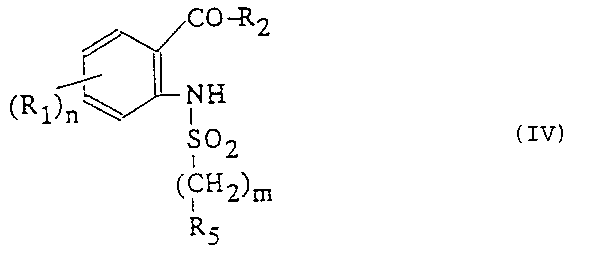 mit einem Halogenderivat der Formel:
Hal' - CH₂ COA (V)
behandelt wird, worin bedeuten:
Hal' Halogen, vorzugsweise Brom,
A entweder die Gruppe NR₆R₇ oder die Gruppe OR, worin R *tert*.-Butyl oder Benzyl ist;
c) falls A eine Gruppe OR bedeutet, gegebenenfalls der so hergestellte Ester der Formel 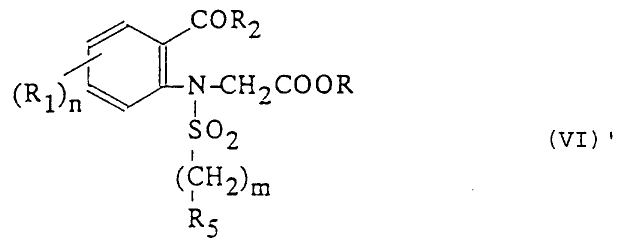 abgetrennt wird oder gegebenenfalls unter geeignet gewählten Bedingungen die Schutzgruppe entfernt wird, und
die hergestellte Säure der Formel 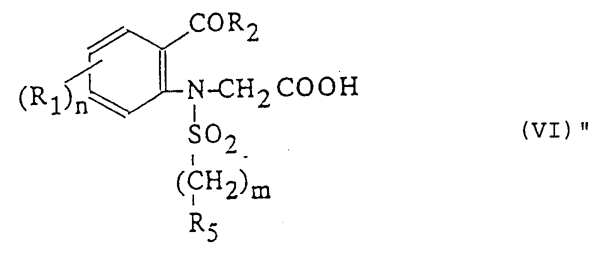 abgetrennt wird, und
d) die in Schritt c) hergestellte Säure gegebenenfalls mit einer Verbindung HNR₆R₇ nach für die Amidbindung geeignet gewählten Verfahren behandelt wird, um eine Verbindung der folgenden Formel herzustellen

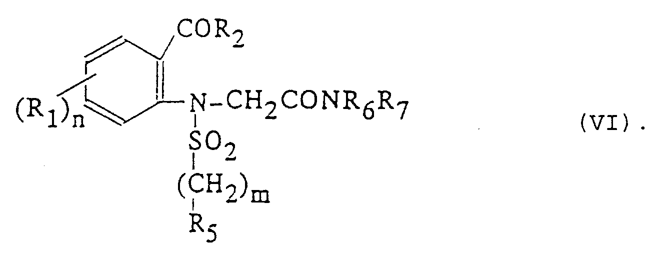

13. Verfahren zur Herstellung einer Verbindung der Formel (I)'', die an den Bindungen 2 und 3 des Indolins die gleiche Konfiguration wie das Ausgangsprodukt aufweist: 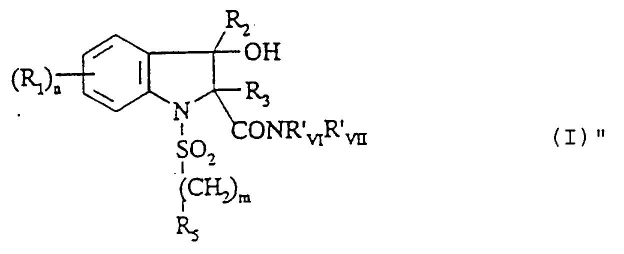 worin bedeuten:
- R₁, R₂, R₃, R₅, m und n die in Anspruch 1 für die Verbindungen der Formel (I) angegebenen Bedeutungen,
- R'_{VI} C₁₋₆-Alkyl,
- R'_{VII} die Gruppe (CH₂)ᵣH
oder R'_{VI} und R'_{VII} bilden gemeinsam, mit dem Stickstoffatom, an das sie gebunden sind, einen Heterocyclus, der ausgewählt ist unter:
. Thiazolidin oder 2,2-Dimethylthiazolidin, die mit einer Gruppe (CH₂)_{q}H substituiert sind
. Piperazin, das in 4-Stellung mit einer Gruppe (CH₂)_{q}H substituiert ist,
. einem 5-gliedrigen ungesättigten Ring mit einem Stickstoffatom, der mit einer Gruppe (CH₂)_{q}H substituiert ist,
. einem 3-, 4-, 5-, 6- oder 7-gliedrigen gesättigten Ring mit einem Stickstoffatom, der mit einer Gruppe (CH₂)_{q}H substituiert ist,
dadurch gekennzeichnet, daß eine Verbindung der Formel (I)' 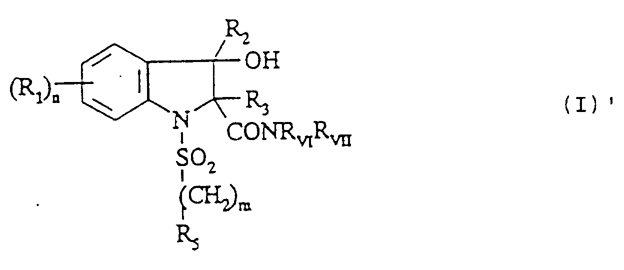 worin R₁, R₂, R₃, R₅, m und n die oben für die in Anspruch 1 für die Verbindungen der Formel (I) angegebenen Bedeutungen aufweisen und ferner bedeuten:
- R_{VI} C₁₋₆-Alkyl,
- R_{VII} (CH₂)ᵣCOOH mit r = 1, 2 oder 3,
- oder R_{VI} und R_{VII} bilden gemeinsam mit dem Stickstoffatom, an das sie gebunden sind, einen Heterocyclus, der ausgewählt ist unter:
. Thiazolidin oder 2,2-Dimethylthiazolidin, die mit einer Gruppe (CH₂)_{q}COOH substituiert sind,
. Piperazin, das in 4-Stellung mit einer Gruppe (CH₂)_{q}COOH substituiert ist,
. einem 5-gliedrigen ungesättigten Ring mit einem Stickstoffatom, der mit einer Gruppe (CH₂)_{q}COOH substituiert ist,
. einem 3-, 4-, 5-, 6-, oder 7-gliedrigen gesättigten Ring, der mit einer Gruppe (CH₂)_{q}COOH substituiert ist,
mit q = 0, 1, 2 oder 3,
radikalisch decarboxyliert wird.

14. Verfahren zur Herstellung einer pharmazeutischen Zusammensetzung, dadurch gekennzeichnet, daß als Wirkstoff eine Verbindung der Formel (I), die nach dem Verfahren nach einem der Ansprüche 1 bis 11 hergestellt ist, mit einem pharmazeutisch akzeptablen Vehikel vermischt wird.

15. Verfahren zur Herstellung einer pharmazeutischen Zusammensetzung, dadurch gekennzeichnet, daß als Wirkstoff eine Verbindung der Formel (I), die nach dem Verfahren nach einem der Ansprüche 1 bis 11 hergestellt ist, in Kombination mit einem weiteren Wirkstoff mit einem pharmazeutisch akzeptablen Vehikel vermischt wird.

## Patentansprüche (Patentansprüche für folgende(n) Vertragsstaat(en): GR)

1. Verbindungen der Formel 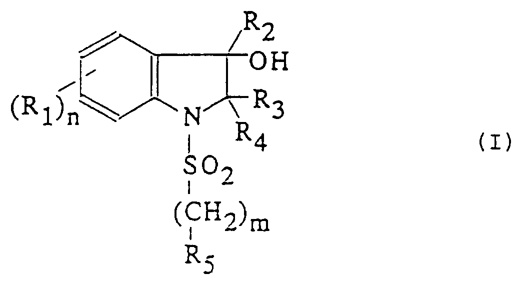 worin bedeuten:
- R₁ Halogen, C₁₋₄-Alkyl, Hydroxy, C₁₋₄-Alkoxy, Benzyloxy, Cyano, Trifluormethyl, Nitro oder Amino;
- R₂ C₁₋₆-Alkyl, C₃₋₇-Cycloalkyl, C₅₋₇-Cycloalkenyl, Phenyl, das unsubstituiert vorliegt oder ein- oder mehrfach mit C₁₋₄-Alkyl, C₁₋₄-Alkoxy, Halogen, Trifluormethyl oder Amino substituiert ist, oder Nitrophenyl, das unsubstituiert vorliegt oder mit einer Trifluormethylgruppe oder ein- oder mehrfach mit C₁₋₄-Alkyl oder Halogen substituiert ist;
- R₃ Wasserstoff;
- R₄ eine Carbamoylgruppe der Formel CONR₆R₇;
- R₅ C₁₋₄-Alkyl, Naphth-1-yl, Naphth-2-yl, 5-Dimethylamino-naphth-1-yl, Phenyl, das unsubstituiert vorliegt oder mit einem oder mehreren Substituenten substituiert ist, die unter Halogen, C₁₋₄-Alkyl, Trifluormethyl, Amino, das unsubstituiert vorliegt oder mit einer oder zwei C₁₋₄-Alkylgruppen substituiert ist, Hydroxy, C₁₋₄-Alkoxy, C₂₋₄-Alkenoxy, C₁₋₄-Alkylthio, Trifluormethoxy, Benzyloxy, Cyano, Carboxy, C₁₋₄-Alkoxycarbonyl, Carbamoyl, das unsubstituiert vorliegt oder mit einer oder zwei C₁₋₄-Alkylgruppen substituiert ist, oder C₁₋₄-Alkylamido ausgewählt sind, oder Nitrophenyl, das unsubstituiert vorliegt oder mit einer Trifluormethylgruppe oder C₂₋₄-Alkenoxygruppe oder ein- oder mehrfach mit Halogen, C₁₋₄-Alkyl, C₁₋₄-Alkoxy, C₁₋₄-Alkylthio, Trifluormethoxy oder Benzyloxy substituiert ist;
- R₆ C₁₋₆-Alkyl oder R₇;
- R₇ Piperidin-4-yl oder Azetidin-3-yl, wobei die Gruppen gegebenenfalls am Stickstoffatom mit C₁₋₄-Alkyl, Benzyloxycarbonyl oder C₁₋₄-Alkoxycarbonyl substituiert sind, eine Gruppe (CH₂)ᵣ, die mit 2-Piperidyl, 3-Piperidyl oder 4-Piperidyl, Hydroxy, Amino, das unsubstituiert vorliegt oder mit einer oder zwei C₁₋₄-Alkylgruppen substituiert ist, Carboxy, C₁₋₄-Alkoxycarbonyl, Benzyloxycarbonyl oder Carbamoyl, das unsubstituiert vorliegt oder mit einer oder zwei C₁₋₄-Alkylgruppen substituiert ist, substituiert ist;
- oder R₆ und R₇ bilden gemeinsam mit dem Stickstoffatom, an das sie gebunden sind, einen Heterocyclus, der ausgewählt ist unter:
. Morpholin,
. Thiomorpholin,
. Thiazolidin oder 2,2-Dimethylthiazolidin, die gegebenenfalls mit R₈ substituiert sind,
. Piperazin, das gegebenenfalls in 4-Stellung mit R''₈ substituiert ist,
. einem 5-gliedrigen ungesättigten Ring mit einem Stickstoffatom, der mit R₈ substituiert ist, oder einem gesättigten 3-, 4-, 5-, 6- oder 7-gliedrigen Ring mit einem Stickstoffatom, der mit R₈ und R₉ substituiert ist;
- R₈ R'₈ oder eine Gruppe (CH₂)ᵣ, die mit Hydroxy oder Amino substituiert ist, welches unsubstituiert vorliegt oder mit einer oder zwei C₁₋₄-Alkylgruppen substituiert ist;
- R'₈ eine Gruppe (CH₂)_{q}, die substituiert ist mit Carboxy, C₁₋₄-Alkoxycarbonyl, Benzyloxycarbonyl, Carbonoyl, das unsubstituiert vorliegt oder mit Hydroxy oder einer oder zwei C₁₋₄-Alkylgruppen substituiert ist, oder Aminocarbothioyl, die unsubstituiert vorliegt oder mit einer oder zwei C₁₋₄-Alkylgruppen substituiert ist;
- R''₈ R'₈ oder eine Gruppe (CH₂)₂NH₂, die unsubstituiert vorliegt oder mit einer oder zwei C₁₋₄-Alkylgruppen substituiert ist;
- R₉ Wasserstoff, Halogen, eine Gruppe (CH₂)ᵣOR₁₀, eine Gruppe (CH₂)ᵣNR₁₁R₁₂, eine Gruppe (CH₂)ₛCONR₁₁R'₁₁ oder eine Gruppe Azido;
- R₁₀ Wasserstoff, C₁₋₄-Alkyl, Mesyl oder Tosyl;
- R₁₁, R'₁₁ und R₁₂ jeweils Wasserstoff oder C₁₋₄-Alkyl oder R₁₁ Wasserstoff und R₁₂ Benzyloxycarbonyl oder C₁₋₄-Alkoxycarbonyl;
- n Null, 1 oder 2;
- m Null, 1 oder 2;
- q Null, 1, 2 oder 3;
- r Null, 1, 2 oder 3, mit der Maßgabe, daß r nicht Null ist, wenn sich R₈ oder R₉ in α-Stellung zum Amidstickstoff im Ring befinden;
- s Null oder 1;
sowie gegebenenfalls die Salze dieser Verbindungen.

2. Verbindungen nach Anspruch 1, worin R₁ Chlor, Brom oder eine Methoxygruppe bedeutet und n 1 ist.

3. Verbindungen nach einem der Ansprüche 1 oder 2, worin R₂ Chlorphenyl, Methoxyphenyl oder Cyclohexyl bedeutet.

4. Verbindungen nach einem der Ansprüche 1 bis 3, worin R₅ Phenyl, das in 3- und 4-Stellung oder in 2- und 4-Stellung mit Methoxy substituiert ist, oder Phenyl bedeutet, das in 4-Stellung mit Methyl substituiert ist.

5. Verbindungen nach einem der Ansprüche 1 bis 4, worin m Null ist.

6. Verbindungen nach einem der Ansprüche 1 bis 5, worin R₄ CONR₆R₇ bedeutet, wobei NR₆R₇ eine Pyrrolidinogruppe ist, die in 2-Stellung mit einer Gruppe (CH₂)_{q} substituiert ist, welche mit einer Carboxygruppe oder einer Carbamoylgruppe substituiert ist, und wobei q Null, 1, 2 oder 3 bedeutet.

7. Verbindung nach einem der Ansprüche 1 bis 5, worin R₄ CONR₆R₇ bedeutet, wobei NR₆R₇ eine Piperidinogruppe ist, die in 4-Stellung mit Amino, C₁₋₄-Alkylamino oder C₁₋₄-Dialkylamino substituiert ist.

8. Verbindungen nach einem der Ansprüche 1 bis 5, worin R₄ CONR₆R₇ bedeutet, wobei NR₆R₇ eine Thiazolidinogruppe ist, die mit einer (CH₂)_{q}-Gruppe substituiert ist, welche mit einer Carboxygruppe oder einer Carbamoylgruppe substituiert ist, und wobei q Null, 1, 2 oder 3 bedeutet.

9. Verbindungen nach einem der Ansprüche 1 bis 5, worin R₄ CONR₆R₇ bedeutet, wobei NR₆R₇ eine Pyrrolidinogruppe ist, die in 2-Stellung mit einer Gruppe (CH₂)_{q} substituiert ist, welche mit einer Carboxygruppe oder einer Carbamoylgruppe substituiert ist, und in 4-Stellung mit einer Aminogruppe, C₁₋₄-Alkylaminogruppe oder C₁₋₄-Dialkylaminogruppe substituiert ist.

10. Verbindungen nach einem der Ansprüche 1 bis 5, worin R₄ CONR₆R₇ bedeutet, wobei R₆ eine C₁₋₄-Alkylgruppe und R₇ eine Gruppe (CH₂)ᵣ bedeutet, die mit einer Carboxygruppe oder einer Carbamoylgruppe substituiert ist, und wobei r 1, 2 oder 3 ist.

11. Verbindungen nach einem der Ansprüche 1 bis 10 in Form der cis-Isomere, worin sich R₂ und R₄ auf der gleichen Seite des Indolinrings befinden.

12. Verfahren zur Herstellung einer Verbindung (I) nach Anspruch 1, dadurch gekennzeichnet, daß:
a) ein 2-Aminophenonderivat der Formel 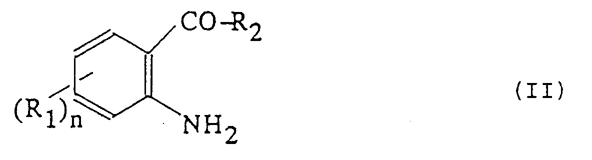 worin R₁, R₂ und n die oben in Anspruch 1 für Formel (I) angegebenen Bedeutungen aufweisen, mit einem Sulfonylderivat der Formel:
Hal - SO₂ - (CH₂)ₘ - R₅ (III)
umgesetzt wird, worin bedeuten:
- Hal Halogen, vorzugsweise Chlor oder Brom,
- m und R₅ die oben in Anspruch 1 für (I) angegebenen Bedeutungen;
b) die so hergestellte Verbindung der Formel 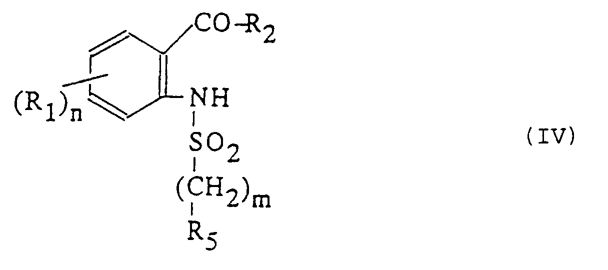 mit einem Halogenderivat der Formel:
Hal' - CH₂ COA (V)
behandelt wird, worin bedeuten:
Hal' Halogen, vorzugsweise Brom,
A entweder die Gruppe NR₆R₇ oder die Gruppe OR, worin R *tert*.-Butyl oder Benzyl ist;
c) falls A eine Gruppe OR bedeutet, gegebenenfalls die Schutzgruppe von dem so hergestellten Ester der Formel 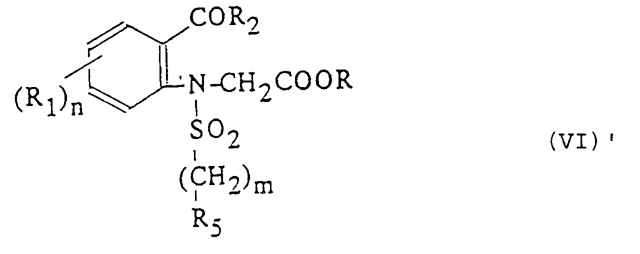 unter geeignet gewählten Bedingungen entfernt wird;
d) gegebenenfalls die in Schritt c) hergestellte Säure der Formel 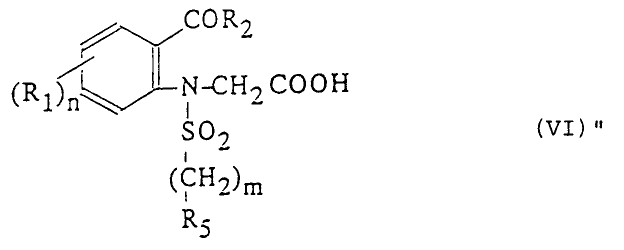 oder ihr Säurechlorid der Formel 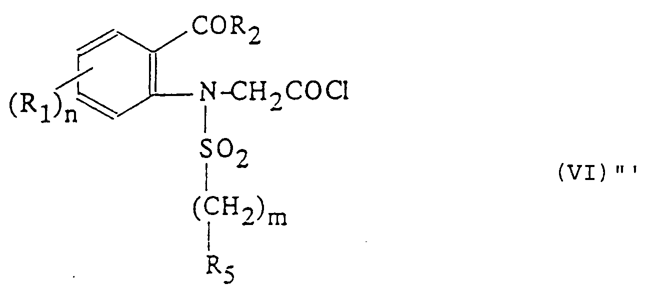 mit einer Verbindung HNR₆R₇ nach geeignet gewählten Verfahren für die Amidbindung behandelt wird;
e) die in Schritt b) oder Schritt d) hergestellte Verbindung der Formel 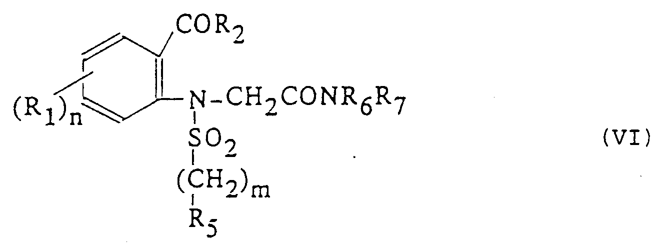 in basischem Medium zur Herstellung der Verbindung (I) nach Anspruch 1 cyclisiert wird; und
f) gegebenenfalls die cis- und trans-Isomere der Verbindung (I) nach Anspruch 1 und gegebenenfalls die Enantiomere getrennt werden.

13. Verbindungen der Formel 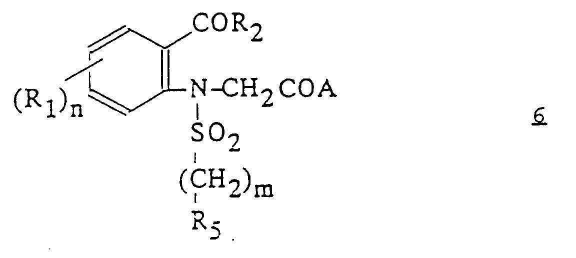 worin bedeuten:
- A' eine Gruppe, die ausgewählt ist unter: NR₆R₇, OH, OtBu, OBz;
- R₁ Halogen, C₁₋₄-Alkyl, Hydroxy, C₁₋₄-Alkoxy, Benzyloxy, Cyano, Trifluormethyl, Nitro oder Amino;
- R₂ C₁₋₆-Alkyl, C₃₋₇-Cycloalkyl, C₅₋₇-Cycloalkenyl, Phenyl, das unsubstituiert vorliegt oder ein- oder mehrfach mit C₁₋₄-Alkyl, C₁₋₄-Alkoxy, Halogen, Trifluormethyl oder Amino substituiert ist, oder Nitrophenyl, das unsubstituiert vorliegt oder mit einer Trifluormethylgruppe oder ein- oder mehrfach mit C₁₋₄-Alkyl oder Halogen substituiert ist;
- R₅ C₁₋₄-Alkyl, Naphth-1-yl, Naphth-2-yl, 5-Dimethylaminonaphth-1-yl, Phenyl, das unsubstituiert vorliegt oder mit einem oder mehreren Substituenten substituiert ist, die unter Halogen, C₁₋₄-Alkyl, Trifluormethyl, Amino, das unsubstituiert vorliegt oder mit einer oder zwei C₁₋₄-Alkylgruppen substituiert ist, Hydroxy, C₁₋₄-Alkoxy, C₂₋₄-Alkenoxy, C₁₋₄-Alkylthio, Trifluormethoxy, Benzyloxy, Cyano, Carboxy, C₁₋₄-Alkoxycarbonyl, Carbamoyl, das unsubstituiert vorliegt oder mit einer oder zwei C₁₋₄-Alkylgruppen substituiert ist, oder C₁₋₄-Alkylamido ausgewählt sind, oder Nitrophenyl, das unsubstituiert vorliegt oder mit einer Trifluormethylgruppe oder C₂₋₄-Alkenoxygruppe oder ein- oder mehrfach mit Halogen, C₁₋₄-Alkyl, C₁₋₄-Alkoxy, C₁₋₄-Alkylthio, Trifluormethoxy oder Benzyloxy substituiert ist;
- R₆ C₁₋₆-Alkyl oder R₇;
- R₇ Piperidin-4-yl oder Azetidin-3-yl, wobei die Gruppen gegebenenfalls am Stickstoffatom mit C₁₋₄-Alkyl, Benzyloxycarbonyl oder C₁₋₄-Alkoxycarbonyl substituiert sind, eine Gruppe (CH₂)ᵣ, die mit 2-Piperidyl, 3-Piperidyl oder 4-Piperidyl, Hydroxy, Amino, das unsubstituiert vorliegt oder mit einer oder zwei C₁₋₄-Alkylgruppen substituiert ist, Carboxy, C₁₋₄-Alkoxycarbonyl, Benzyloxycarbonyl oder Carbamoyl, das unsubstituiert vorligt oder mit einer oder zwei C₁₋₄-Alkylgruppen substituiert ist, substituiert ist;
- oder R₆ und R₇ bilden gemeinsam mit dem Stickstoffatom, an das sie gebunden sind, einen Heterocyclus, der ausgewählt ist unter:
. Morpholin,
. Thiomorpholin,
. Thiazolidin oder 2,2-Dimethylthiazolidin, die gegebenenfalls mit R₈ substituiert sind,
. Piperazin, das gegebenenfalls in 4-Stellung mit R''₈ substituiert ist,
. einem 5-gliedrigen ungesättigten Ring mit einem Stickstoffatom, der mit R₈ substituiert ist, oder einem gesättigten 3-, 4-, 5-, 6- oder 7-gliedrigen Ring mit einem Stickstoffatom, der mit R₈ und R₉ substituiert ist;
- R₈ R'₈ oder eine Gruppe (CH₂)ᵣ, die mit Hydroxy oder Amino substituiert ist, welches unsubstituiert vorliegt oder mit einer oder zwei C₁₋₄-Alkylgruppen substituiert ist;
- R'₈ eine Gruppe (CH₂)_{q}, die substituiert ist mit Carboxy, C₁₋₄-Alkoxycarbonyl, Benzyloxycarbonyl, Carbamoyl, das unsubstituiert vorliegt oder mit Hydroxy oder einer oder zwei C₁₋₄-Alkylgruppen substituiert ist, oder Aminocarbothioyl, das unsubstituiert vorliegt oder mit einer oder zwei C₁₋₄-Alkylgruppen substituiert ist;
- R''₈ R'₈ oder eine Gruppe (CH₂)₂NH₂, die unsubstituiert vorliegt oder mit einer oder zwei C₁₋₄-Alkylgruppen substituiert ist;
- R₉ Wasserstoff, Halogen, eine Gruppe (CH₂)ᵣOR₁₀, eine Gruppe (CH₂)ᵣNR₁₁R₁₂, eine Gruppe (CH₂)ₛCONR₁₁R'₁₁ oder eine Gruppe Azido;
- R₁₀ Wasserstoff, C₁₋₄-Alkyl, Mesyl oder Tosyl;
- R₁₁, R'₁₁ und R₁₂ jeweils Wasserstoff oder C₁₋₄-Alkyl oder R₁₁ Wasserstoff und R₁₂ Benzyloxycarbonyl oder C₁₋₄-Alkoxycarbonyl;
- n Null, 1 oder 2;
- m Null, 1 oder 2;
- q Null, 1, 2 oder 3;
- r Null, 1, 2 oder 3, mit der Maßgabe, daß r nicht Null ist, wenn sich R₈ oder R₉ in α-Stellung zum Amidstickstoff im Ring befinden;
- s Null oder 1;

14. Verwendung von Verbindungen der Formel 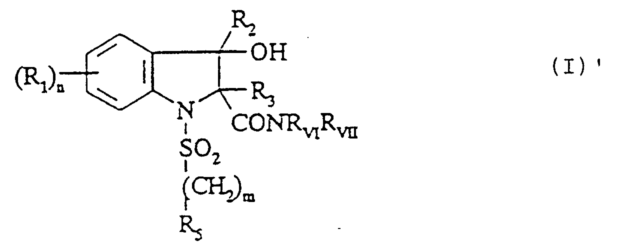 worin R₁, R₂, R₃, R₅, m und n die in Anspruch 1 für die Verbindungen der Formel (I) angegebenen Bedeutungen aufweisen und ferner bedeuten:
- R_{VI} C₁₋₆-Alkyl,
- R_{VII} (CH₂)ᵣCOOH mit r = 1, 2 oder 3,
- oder R_{VI} und R_{VII} bilden gemeinsam mit dem Stickstoffatom, an das sie gebunden sind, einen Heterocyclus, der ausgewählt ist unter:
. Thiazolidin oder 2,2-Dimethylthiazolidin, die mit einer Gruppe (CH₂)_{q}COOH substituiert sind,
. Piperazin, das in 4-Stellung mit einer Gruppe (CH₂)_{q}COOH substituiert ist,
. einem 5-gliedrigen ungesättigten Ring mit einem Stickstoffatom, der mit einer Gruppe (CH₂)_{q}COOH substituiert ist,
. einem 3-, 4-, 5-, 6-, oder 7-gliedrigen gesättigten Ring, der mit einer Gruppe (CH₂)_{q}COOH substituiert ist,
mit q = 0, 1, 2 oder 3
zur Herstellung einer Verbindung der Formel (I)'', die an den Bindungen 2,3 des Indolins die gleiche Konfiguration wie das Ausgangsprodukt aufweist: 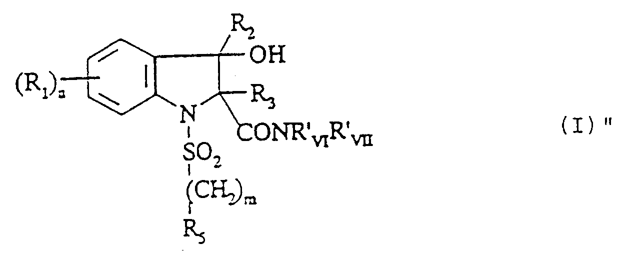 worin bedeuten:
- R₁, R₂, R₃, R₅, m und n die oben angegebenen Bedeutungen,
- R'_{VI} C₁₋₆-Alkyl,
- R'_{VII} die Gruppe (CH₂)ᵣH
oder R'_{VI} und R'_{VII} bilden gemeinsam, mit dem Stickstoffatom, an das sie gebunden sind, einen Heterocyclus, der ausgewählt ist unter:
. Thiazolidin oder 2,2-Dimethylthiazolidin, die mit einer Gruppe (CH₂)_{q}H substituiert sind
. Piperazin, das in 4-Stellung mit einer Gruppe (CH₂)_{q}H substituiert ist,
. einem 5-gliedrigen ungesättigten Ring mit einem Stickstoffatom, der mit einer Gruppe (CH₂)_{q}H substituiert ist,
. einem 3-, 4-, 5-, 6- oder 7-gliedrigen gesättigten Ring mit einem Stickstoffatom, der mit einer Gruppe (CH₂)_{q}H substituiert ist.

15. Verfahren zur Herstellung einer pharmazeutischen Zusammensetzung, dadurch gekennzeichnet, daß als Wirkstoff eine Verbindung der Formel (I) nach einem der Ansprüche 1 bis 11 mit einem pharmazeutisch akzeptablen Vehikel vermischt wird.

16. Verfahren zur Herstellung einer pharmazeutischen Zusammensetzung, dadurch gekennzeichnet, daß als Wirkstoff eine Verbindung der Formel (I) nach einem der Ansprüche 1 bis 11 in Kombination mit einem weiteren Wirkstoff mit einem pharmazeutisch akzeptablen Vehikel vermischt wird.
